# EUROPEAN PATENT APPLICATION

(11) **EP 2 452 942 A1**
(43) Date of publication of application: **16.05.2012**
(21) Application number: 10797180.6
(22) Date of filing: 08.07.2010
(51) Int. Cl.: C07D 495/14, H01L 29/786, H01L 51/05, H01L 51/30

(54) **SUBSTITUTED BENZOCHALCOGENOACENE COMPOUND, THIN FILM COMPRISING THE COMPOUND, AND ORGANIC SEMICONDUCTOR DEVICE INCLUDING THE THIN FILM**

(30) Priority: 10.07.2009 JP 2009163919
(71) Applicant: Sumitomo Chemical Company, Limited, Tokyo 104-8260 (JP); National University Corporation Nagoya University, Nagoya-shi, Aichi 464-8601 (JP)
(72) Inventor: MIYATA, Yasuo, Kanagawa 213-0013 (JP); YOSHIKAWA, Eiji, Tsukuba-shi, Ibaraki 305-0821 (JP); YAMAGUCHI, Shigehiro, Nagoya-shi Aichi 464-8601 (JP)
(74) Representative: Harmsen, Dirk
(86) International application number: PCT/JP2010/061634
(87) International publication number: WO 2011/004869

(57) **Abstract**

Provided are a novel compound suitable as an organic semiconductor material, the compound being a substituted benzochalcogenoacene compound represented by the formula (1), a thin film comprising the compound, and an organic semiconductor device having the thin film as a component. In the formula (1), each E independently represents a sulfur or selenium atom, and R¹ and R² each independently represents a hydrogen atom, an optionally substituted C₄₋₃₀ alkyl group, an optionally substituted C₄₋₃₀ alkoxy group, an optionally substituted C₆₋₃₀ aryl group, an optionally substituted C₇₋₃₀ aralkyl group, an optionally substituted C₄-30 heteroaryl group, an optionally substituted C₅₋₃₀ heteroaralkyl group, or an optionally fluorinated C₃₋₃₀ trialkylsilyl group, both R¹ and R² being not hydrogen atoms.

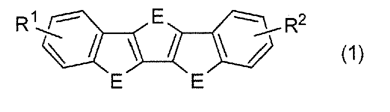

## Description

### TECHNICAL FIELD

The present invention relates to a substituted benzochalcogenoacene compound, a thin film comprising the compound and an organic semiconductor device comprising the thin film.

### BACKGROUND ART

In the non patent document 1, is described dibenzo[d,d']thieno[3,2-b; 4,5'-b']dithiophene, and in the patent document 1, is described a dibenzochalcogenoacene compound represented by the following formula:

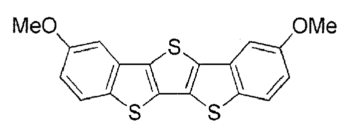

### REFERENCES CITED

Patent Document 1: W02005/087780 [Formula 11]

Non-patent Document 1: Adv. Mater., 2007, 19, 3008-3011

### DISCLOSURE OF INVENTION

### Problem to be solved by invention

In the circumstances mentioned above, a suitable new compound as an organic semiconductor material has been investigated.

### Means for solving the problem

In order to solve these problems, we, the inventors of the present application have made intensive studies on the substituted benzochalcogenoacene compounds, and have attained the following inventions.
That is, the present invention provides:
<1> A substituted benzochalcogenoacene compound represented by the formula (1): 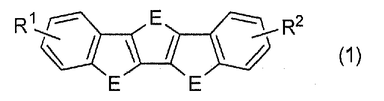 wherein each E independently represents a sulfur or selenium atom, and each of R¹ and R² independently represents a hydrogen atom, an optionally substituted C₄₋₃₀ alkyl group, an optionally substituted C₄₋₃₀ alkoxy group, an optionally substituted C₆₋₃₀ aryl group, an optionally substituted C₇₋₃₀ aralkyl group, an optionally substituted C₄₋₃₀ heteroaryl group, an optionally substituted C₅₋₃₀ heteroaralkyl group, or an optionally fluorinated C₃₋₃₀ trialkylsilyl group, wherein R¹ and R² are not hydrogen atoms all together;
<2> The compound according to <1> wherein all E's in the formula (1) are sulfur atoms;
<3> The compound according to <1> or <2> wherein each of R¹ and R² in the formula (1) independently represents a hydrogen atom, an optionally fluorinated C₄₋₃₀ alkyl group, an optionally fluorinated C₄₋₃₀ alkoxy group, an optionally alkylated or alkoxylated C₆₋₃₀ aryl group which is optionally fluorinated, an optionally fluorinated C₇₋₃₀ aralkyl group, an optionally alkylated or alkoxylated C₄₋₃₀ heteroaryl group which is optionally fluorinated, or an optionally fluorinated C₅₋₃₀ heteroaralkyl group;
<4> The compound according to any one of <1> to <3> wherein the compound represented by the formula (1) is a compound represented by the formula (2): 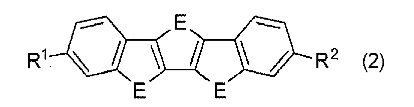 wherein E, R¹ and R² represent the same meanings as described above;
<5> The compound according to <4> wherein, in the formula (2), each E independently represents a sulfur or selenium atom, and each of R¹ and R² independently represents a hydrogen atom, an optionally fluorinated C₄₋₃₀ alkyl group, or an optionally alkylated or fluorinated C₃₋₃₀ trialkylsilyl group;
<6> The compound according to <5> wherein each of R¹ and R² in the formula (2) independently represents a C₄₋₃₀ alkyl group or a C₃₋₃₀ trialkylsilyl group;
<7> The compound according to <5> wherein R¹ and R² in the formula (2) represent C₄₋₃₀ alkyl groups;
<8> The compound according to <5> wherein R¹ and R² in the formula (2) are the same and represent C₄₋₂₀ alkyl groups;
<9> The compound according to <5> wherein R¹ and R² in the formula (2) represent C₆₋₁₂ alkyl groups;
<10> The compound according to <4> wherein each of R¹ and R² in the formula (2) independently represents a hydrogen atom, an optionally fluorinated C₄₋₃₀ alkyl group, an optionally fluorinated C₄₋₃₀ alkoxy group, an optionally alkylated C₆₋₃₀ aryl group which is optionally fluorinated, or an optionally fluorinated C₇₋₃₀ aralkyl group;
<11> The compound according to <4> wherein R¹ and R² in the formula (2) are the same and represent C₄₋₂₀ alkoxy groups;
<12> The compound according to <4> wherein R¹ and R² in the formula (2) are the same and represent C₆₋₁₀ aryl groups having C₁₋₂₀ alkyl groups;
< 13> The compound according to <4> wherein R¹ and R² in the formula (2) are the same and represent C₇₋₂₀ aralkyl groups;
< 14> The compound according to <5> wherein each of R¹ and R² in the formula (2) independently represents a C₃₋₃₀ trialkylsilyl group;
< 15> The compound according to <5> wherein each of R¹ and R² in the formula (2) independently represents a C₃₋₁₄ trialkylsilyl group;
<16> The compound according to <4> or <5> wherein R¹ and R² in the formula (2) are the same and represent hexyl or dodecyl;
< 17> The compound according to any one of <4> to < 16> wherein all E's in the formula (2) represent sulfur atoms;
< 18> The compound according to <4> wherein all E's in the formula (2) represent sulfur atoms, and R¹ and R² in the formula (2) are the same and represent hexyl;
< 19> The compound according to <4> wherein all E's in the formula (2) represent sulfur atoms, and R¹ and R² in the formula (2) are the same and represent dodecyl;
<20> The compound according to <4> wherein all E's in the formula (2) represent sulfur atoms, and each of R¹ and R² in the formula (2) independently represents a C₆₋₁₂ alkyl group.
<21> A compound represented by the formula [5], [7], [12], [15], [18] or [42] below: 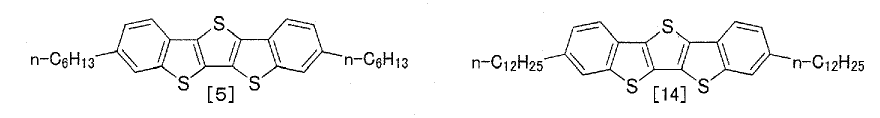 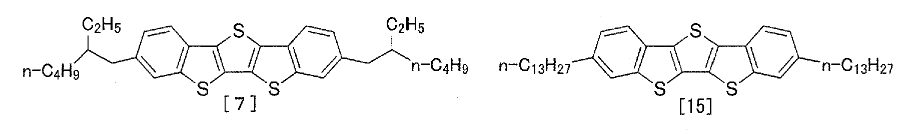 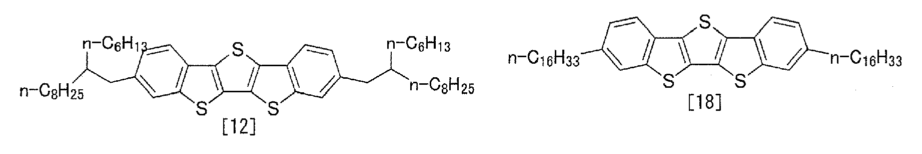 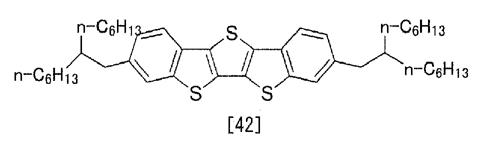
<22> The compound according to any one of <1> to <3> wherein the compound represented by the formula (1) is a compound represented by the formula (3): 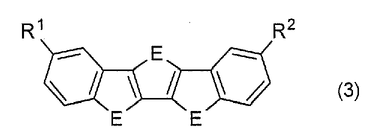 wherein E, R¹ and R² represent the same meanings as described above;
<23> The compound according to <22> wherein R¹ and R² in the formula (3) are the same and represent C₄₋₂₀ alkyl groups;
<24> A thin film comprising the compound according to any one of <1> to <23>;
<25> A thin film consisting of the compound according to any one of <1> to <23>:
<26> An organic semiconductor device comprising the thin film according to <24> or <25>;
<27> An organic transistor comprising the thin film according to <24> or <25>.

### Effect of the invention

The present invention can provide novel substituted benzochalcogenoacene compounds.

### BRIEF DESCRIPTION OF DRAWINGS

Figure 1 is a cross-section diagram illustrating one embodiment of the organic transistor in the present invention.
Figure 2 is a cross-section diagram illustrating one embodiment of the organic transistor in the present invention.

### BEST MODE FOR CARRYING OUT THE INVENTION

First of all, the substituted benzochalcogenoacene compound represented by the formula (1) (hereinafter called "substituted benzochalcogenoacene compound (1) as the case may be") of the present invention will be explained in detail.
Each E in the formulae (1), (2) and (3) independently represents a sulfur or selenium atom. Each of R¹ and R² independently represents a hydrogen atom, an optionally substituted C₄₋₃₀ alkyl group, an optionally substituted C₄₋₃₀ alkoxy group, an optionally substituted C₆₋₃₀ aryl group, an optionally substituted C₇₋₃₀ aralkyl group, an optionally substituted C₄₋₃₀ heteroaryl group, an optionally substituted C₅₋₃₀ heteroaralkyl group, or an optionally fluorinated C₃₋₃₀ trialkylsilyl group. However, at least one of R¹ and R² is not a hydrogen atom.

The "C₄₋₃₀ alkyl group" in the "optionally substituted C₄₋₃₀ alkyl group" in R¹ and R² is any one of a linear, branched or cyclic alkyl group. The specific examples of the C₄₋₃₀ alkyl group include n-butyl, s-butyl, t-butyl, n-pentyl, neopentyl, n-hexyl, 2-ethylhexyl, n-heptyl, n-octyl, 2-hexyloctyl, n-nonyl, n-decyl, 2-hexyldecyl, n-undecyl, n-dodecyl, n-tridecyl, n-tetradecyl, n-pentadecyl, n-hexadecyl, n-heptadecyl, n-octadecyl, n-nonadecyl, n-icosyl, n-henicosyl, n-docosyl, n-tricosyl, n-tetracosyl, n-pentacosyl, n-hexacosyl, n-pehptacosyl, n-octacosyl, n-nonacosyl, n-triacontyl, cyclopentyl, cyclohexyl and cycloheptyl, and preferably, n-butyl, s-butyl, t-butyl, n-pentyl, neopentyl, n-hexyl, 2-ethylhexyl, n-heptyl, n-octyl, n-nonyl, n-decyl, 2-hexyldecyl, n-undecyl, n-dodecyl, n-tridecyl, n-tetradecyl, n-pentadecyl, n-hexadecyl, n-heptadecyl, n-octadecyl, n-nonadecyl, n-icosyl, and more preferably, a C₄₋₁₆ alkyl group such as n-butyl, n-pentyl, n-hexyl, cyclohexyl, 2-ethylhexyl, n-heptyl, n-octyl, n-nonyl, n-decyl, n-undecyl, n-dodecyl, n-tridecyl, 2-hexyloctyl, n-tetradecyl, n-pentadecyl, n-hexadecyl, cyclohexyl and cycloheptyl.

Examples of the substituent on the C₄₋₃₀ alkyl group include a halogen atom and a C₁₋₃₀ alkoxy group.
Examples of the halogen atom include a fluorine atom, a chlorine atom and a bromine atom.
Examples of the C₁₋₃₀ alkoxy group include methoxy, ethoxy, n-propoxy, n-butoxy, n-pentyloxy, n-hexyloxy, n-heptyloxy, n-octyloxy, n-nonyloxy, n-decyloxy, n-undecyloxy, n-dodecyloxy, n-tridecyloxy, n-tetradecyloxy, n-pentadecyloxy, n-hexadecyloxy, n-heptadecyloxy, n-octadecyloxy, n-nonadecyloxy, n-icosyloxy, n-henicosyloxy, n-docosyloxy, n-tricosyloxy, n-tetracosyloxy, n-pentacosyloxy, n-hexacosyloxy, n-heptacosyloxy, n-octacosyloxy, n-nonacosyloxy and n-triacontyloxy.

A fluorine atom is preferable as a substituent on the C₄₋₃₀ alkyl group.
Examples of the fluorine atom-substituted C₄₋₃₀ alkyl group include perfluorohexyl, perfluorooctyl, perfluorodecyl, perfluorododecyl and perfluorotridecyl.

Examples of the "C₄₋₃₀ alkoxy group" in the "optionally substituted C₄₋₃₀ alkoxy group" in R¹ and R² include n-butoxy, n-pentyloxy, n-hexyloxy, n-heptyloxy, n-octyloxy, n-nonyloxy, n-decyloxy, n-undecyloxy, n-dodecyloxy, n-tridecyloxy, n-tetradecyloxy, n-pentadecyloxy, n-hexadecyloxy, n-heptadecyloxy, n-octadecyloxy, n-nonadecyloxy, n-icosyloxy, n-henicosyloxy, n-docosyloxy, n-tricosyloxy, n-tetracosyloxy, n-pentacosyloxy, n-hexacosyloxy, n-heptacosyloxy, n-octacosyloxy, n-nonacosyloxy and n-triacontyloxy. Preferably, C₄₋₂₀ alkoxy groups such as n-butoxy, n-pentyloxy, n-hexyloxy, n-heptyloxy, n-octyloxy, n-nonyloxy, n-decyloxy, n-undecyloxy, n-dodecyloxy, n-tridecyloxy, n-tetradecyloxy, n-pentadecyloxy, n-hexadecyloxy, n-heptadecyloxy, n-octadecyloxy, n-nonadecyloxy and n-icosyloxy are exemplified.

Examples of the substituent in the "optionally substituted C₄₋₃₀ alkoxy group" include halogen atoms such as a fluorine atom, a chlorine atom and a bromine atom, a C₁₋₃₀ alkoxy group, a C₆₋₃₀ aryl group, a C₇₋₃₀ aralkyl group, a C₄₋₃₀ heteroaryl group and a C₅₋₃₀ heteroaralkyl group. A hydrogen atom in the substituent may be substituted by a fluorine atom. Examples of the aryl group include phenyl, 1-naphthyl and 2-naphthyl. Examples of aralkyl group include the groups represented by the following formulae: 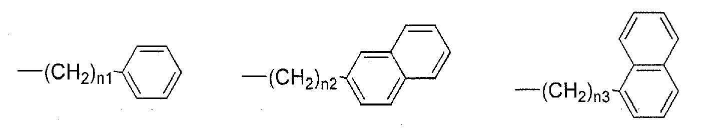 wherein n1 represents an integer from 1 to 24, and each of n2 and n3 represents an integer from 1 to 20, respectively.
The heteroaryl group means an aryl group in which at least one carbon atom among carbon atoms in the aromatic ring is replaced by a heteroatom such as a nitrogen atom, an oxygen atom, a sulfur atom or a selenium atom. Examples of the heteroaryl group include thienyl, furyl, thiazolyl, thieno[3,2-b]thienyl, furoro[3,2-b]furyl, thieno[3,2-b]furyl, benzo[b]thienyl and benzo[b]furyl. As the heteroaryl group, thienyl, thiazolyl, thieno[3,2-b]thienyl, benzo[b]thienyl and benzo[b]furyl are preferable.
The heteroaralkyl group means a group in which at least one carbon atom in the aromatic ring in the aralkyl group is substituted by a heteroatom such as a nitrogen atom, an oxygen atom, a sulfur atom and a selenium atom. Examples of the heteroaralkyl group are represented by the following formulae: 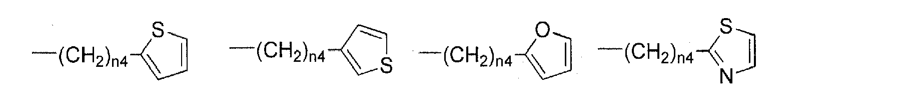 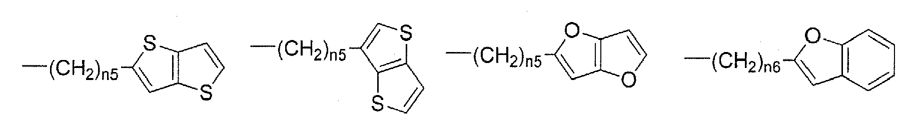 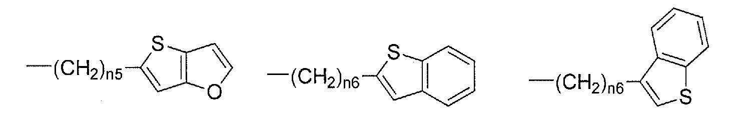 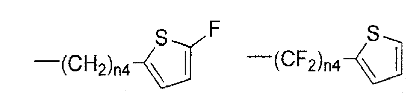 wherein n4 represents an integer from 1 to 26, n5 represents an integer from 1 to 24 and n6 represents an integer from 1 to 22.
Further preferable examples are represented by the following formulae: 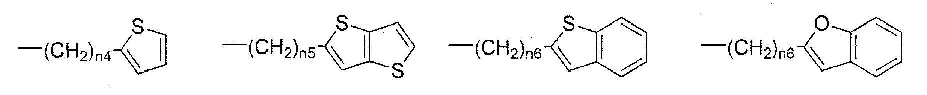 wherein n4 represents an integer from 1 to 26, n5 represents an integer from 1 to 24 and n6 represents an integer from 1 to 22.

A fluorine atom is preferable as a substituent in the C₄₋₃₀ alkoxy group. Examples of the substituted C₄₋₃₀ alkoxy group include perfluorohexyloxy, perfluorooctyloxy, perfluorodecyloxy, perfluorododecyloxy, perfluorotridecyloxy and methoxyethoxy.

The "aryl group" in the "optionally substituted C₆₋₃₀ aryl group" in R¹ and R² is, preferably, a monocyclic or bicyclic aryl group, and more preferably, phenyl, 1-naphtyl and 2-naphtyl.

Examples of the substituent in the "optionally substituted aryl group" include a halogen atom such as a fluorine atom, a chlorine atom and a bromine atom, a C₁₋₃₀ alkyl group, a C₁₋₃₀ alkoxy group, a C₆₋₃₀ aryl group, a C₇₋₃₀ aralkyl group, a C₄₋₃₀ heteroaryl group and a C₅₋₃₀ heteroaralkyl group. A hydrogen atom included in the substituent may be substituted by a fluorine atom.
Examples of the "optionally substituted aryl group" include phenyl, 1-naphtyl, 2-naphtyl, perfluorophenyl, 4-hexylphenyl and 4-hexyloxyphenyl.

Examples of the C₁₋₃₀ alkyl group include methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-icosyl, n-henicosyl, n-docosyl, n-tricosyl, n-tetracosyl, n-pentacosyl, n-hexacosyl, n-heptacosyl, n-octacosyl, n-nonacosyl and n-triacontyl.

Examples of the substituent in the "optionally substituted C₇₋₃₀ aralkyl group" in R¹ and R² include a halogen atom such as a fluorine atom, a chlorine atom and a bromine atom, a C₁₋₃₀ alkyl group, a C₁₋₃₀ alkoxy group, a C₇₋₃₀ aralkyl group, a C₄₋₃₀ heteroaryl group and a C₅₋₃₀ heteroaralkyl group. The hydrogen atom in the substituent alkyl, alkoxy, aralkyl, heteroaryl or heteroaralkyl may be substituted by a fluorine atom. As the substituent in the "optionally substituted C₇₋₃₀ aralkyl group", a fluorine atom is preferable.
Examples of the "optionally substituted C₇₋₃₀ aralkyl group" include C₇₋₃₀ aralkyl groups represented by the following formulae: 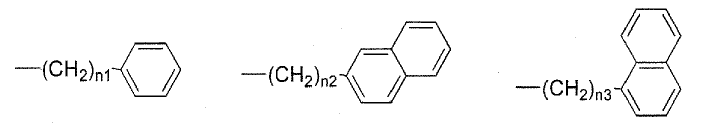 wherein n1 represents an integer from 1 to 24, and each of n2 and n3 represents an integer from 1 to 20, and
substituted C₇₋₃₀ aralkyl groups represented by the following formulae: 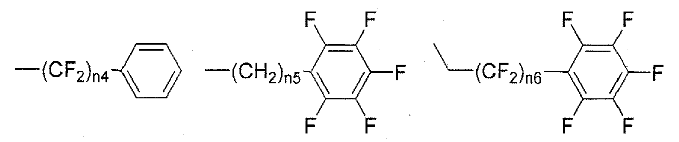 wherein each of n4 and n5 represents an integer from 1 to 24, and n6 represents an integer from 1 to 23.

Examples of the "optionally substituted C₄₋₃₀ heteroaryl group" in R¹ and R² include thienyl, furyl, thiazolyl, thieno[3,2-b]thienyl, furolo[3,2-b]furyl, thieno[3,2-b]furyl, benzo[b]thienyl and benzo[b]furyl. The heteroaryl groups are exemplified by thienyl, thiazolyl, thieno[3,2-b]thienyl, benzo[b]thienyl and benzo[b]furyl, and more preferably exemplified by heteroaryl groups represented by the following formulae:

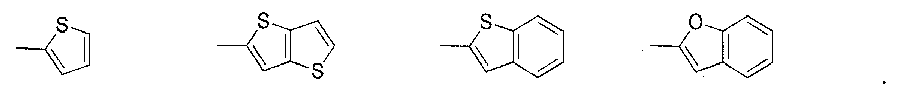

Examples of the substituent in the "optionally substituted heteroaryl group" include a halogen atom such as a fluorine atom, a chlorine atom or a bromine atom, a C₁₋₃₀ alkyl group, a C₁₋₃₀ alkoxy group, a C₆₋₃₀ aryl group, a C₇₋₃₀ aralkyl group, a C₄₋₃₀ heteroaryl group and a C₅₋₃₀ heteroaralkyl group. The hydrogen atom in the substituent may be substituted by a fluorine atom.
The "optionally substituted heteroaryl groups" are exemplified by 2-thienyl, 2-thieno[3,2-b]thienyl, 2-benzo[b]thienyl, 5-fuluoro-2-thienyl, 5-hexyl-2-thienyl and 4-hexyloxy-2-thienyl.

Examples of the substituent in the "optionally substituted C₅₋₃₀ heteroaralkyl group" include a halogen atom such as a fluorine atom, a chlorine atom and a bromine atom, a C₁₋₃₀ alkyl group, a C₁₋₃₀ alkoxy group, a C₇₋₃₀ aralkyl group, a C₄₋₃₀ heteroaryl group and a C₅₋₃₀ heteroaralkyl group. The hydrogen atom in the substituent may be substituted by a fluorine atom.
As the substituent in the "optionally substituted C₅₋₃₀ heteroaralkyl group", a fluorine atom is preferable.
Examples of the "optionally substituted C₅₋₃₀ heteroaralkyl group" include heteroaralkyl groups represented by the following formulae: 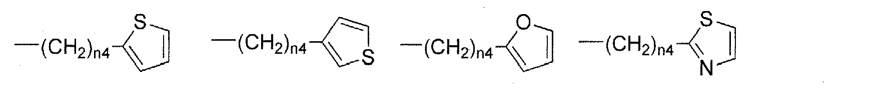 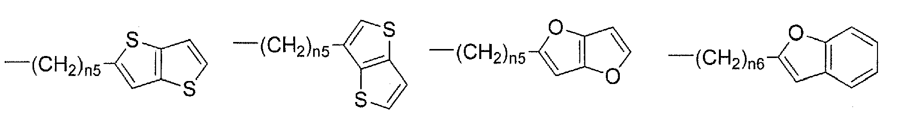 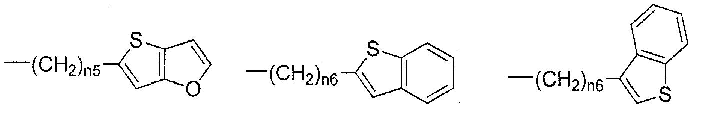 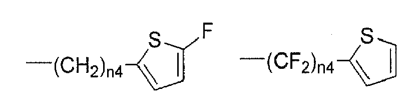 wherein n4 represents an integer from 1 to 26, n5 represents an integer from 1 to 24 and n6 represents an integer from 1 to 22.

The trialkylsilyl group in the "optionally fluorine atom-substituted C₃₋₃₀ trialkylsilyl group" in R¹ and R² is a silyl group in which the sum of the carbon atoms of alkyl groups connected to the silicon atom is 3 to 30. The maximum number of the carbon atoms in one alkyl group connected to the silicon atom is 28 and the alkyl group is an optionally fluorine atom-substituted C₁₋₃₀ alkyl group. And, the fluorine atom-substituted trialkylsilyl group means that a part or all of hydrogen atoms in the alkyl groups connected to the silicon atom are substituted by fluorine atoms. Specific examples of the trialkylsilyl group are trimethylsilyl, triethylsilyl, tri(i-propyl)silyl, t-butyldimethylsilyl, dimethylhexylsilyl and dimethyldodecylsilyl.

The bonding positions of R¹ and R² included in the substituted benzochalcogenoacene compound (1) of the present invention are preferably symmetrical. The symmetrical positions here can be illustrated using the following formula: 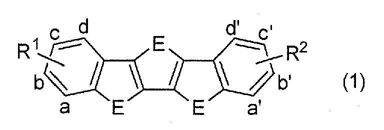 wherein E, R¹ and R² have the same meanings as described above; that is, the symmetrical positions are explained as the cases in which R¹ is connected to a and R² is connected to a', R¹ is connected to b and R² is connected to b', R¹ is connected to c and R² is connected to c', and R¹ is connected to d and R² is connected to d'. Preferable case is exemplified by the case in which R¹ is connected to b and R² is connected to b', that is, a preferable compound is represented by the formula (2): 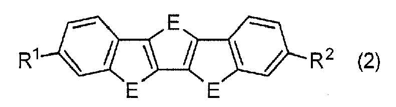 wherein E, R¹ and R² have the same meanings as described above, or the case in which R¹ is connected to c and R² is connected to c', that is, a preferable compound is represented by the formula (3): 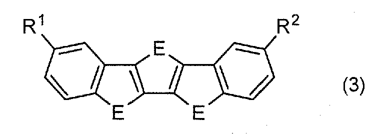 wherein E, R¹ and R² have the same meanings as described above.

In particular, the following compounds are included in the compound of the present invention:
a compound represented by the formula (1) wherein all E are sulfur atoms;
a compound represented by the formula (1) wherein each of R¹ and R² independently represents a hydrogen atom, an optionally fluorinated C₄₋₃₀ alkyl group, an optionally fluorinated C₄₋₃₀ alkoxy group, an optionally alkylated or alkoxylated C₆₋₃₀ aryl group which is optionally fluorinated, an optionally fluorinated C₇₋₃₀ aralkyl group, an optionally alkylated or alkoxylated C₄₋₃₀ heteroaryl group which is optionally fluorinated, or an optionally fluorinated C₅₋₃₀ heteroaralkyl group, wherein at least any one of R¹ and R² is not a hydrogen atom;
a compound represented by the formula (1) wherein each of R¹ and R² independently represents an optionally fluorinated C₄₋₃₀ alkyl group, an optionally fluorinated C₄₋₃₀ alkoxy group or an optionally alkylated or alkoxylated C₆₋₃₀ aryl group which is optionally fluorinated;
a compound represented by the formula (1) wherein each of R¹ and R² independently represents a C₄₋₃₀ alkyl group or a C₃₋₃₀ trialkylsilyl group;
a compound represented by the formula (1) wherein each of R¹ and R² independently represents a C₄₋₃₀ alkyl group;
a compound represented by the formula (1) wherein R¹ and R² are the same and represent C₄₋₂₀ alkyl groups;
a compound represented by the formula (1) wherein each of R¹ and R² independently represents a C₆₋₁₂ alkyl group;
a compound represented by the formula (1) wherein R¹ and R² are the same and represent C₄₋₂₀ alkoxy groups;
a compound represented by the formula (1) wherein R¹ and R² are the same and represent C₆₋₁₀ aryl groups having C₄₋₂₀ alkyl groups;
a compound represented by the formula (1) wherein R¹ and R² are the same and represent C₇₋₂₀ aralkyl groups;
a compound represented by the formula (1) wherein each of R¹ and R² independently represents a C₃₋₃₀ trialkylsilyl group;
a compound represented by the formula (1) wherein each of R¹ and R2 independently represents a C₃₋₁₄ trialkylsilyl group;
a compound represented by the formula (1) wherein each of R¹ and R² independently represents hexyl or dodecyl;
a compound represented by the formula (2) wherein each E independently represents a sulfur or selenium atom, and each of R¹ and R² independently represents a hydrogen atom, a C₄₋₃₀ alkyl group, a C₄₋₃₀ alkoxy group, an optionally alkylated or alkoxylated C₆₋₃₀ aryl group, an optionally fluorinated C₇₋₃₀ aralkyl group, an optionally alkylated or alkoxylated C₄₋₃₀ heteroaryl group which is optionally fluorinated or an optionally fluorinated C₅₋₃₀ heteroaralkyl group, wherein at least one of R¹ and R² is not a hydrogen atom;
a compound represented by the formula (2) wherein each E independently represents a sulfur or selenium atom, and each of R¹ and R² independently represents a hydrogen atom, an optionally fluorinated C₄₋₃₀ alkyl group, or an optionally fluorinated C₃₋₃₀ trialkylsilyl group;
a compound represented by the formula (2) wherein all E's represent sulfur atoms;
a compound represented by the formula (1) wherein, among three E's, two of E's represent sulfur atoms and one of E's represents a selenium atom;
a compound represented by the formula (2) wherein each of R¹ and R² independently represents a C₄₋₃₀ alkyl group or a C₃₋₃₀ trialkylsilyl group;
a compound represented by the formula (2) wherein R¹ and R² represent C₄₋₃₀ alkyl groups;
a compound represented by the formula (2) wherein R¹ and R² are the same and represent C₄₋₂₀ alkyl groups;
a compound represented by the formula (2) wherein R¹ and R² represent C₆₋₁₂ alkyl groups;
a compound represented by the formula (2) wherein all E's represent sulfur atoms, and each of R¹ and R² independently represents a C₆₋₁₂ alkyl group; a compound represented by the formula (2) wherein R¹ and R² are the same and represent C₆₋₁₀ aryl groups having C₁₋₂₀ alkyl groups;
a compound represented by the formula (2) wherein R¹ and R² are the same and represent C₇₋₂₀ aralkyl groups;
a compound represented by the formula (2) wherein each of R¹ and R2 independently represents a C₃₋₃₀ trialkylsilyl group;
a compound represented by the formula (2) wherein each of R¹ and R2 independently represents a C₃₋₁₄ trialkylsilyl group;
a compound represented by the formula (2) wherein R¹ and R² are the same and represent hexyl or dodecyl;
a compound represented by the formula (2) wherein all E's are sulfur atoms, and R¹ and R² are hexyl;
a compound represented by the formula (2) wherein all E's are sulfur atoms, and R¹ and R² are hexyl; and
a compound represented by the formula (3) wherein R¹ and R² are the same and represent C₄₋₂₀ aralkyl groups;

Specific Examples of the substituted benzochalcogenoacene compound (1) are shown in the following tables:

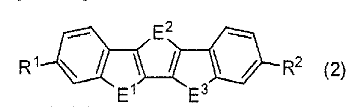

| Table 1 | | | | | |
|---|---|---|---|---|---|
| Compound No. | E¹ | E² | E³ | R¹ | R² |
| 1 | S | S | S | n-C₄H₉ | n-C₄H₉ |
| 2 | S | S | S | s-C₄H₉ | s-C₄H₉ |
| 3 | S | S | S | n-C₅H₁₁ | n-C₅H₁₁ |
| 4 | S | S | S | 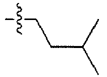 | 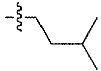 |
| 5 | S | S | S | n-C₆H₁₃ | n-C₆H₁₃ |
| 6 | S | S | S | 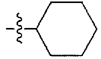 | 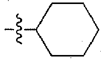 |
| 7 | S | S | S | 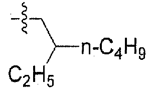 | 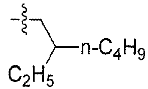 |
| 8 | S | S | S | n-C₇H₁₅ | n-C₇H₁₅ |
| 9 | S | S | S | n-C₈H₁₇ | n-C₈H₁₇ |
| 10 | S | S | S | n-C₉H₁₉ | n-C₉H₁₉ |
| 11 | S | S | S | n-C₁₀H₂₁ | n-C₁₀H₂₁ |
| 12 | S | S | S | 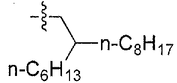 | 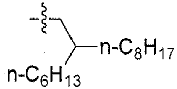 |
| 13 | S | S | S | n-C₁₁H₂₃ | n-C₁₁H₂₃ |
| 14 | S | S | S | n-C₁₂H₂₅ | n-C₁₂H₂₅ |
| 15 | S | S | S | n-C₁₃H₂₇ | n-C₁₃H₂₇ |

| | | | | | |
|---|---|---|---|---|---|
| Dashed line indicates a chemical bond. | | | | | |

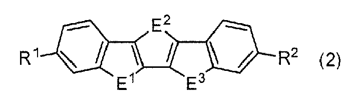

| Table 2 | | | | | |
|---|---|---|---|---|---|
| Compound No. | E¹ | E² | E³ | R¹ | R² |
| 16 | S | S | S | n-C₁₄H₂₉ | n-C₁₄H₂₉ |
| 17 | S | S | S | n-C₁₅H₃₁ | n-C₁₅H₃₁ |
| 18 | S | S | S | n-C₁₆H₃₃ | n-C₁₆H₃₃ |
| 19 | S | S | S | n-C₁₇H₃₅ | n-C₁₇H₃₅ |
| 20 | S | S | S | n-C₁₈H₃₇ | n-C₁₈H₃₇ |
| 21 | S | S | S | n-C₁₉H₃₉ | n-C₁₉H₃₉ |
| 22 | S | S | S | n-C₂₀H₄₁ | n-C₂₀H₄₁ |
| 23 | S | S | S | n-C₂₁H₄₃ | n-C₂₁H₄₃ |
| 24 | S | S | S | n-C₂₂H₄₅ | n-C₂₂H₄₅ |
| 25 | S | S | S | n-C₂₃H₄₇ | n-C₂₃H₄₇ |
| 26 | S | S | S | n-C₂₄H₄₉ | n-C₂₄H₄₉ |
| 27 | S | S | S | n-C₂₅H₅₁ | n-C₂₅H₅₁ |
| 28 | S | S | S | n-C₂₆H₅₃ | n-C₂₆H₅₃ |
| 29 | S | S | S | n-C₂₇H₅₅ | n-C₂₇H₅₅ |
| 30 | S | S | S | n-C₂₈H₅₇ | n-C₂₈H₅₇ |
| 31 | S | S | S | n-C₂₉H₅₉ | n-C₂₉H₅₉ |
| 32 | S | S | S | n-C₃₀H₆₁ | n-C₃₀H₆₁ |
| 33 | S | S | S | n-C₆F₁₃ | n-C₆F₁₃ |
| 34 | S | S | S | n-C₈F₁₇ | n-C₈F₁₇ |
| 35 | S | S | S | n-C₁₂F₂₅ | n-C₁₂F₂₅ |
| 36 | S | Se | S | n-C₆H₁₃ | n-C₆H₁₃ |
| 37 | Se | S | Se | n-C₈H₁₇ | n-C₈H₁₇ |
| 38 | S | S | S | n-C₆H₁₃ | H |
| 39 | S | S | S | n-C₁₂H₂₅ | H |
| 40 | S | S | S | O(n-C₄H₉) | O(n-C₄H₉) |
| 41 | S | S | S | O(n-C₅H₁₁) | O(n-C₅H₁₁) |
| 42 | S | S | S | 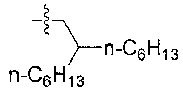 | 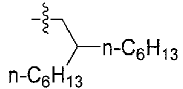 |
| 43 | S | S | S | O(n-C₆H₁₃) | O(n-C₆H₁₃) |
| 44 | Se | Se | Se | n-C₆H₁₃ | n-C₆H₁₃ |
| 46 | S | S | S | O(n-C₇H₁₅) | O(n-C₇H₁₅) |
| 47 | S | S | S | O(n-C₈H₁₇) | O(n-C₈H₁₇) |
| 48 | S | S | S | O(n-C₉H₁₉) | O(n-C₉H₁₉) |
| 49 | S | S | S | O(n-C₁₀H₂₁) | O(n-C₁₀H₂₁) |

| | | | | | |
|---|---|---|---|---|---|
| Dashed line indicates a chemical bond. | | | | | |

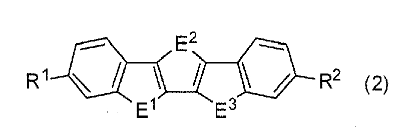

| Table 3 | | | | | |
|---|---|---|---|---|---|
| Compound No. | E¹ | E² | E³ | R¹ | R² |
| 51 | S | S | S | O(n-C₁₁H₂₃) | O(n-C₁₁H₂₃) |
| 52 | S | S | S | O(n-C₁₂H₂₅) | O(n-C₁₂H₂₅) |
| 53 | S | S | S | O(n-C₁₃H₂₇) | O(n-C₁₃H₂₇) |
| 54 | S | S | S | O(n-C₁₄H₂₉) | O(n-C₁₄H₂₉) |
| 55 | S | S | S | O(n-C₁₅H₃₁) | O(n-C₁₅H₃₁) |
| 56 | S | S | S | O(n-C₁₆H₃₃) | O(n-C₁₆H₃₃) |
| 57 | S | S | S | O(n-C₁₇H₃₅) | O(n-C₁₇H₃₅) |
| 58 | S | S | S | O(n-C₁₈H₃₇) | O(n-C₁₈H₃₇) |
| 59 | S | S | S | O(n-C₁₉H₃₉) | O(n-C₁₉H₃₉) |
| 60 | S | S | S | O(n-C₂₀H₄₁) | O(n-C₂₀H₄₁) |
| 61 | S | S | S | O(n-C₂₁H₄₃) | O(n-C₂₁H₄₃) |
| 62 | S | S | S | O(n-C₂₂H₄₅) | O(n-C₂₂H₄₅) |
| 63 | S | S | S | O(n-C₂₃H₄₇) | O(n-C₂₃H₄₇) |
| 64 | S | S | S | O(n-C₂₄H₄₉) | O(n-C₂₄H₄₉) |
| 65 | S | S | S | O(n-C₂₅H₅₁) | O(n-C₂₅H₅₁) |
| 66 | S | S | S | O(n-C₂₆H₅₃) | O(n-C₂₆H₅₃) |
| 67 | S | S | S | O(n-C₂₇H₅₅) | O(n-C₂₇H₅₅) |
| 68 | S | S | S | O(n-C₂₈H₅₇) | O(n-C₂₈H₅₇) |
| 69 | S | S | S | O(n-C₂₉H₅₉) | O(n-C₂₉H₅₉) |
| 70 | S | S | S | O(n-C₃₀H₆₁) | O(n-C₃₀H₆₁) |
| 71 | S | S | S | O(n-C₆F₁₃) | O(n-C₆F₁₃) |
| 72 | S | S | S | O(n-C₈F₁₇) | O(n-C₈F₁₇) |
| 73 | S | S | S | O(n-C₁₂F₂₅) | O(n-C₁₂F₂₅) |
| 74 | S | Se | S | O(n-C₆H₁₃) | O(n-C₆H₁₃) |
| 75 | Se | S | Se | O(n-C₈H₁₇) | O(n-C₈H₁₇) |
| 76 | S | S | S | O(n-C₈H₁₇) | H |
| 77 | S | S | S | O(n-C₈H₁₇) | O(n-C₁₂H₂₅) |
| 78 | S | S | S | 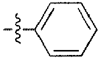 | 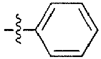 |
| 79 | S | S | S | 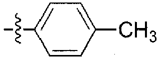 | 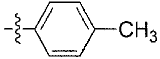 |

| | | | | | |
|---|---|---|---|---|---|
| Dashed line indicates a chemical bond. | | | | | |

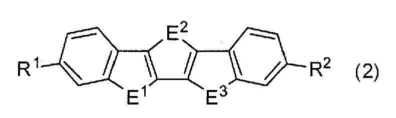

| Table 4 | | | | | |
|---|---|---|---|---|---|
| Compound No. | E¹ | E² | E³ | R¹ | R² |
| 80 | S | S | S | 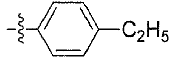 | 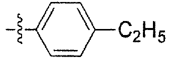 |
| 81 | S | S | S | 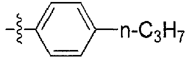 | 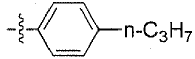 |
| 82 | S | S | S | 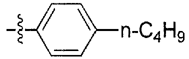 | 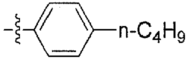 |
| 84 | S | S | S | 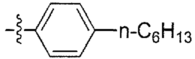 | 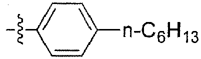 |
| 86 | S | S | S | 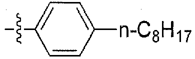 | 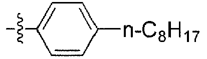 |
| 87 | S | S | S | 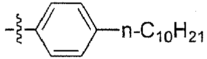 | 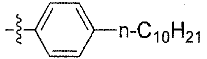 |
| 89 | S | S | S | 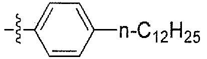 | 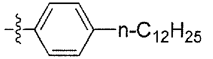 |
| 90 | S | S | S | 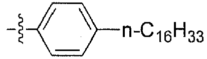 | 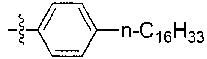 |
| 91 | S | S | S | 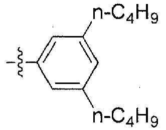 | 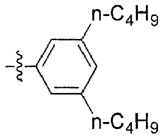 |
| 93 | S | S | S | 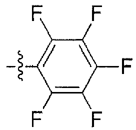 | 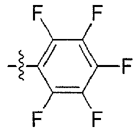 |
| 94 | S | S | S | 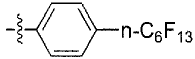 | 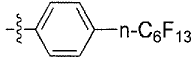 |
| 95 | S | S | S | 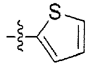 | 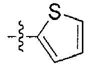 |

| | | | | | |
|---|---|---|---|---|---|
| Dashed line indicates a chemical bond. | | | | | |

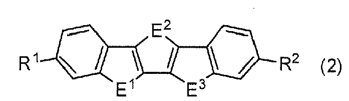

| Table 5 | | | | | |
|---|---|---|---|---|---|
| Compound No. | E¹ | E² | E³ | R¹ | R² |
| 96 | S | S | S | 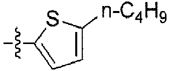 | 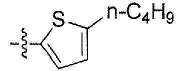 |
| 97 | S | S | S | 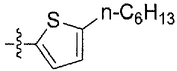 | 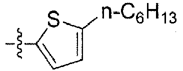 |
| 99 | S | S | S | 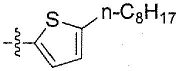 | 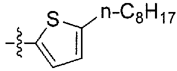 |
| 100 | S | S | S | 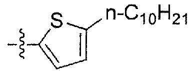 | 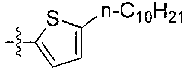 |
| 102 | S | S | S | 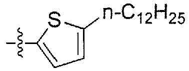 | 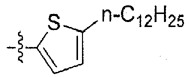 |
| 103 | S | S | S | 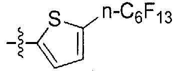 | 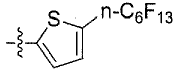 |
| 104 | S | S | S | 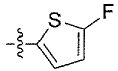 | 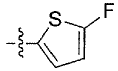 |
| 105 | S | S | S | 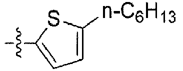 | H |
| 106 | S | S | S | 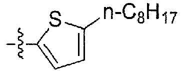 | 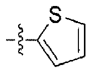 |
| 107 | S | Se | S | 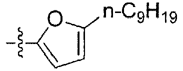 | 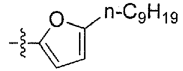 |
| 108 | S | S | S | 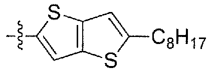 | 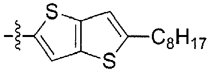 |

| | | | | | |
|---|---|---|---|---|---|
| Dashed line indicates a chemical bond. | | | | | |

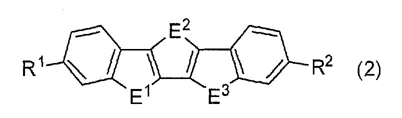

| Table 6 | | | | | |
|---|---|---|---|---|---|
| Compound No. | E¹ | E² | E³ | R¹ | R² |
| 114 | S | S | S | 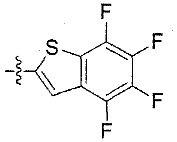 | 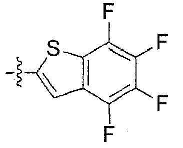 |
| 117 | S | S | S | 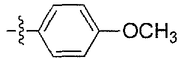 | 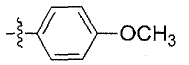 |
| 118 | S | S | S | 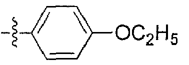 | 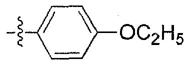 |
| 119 | S | S | S | 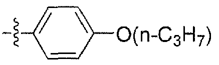 | 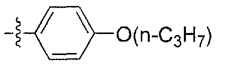 |
| 120 | S | S | S | 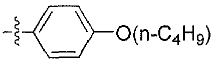 | 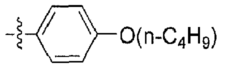 |
| 122 | S | S | S | 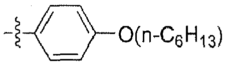 | 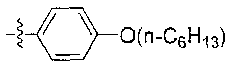 |
| 124 | S | S | S | 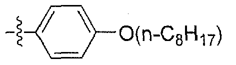 | 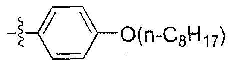 |

| | | | | | |
|---|---|---|---|---|---|
| Dashed line indicates a chemical bond. | | | | | |

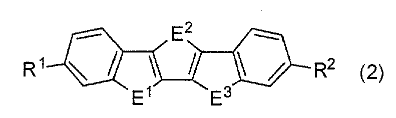

| Table 7 | | | | | |
|---|---|---|---|---|---|
| Compound No. | E¹ | E² | E³ | R¹ | R² |
| 125 | S | S | S | 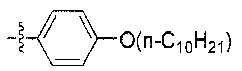 | 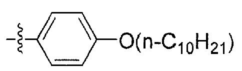 |
| 127 | S | S | S | 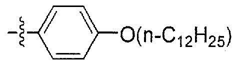 | 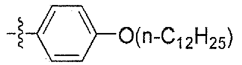 |
| 128 | S | S | S | 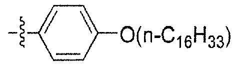 | 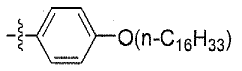 |
| 129 | S | S | S | 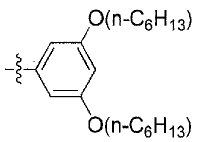 | 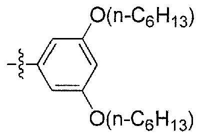 |
| 131 | S | S | S | 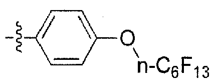 | 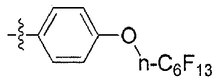 |
| 132 | S | S | S | 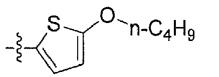 | 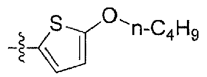 |
| 133 | S | S | S | 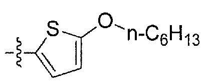 | 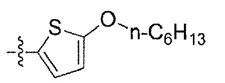 |
| 135 | S | S | S | 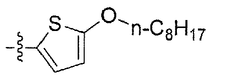 | 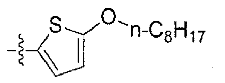 |
| 136 | S | S | S | 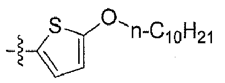 | 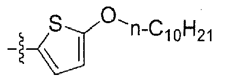 |
| 138 | S | S | S | 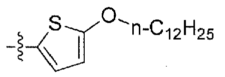 | 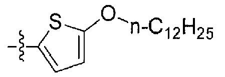 |
| 139 | S | S | S | 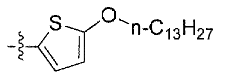 | 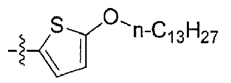 |

| | | | | | |
|---|---|---|---|---|---|
| Dashed line indicates a chemical bond. | | | | | |

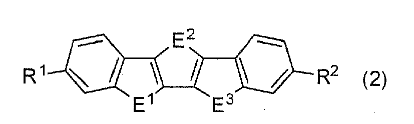

| Table 8 | | | | | |
|---|---|---|---|---|---|
| Compound No. | E¹ | E² | E³ | R¹ | R² |
| 140 | S | Se | S | 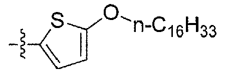 | 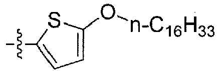 |
| 141 | Se | S | Se | 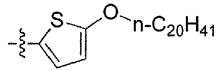 | 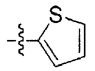 |
| 142 | S | S | S | 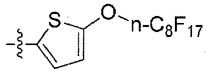 | 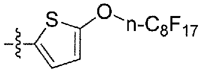 |
| 143 | S | Se | S | 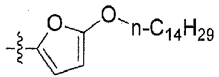 | 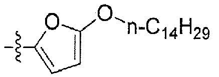 |
| 144 | S | S | S | 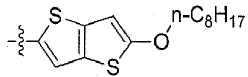 | 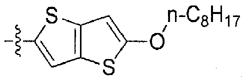 |
| 145 | S | S | S | -Si(CH₃)₂(n-C₈H₁₇) | -Si(CH₃)₂(n-C₈H₁₇) |
| 146 | S | S | S | -Si(CH₃)₂(n-C₁₀H₂₁) | -Si(CH₃)₂(n-C₁₀H₂₁) |

| | | | | | |
|---|---|---|---|---|---|
| Dashed line indicates a chemical bond. | | | | | |

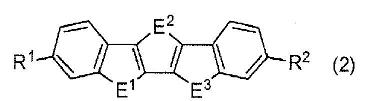

| Table 9 | | | | | |
|---|---|---|---|---|---|
| Compound No. | E¹ | E² | E³ | R¹ | R² |
| 152 | S | S | S | 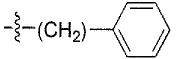 | 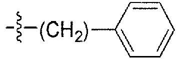 |
| 153 | S | S | S | 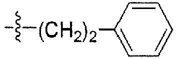 | 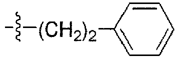 |
| 154 | S | S | S | 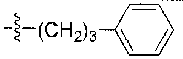 | 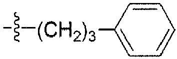 |
| 155 | S | S | S | 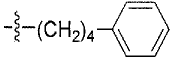 | 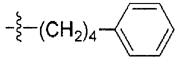 |
| 156 | S | S | S | 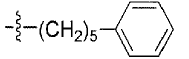 | 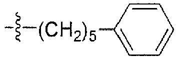 |
| 157 | S | S | S | 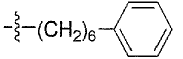 | 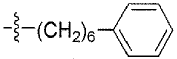 |
| 158 | S | S | S | 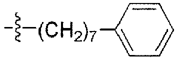 | 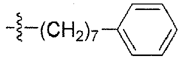 |
| 159 | S | S | S | 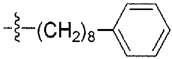 | 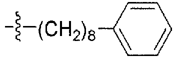 |
| 160 | S | S | S | 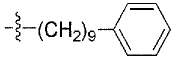 | 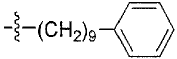 |
| 161 | S | S | S | 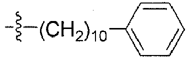 | 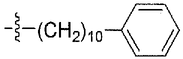 |
| 162 | S | S | S | 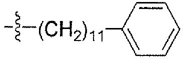 | 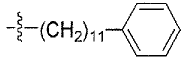 |
| 163 | S | S | S | 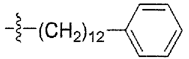 | 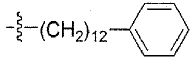 |
| 164 | S | S | S | 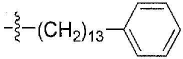 | 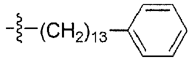 |
| 165 | S | S | S | 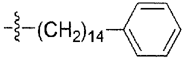 | 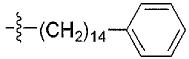 |

| | | | | | |
|---|---|---|---|---|---|
| Dashed line indicates a chemical bond. | | | | | |

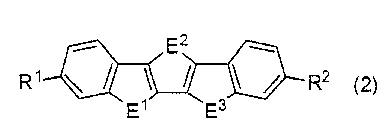

| Table 10-1 | | | | | |
|---|---|---|---|---|---|
| Compound No. | E¹ | E² | E³ | R¹ | R² |
| 166 | S | S | S | 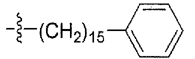 | 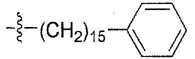 |
| 167 | S | S | S | 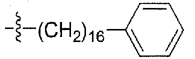 | 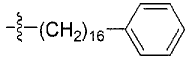 |
| 168 | S | S | S | 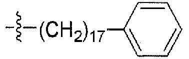 | 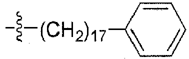 |
| 169 | S | S | S | 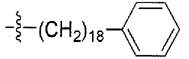 | 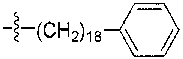 |
| 170 | S | S | S | 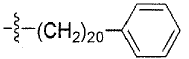 | 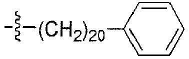 |
| 171 | S | S | S | 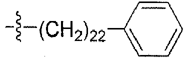 | 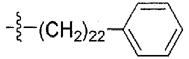 |
| 172 | S | S | S | 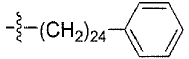 | 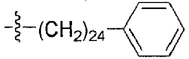 |
| 173 | S | S | S | 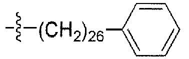 | 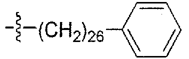 |
| 174 | S | S | S | 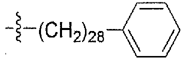 | 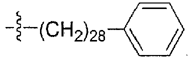 |
| 175 | S | S | S | 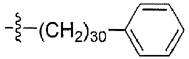 | 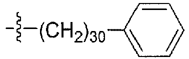 |

| | | | | | |
|---|---|---|---|---|---|
| Dashed line indicates a chemical bond. | | | | | |

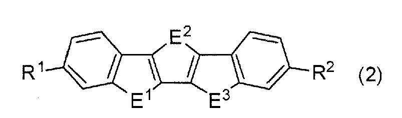

| Table 10-2 | | | | | |
|---|---|---|---|---|---|
| Compound No. | E¹ | E² | E³ | R¹ | R² |
| 176 | S | S | S | 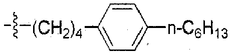 | 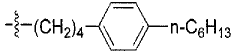 |
| 177 | S | S | S | 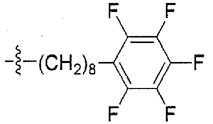 | 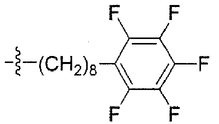 |
| 178 | S | S | S | 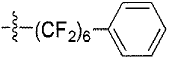 | 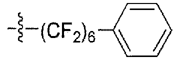 |
| 179 | Se | Se | Se | 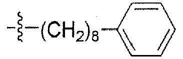 | n-C₆H₁₃ |
| 180 | S | S | S | 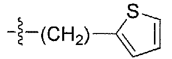 | 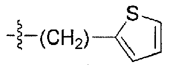 |
| 181 | S | S | S | 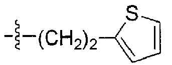 | 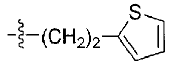 |
| 182 | S | Se | S | 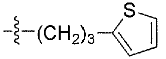 | 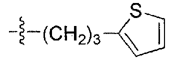 |
| 183 | S | Se | S | 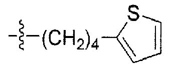 | 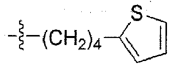 |
| 184 | S | Se | S | 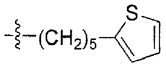 | 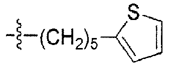 |

| | | | | | |
|---|---|---|---|---|---|
| Dashed line indicates a chemical bond. | | | | | |

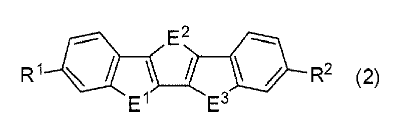

| Table 11-1 | | | | | |
|---|---|---|---|---|---|
| Compound No. | E¹ | E² | E³ | R¹ | R² |
| 185 | S | S | S | 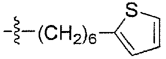 | 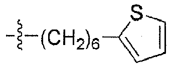 |
| 186 | S | S | S | 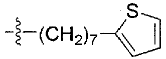 | 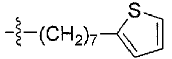 |
| 187 | S | S | S | 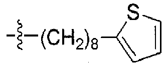 | 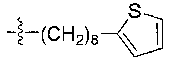 |
| 188 | S | S | S | 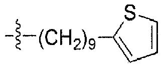 | 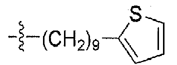 |
| 189 | S | S | S | 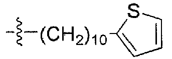 | 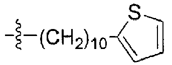 |
| 190 | S | S | S | 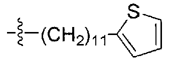 | 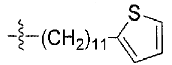 |
| 191 | S | S | S | 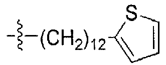 | 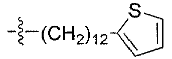 |
| 192 | S | S | S | 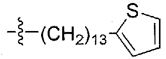 | 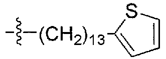 |
| 193 | S | S | S | 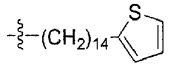 | 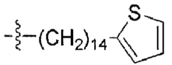 |
| 194 | S | S | S | 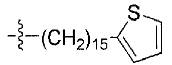 | 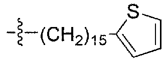 |

| | | | | | |
|---|---|---|---|---|---|
| Dashed line indicates a chemical bond. | | | | | |

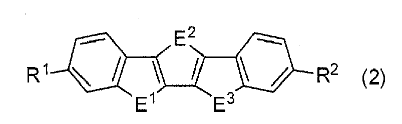

| Table 11-2 | | | | | |
|---|---|---|---|---|---|
| Compound No. | E¹ | E² | E³ | R¹ | R² |
| 195 | S | S | S | 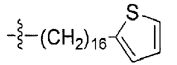 | 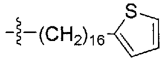 |
| 196 | S | S | S | 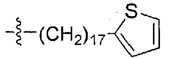 | 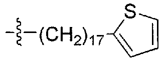 |
| 197 | S | S | S | 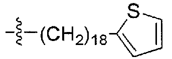 | 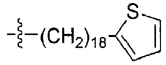 |
| 198 | S | S | S | 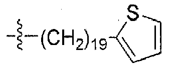 | 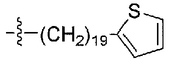 |
| 199 | S | S | S | 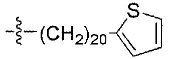 | 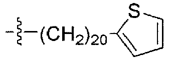 |
| 200 | S | S | S | 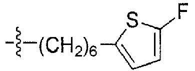 | 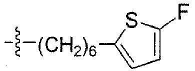 |
| 201 | S | S | S | 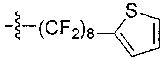 | 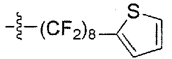 |
| 202 | S | S | S | 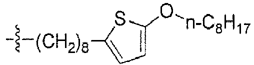 | 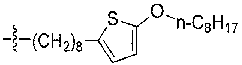 |
| 203 | S | S | S | 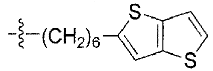 | 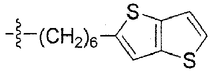 |

| | | | | | |
|---|---|---|---|---|---|
| Dashed line indicates a chemical bond. | | | | | |

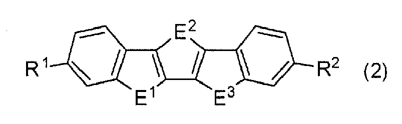

| Table 12 | | | | | |
|---|---|---|---|---|---|
| Compound No. | E¹ | E² | E³ | R¹ | R² |
| 204 | S | S | S | 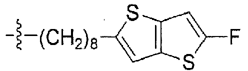 | 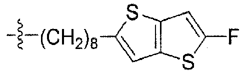 |
| 205 | S | S | S | 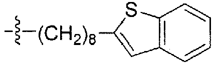 | 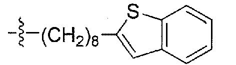 |
| 206 | Se | Se | Se | 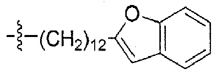 | 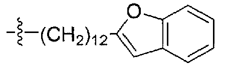 |
| 207 | S | S | S | 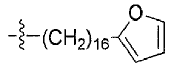 | 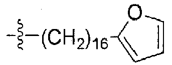 |
| 208 | S | S | S | -Si(CH₃)₃ | -Si(CH₃)₃ |
| 209 | S | S | S | -Si(C₂H₅)₃ | -Si(C₂H₅)₃ |
| 210 | S | S | S | -Si(i-C₃H₇)₃ | -Si(i-C₃H₇)₃ |
| 211 | S | S | S | -Si(CH₃)₂(t-C₄H₉) | -Si(CH₃)₂(t-C₄H₉) |
| 212 | S | S | S | -Si(CH₃)₂(n-C₆H₁₃) | -Si(CH₃)₂(n-C₆H₁₃) |
| 213 | S | S | S | -Si(CH₃)₂(n-C₁₂H₂₅) | -Si(CH₃)₂(n-C₁₂H₂₅) |

| | | | | | |
|---|---|---|---|---|---|
| Dashed line indicates a chemical bond. | | | | | |

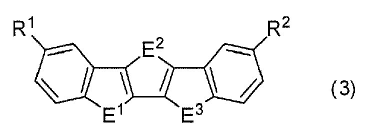

| Table 13 | | | | | |
|---|---|---|---|---|---|
| Compound No. | E¹ | E² | E³ | R¹ | R² |
| 214 | S | S | S | n-C₄H₉ | n-C₄H₉ |
| 215 | S | S | S | s-C₄H₉ | s-C₄H₉ |
| 216 | S | S | S | n-C₅H₁₁ | n-C₅H₁₁ |
| 217 | S | S | S | 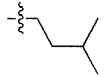 | 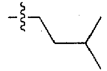 |
| 218 | S | S | S | n-C₆H₁₃ | n-C₆H₁₃ |
| 219 | S | S | S | 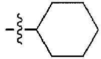 | 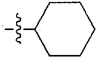 |
| 220 | S | S | S | 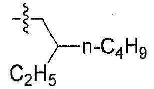 | 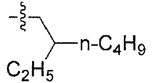 |
| 221 | S | S | S | n-C₇H₁₅ | n-C₇H₁₅ |
| 222 | S | S | S | n-C₈H₁₇ | n-C₈H₁₇ |
| 223 | S | S | S | n-C₉H₁₉ | n-C₉H₁₉ |
| 224 | S | S | S | n-C₁₀H₂₁ | n-C₁₀H₂₁ |

| | | | | | |
|---|---|---|---|---|---|
| Dashed line indicates a chemical bond. | | | | | |

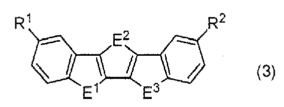

| Table 14 | | | | | |
|---|---|---|---|---|---|
| Compound No. | E¹ | E² | E³ | R¹ | R² |
| 225 | S | S | S | 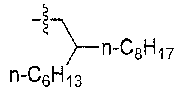 | 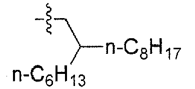 |
| 226 | S | S | S | n-C₁₁H₂₃ | n-C₁₁H₂₃ |
| 227 | S | S | S | n-C₁₂H₂₅ | n-C₁₂H₂₅ |
| 228 | S | S | S | n-C₁₃H₂₇ | n-C₁₃H₂₇ |
| 229 | S | S | S | n-C₁₄H₂₉ | n-C₁₄H₂₉ |
| 230 | S | S | S | n-C₁₅H₃₁ | n-C₁₅H₃₁ |
| 231 | S | S | S | n-C₁₆H₃₃ | n-C₁₆H₃₃ |
| 232 | S | S | S | n-C₁₇H₃₅ | n-C₁₇H₃₅ |
| 233 | S | S | S | n-C₁₈H₃₇ | n-C₁₈H₃₇ |
| 234 | S | S | S | n-C₁₉H₃₉ | n-C₁₉H₃₉ |
| 235 | S | S | S | n-C₂₀H₄₁ | n-C₂₀H₄₁ |
| 236 | S | S | S | n-C₂₁H₄₃ | n-C₂₁H₄₃ |
| 237 | S | S | S | n-C₂₂H₄₅ | n-C₂₂H₄₅ |
| 238 | S | S | S | n-C₂₃H₄₇ | n-C₂₃H₄₇ |
| 239 | S | S | S | n-C₂₄H₄₉ | n-C₂₄H₄₉ |
| 240 | S | S | S | n-C₂₅H₅₁ | n-C₂₅H₅₁ |
| 241 | S | S | S | n-C₂₆H₅₃ | n-C₂₆H₅₃ |
| 242 | S | S | S | n-C₂₇H₅₅ | n-C₂₇H₅₅ |
| 243 | S | S | S | n-C₂₈H₅₇ | n-C₂₈H₅₇ |
| 244 | S | S | S | n-C₂₉H₅₉ | n-C₂₉H₅₉ |
| 245 | S | S | S | n-C₃₀H₆₁ | n-C₃₀H₆₁ |
| 246 | S | S | S | n-C₆F₁₃ | n-C₆F₁₃ |
| 247 | S | S | S | n-C₈F₁₇ | n-C₈F₁₇ |
| 248 | S | S | S | n-C₁₂F₂₅ | n-C₁₂F₂₅ |
| 249 | S | S | S | n-C₁₆F₃₃ | n-C₁₆F₃₃ |
| 250 | S | Se | S | n-C₆H₁₃ | n-C₆H₁₃ |
| 251 | Se | S | Se | n-C₈H₁₇ | n-C₈H₁₇ |
| 252 | S | S | S | n-C₈H₁₇ | H |
| 253 | S | S | S | O(n-C₄H₉) | O(n-C₄H₉) |
| 254 | S | S | S | O(n-C₅H₁₁) | O(n-C₅H₁₁) |

| | | | | | |
|---|---|---|---|---|---|
| Dashed line indicates a chemical bond. | | | | | |

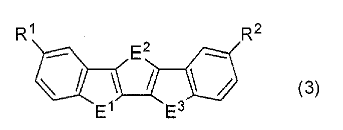

| Table 15 | | | | | |
|---|---|---|---|---|---|
| Compound No. | E¹ | E² | E³ | R¹ | R² |
| 256 | S | S | S | O(n-C₆H₁₃) | O(n-C₆H₁₃) |
| 259 | S | S | S | O(n-C₇H₁₅) | O(n-C₇H₁₅) |
| 260 | S | S | S | O(n-C₈H₁₇) | O(n-C₈H₁₇) |
| 261 | S | S | S | O(n-C₉H₁₉) | O(n-C₉H₁₉) |
| 262 | S | S | S | O(n-C₁₀H₂₁) | O(n-C₁₀H₂₁) |
| 264 | S | S | S | O(n-C₁₁H₂₃) | O(n-C₁₁H₂₃) |
| 265 | S | S | S | O(n-C₁₂H₂₅) | O(n-C₁₂H₂₅) |
| 266 | S | S | S | O(n-C₁₃H₂₇) | O(n-C₁₃H₂₇) |
| 267 | S | S | S | O(n-C₁₄H₂₉) | O(n-C₁₄H₂₉) |
| 268 | S | S | S | O(n-C₁₅H₃₁) | O(n-C₁₅H₃₁) |
| 269 | S | S | S | O(n-C₁₆H₃₃) | O(n-C₁₆H₃₃) |
| 270 | S | S | S | O(n-C₁₇H₃₅) | O(n-C₁₇H₃₅) |
| 271 | S | S | S | O(n-C₁₈H₃₇) | O(n-C₁₈H₃₇) |
| 272 | S | S | S | O(n-C₁₉H₃₉) | O(n-C₁₉H₃₉) |
| 273 | S | S | S | O(n-C₂₀H₄₁) | O(n-C₂₀H₄₁) |
| 274 | S | S | S | O(n-C₂₁H₄₃) | O(n-C₂₁H₄₃) |
| 275 | S | S | S | O(n-C₂₂H₄₅) | O(n-C₂₂H₄₅) |
| 276 | S | S | S | O(n-C₂₃H₄₇) | O(n-C₂₃H₄₇) |
| 277 | S | S | S | O(n-C₂₄H₄₉) | O(n-C₂₄H₄₉) |
| 278 | S | S | S | O(n-C₂₅H₅₁) | O(n-C₂₅H₅₁) |
| 279 | S | S | S | O(n-C₂₆H₅₃) | O(n-C₂₆H₅₃) |
| 280 | S | S | S | O(n-C₂₇H₅₅) | O(n-C₂₇H₅₅) |
| 281 | S | S | S | O(n-C₂₈H₅₇) | O(n-C₂₈H₅₇) |
| 282 | S | S | S | O(n-C₂₉H₅₉) | O(n-C₂₉H₅₉) |
| 283 | S | S | S | O(n-C₃₀H₆₁) | O(n-C₃₀H₆₁) |
| 284 | S | S | S | O(n-C₆F₁₃) | O(n-C₆F₁₃) |
| 285 | S | S | S | O(n-C₈F₁₇) | O(n-C₈F₁₇) |
| 286 | S | S | S | O(n-C₁₂F₂₅) | O(n-C₁₂F₂₅) |
| 287 | S | S | S | O(n-C₁₆F₃₃) | O(n-C₁₆F₃₃) |
| 288 | S | Se | S | O(n-C₆H₁₃) | O(n-C₆H₁₃) |
| 289 | Se | S | Se | O(n-C₈H₁₇) | O(n-C₈H₁₇) |

| | | | | | |
|---|---|---|---|---|---|
| Dashed line indicates a chemical bond. | | | | | |

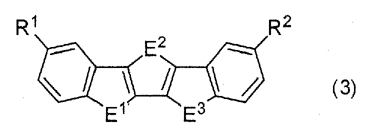

| Table 16 | | | | | |
|---|---|---|---|---|---|
| Compound No. | E¹ | E² | E³ | R¹ | R² |
| 290 | S | S | S | O(n-C₈H₁₇) | O(n-C₁₂H₂₅) |
| 291 | S | S | S | 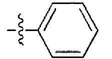 | 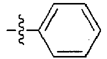 |
| 292 | S | S | S | 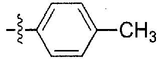 | 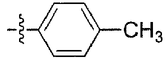 |
| 293 | S | S | S | 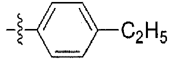 | 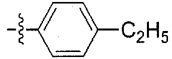 |
| 294 | S | S | S | 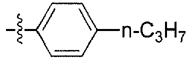 | 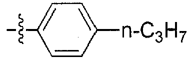 |
| 295 | S | S | S | 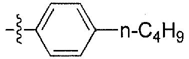 | 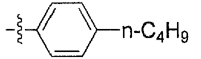 |
| 297 | S | S | S | 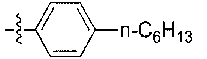 | 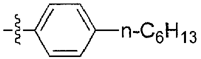 |
| 299 | S | S | S | 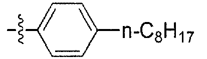 | 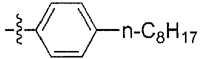 |
| 300 | S | S | S | 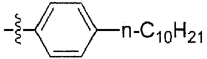 | 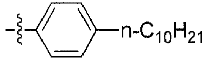 |
| 302 | S | S | S | 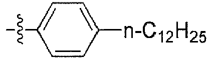 | 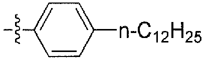 |
| 303 | S | S | S | 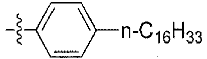 | 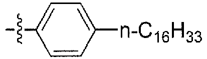 |
| 304 | S | S | S | 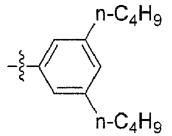 | 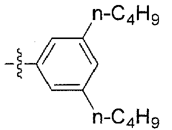 |

| | | | | | |
|---|---|---|---|---|---|
| Dashed line indicates a chemical bond. | | | | | |

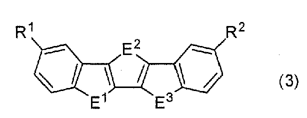

| Table 17 | | | | | |
|---|---|---|---|---|---|
| Compound No. | E¹ | E² | E³ | R¹ | R² |
| 306 | S | S | S | 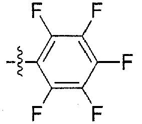 | 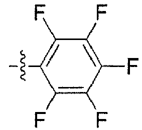 |
| 307 | S | S | S | 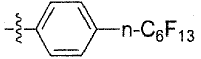 | 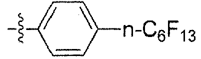 |
| 308 | S | S | S | 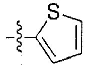 | 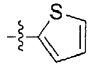 |
| 309 | S | S | S | 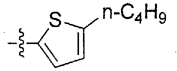 | 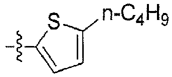 |
| 310 | S | S | S | 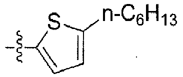 | 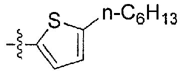 |
| 312 | S | S | S | 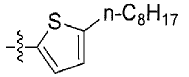 | 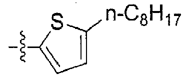 |
| 313 | S | S | S | 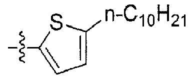 | 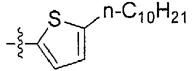 |
| 315 | S | S | S | 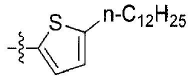 | 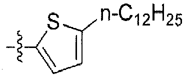 |
| 316 | S | S | S | 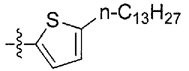 | 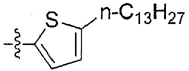 |
| 317 | S | S | S | 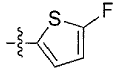 | 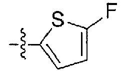 |
| 318 | S | S | S | 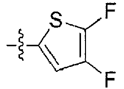 | 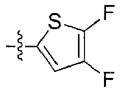 |
| 319 | S | S | S | 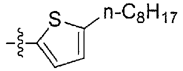 | 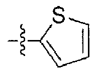 |
| 320 | S | Se | S | 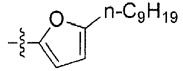 | 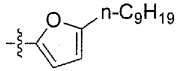 |

| | | | | | |
|---|---|---|---|---|---|
| Dashed line indicates a chemical bond. | | | | | |

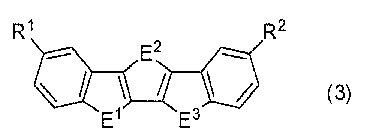

| Table 18 | | | | | |
|---|---|---|---|---|---|
| Compound No. | E¹ | E² | E³ | R¹ | R² |
| 321 | S | S | S | 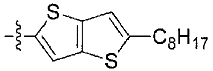 | 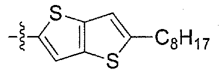 |
| 327 | S | S | S | 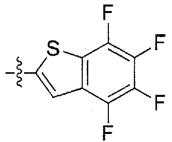 | 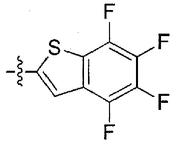 |
| 330 | S | S | S | 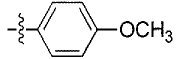 | 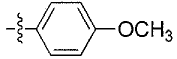 |
| 331 | S | S | S | 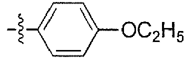 | 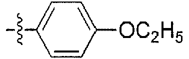 |
| 332 | S | S | S | 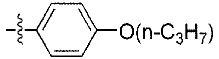 | 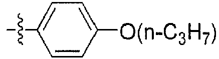 |
| 333 | S | S | S | 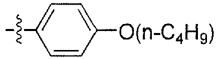 | 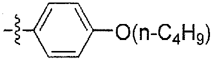 |
| 335 | S | S | S | 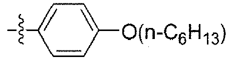 | 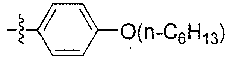 |

| | | | | | |
|---|---|---|---|---|---|
| Dashed line indicates a chemical bond. | | | | | |

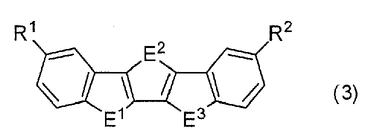

| Table 19 | | | | | |
|---|---|---|---|---|---|
| Compound No. | E¹ | E² | E³ | R¹ | R² |
| 337 | S | S | S | 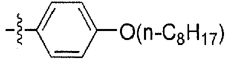 | 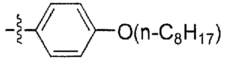 |
| 338 | S | S | S | 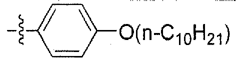 | 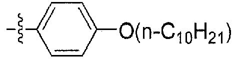 |
| 340 | S | S | S | 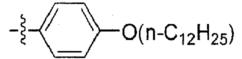 | 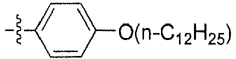 |
| 341 | S | S | S | 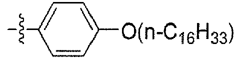 | 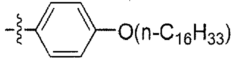 |
| 342 | S | S | S | 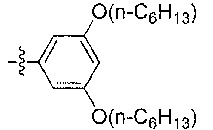 | 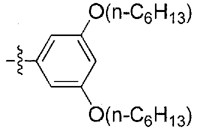 |
| 344 | S | S | S | 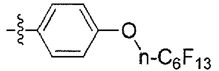 | 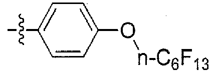 |
| 345 | S | S | S | 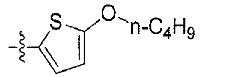 | 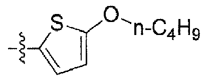 |
| 346 | S | S | S | 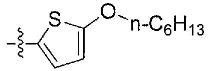 | 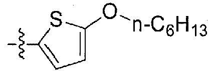 |
| 348 | S | S | S | 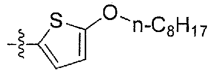 | 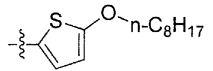 |
| 349 | S | S | S | 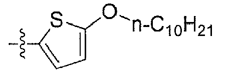 | 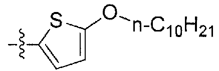 |

| | | | | | |
|---|---|---|---|---|---|
| Dashed line indicates a chemical bond. | | | | | |

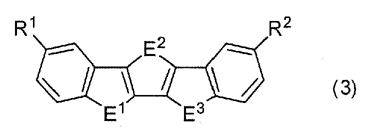

| Table 20 | | | | | |
|---|---|---|---|---|---|
| Compound No. | E¹ | E² | E³ | R¹ | R² |
| 351 | S | S | S | 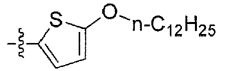 | 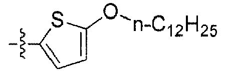 |
| 352 | S | S | S | 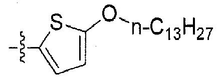 | 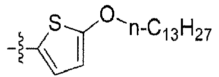 |
| 353 | S | Se | S | 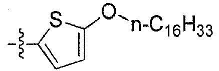 | 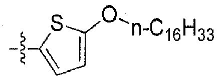 |
| 354 | Se | S | Se | 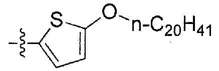 | 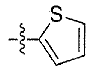 |
| 355 | S | S | S | 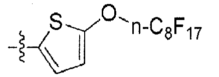 | 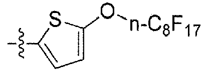 |
| 356 | S | Se | S | 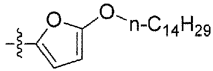 | 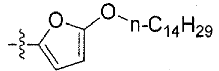 |
| 357 | S | S | S | 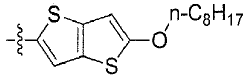 | 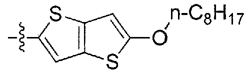 |

| | | | | | |
|---|---|---|---|---|---|
| Dashed line indicates a chemical bond. | | | | | |

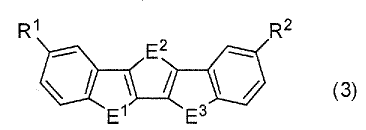

| Table 21 | | | | | |
|---|---|---|---|---|---|
| Compound No. | E¹ | E² | E³ | R¹ | R² |
| 365 | S | S | S | 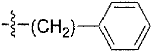 | 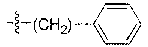 |
| 366 | S | S | S | 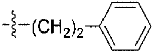 | 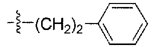 |
| 367 | S | S | S | 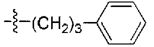 | 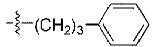 |
| 368 | S | S | S | 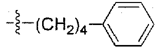 | 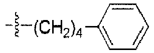 |
| 369 | S | S | S | 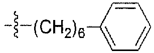 | 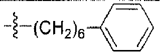 |
| 370 | S | S | S | 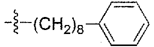 | 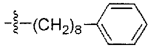 |
| 371 | S | S | S | 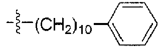 | 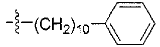 |
| 372 | S | S | S | 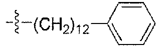 | 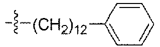 |
| 373 | S | S | S | 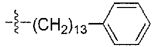 | 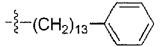 |
| 374 | S | S | S | 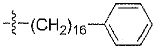 | 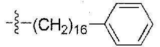 |
| 375 | S | S | S | 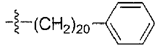 | 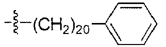 |
| 376 | S | Se | S | 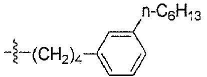 | 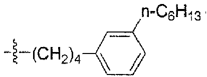 |
| 377 | S | S | S | 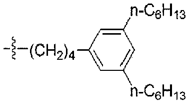 | 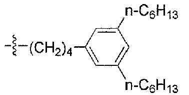 |
| 378 | S | S | S | 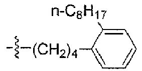 | 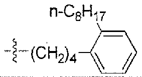 |
| 379 | S | S | S | 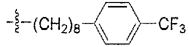 | 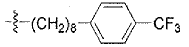 |
| 380 | Se | S | Se | 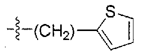 | 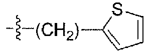 |
| 381 | S | S | S | 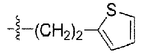 | 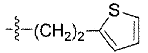 |

| | | | | | |
|---|---|---|---|---|---|
| Dashed line indicates a chemical bond. | | | | | |

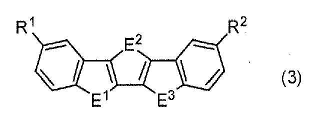

| Table 22 | | | | | |
|---|---|---|---|---|---|
| Compound No. | E¹ | E² | E³ | R¹ | R² |
| 382 | S | S | S | 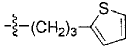 | 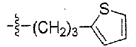 |
| 383 | S | Se | S | 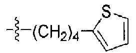 | 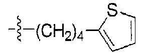 |
| 384 | S | S | S | 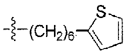 | 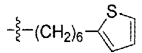 |
| 385 | S | S | S | 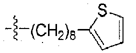 | 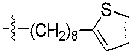 |
| 386 | S | S | S | 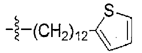 | 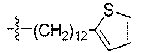 |
| 387 | S | S | S | 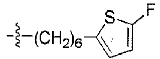 | 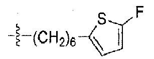 |
| 388 | S | S | S | 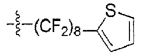 | 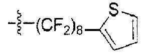 |
| 389 | S | S | S | 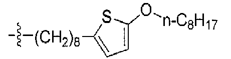 | 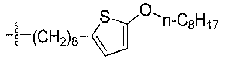 |
| 390 | S | S | S | 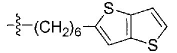 | 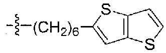 |
| 391 | S | S | S | 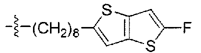 | 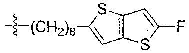 |
| 392 | S | S | S | 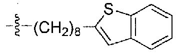 | 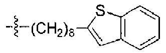 |
| 393 | Se | Se | Se | 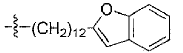 | 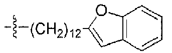 |
| 394 | S | S | S | -Si(CH₃)₃ | -Si(CH₃)₃ |
| 395 | S | S | S | -Si(C₂H₅)₃ | -Si(C₂Hs)₃ |
| 396 | S | S | S | -Si(i-C₃H₇)₃ | -Si(i-C₃H₇)₃ |
| 397 | S | S | S | -Si(CH₃)₂(t-C₄H₉) | -Si(CH₃)₂(t-C₄H₉) |
| 398 | S | S | S | -Si(CH₃)₂(n-C₆H₁₃) | -Si(CH₃)₂(n-C₆H₁₃) |
| 399 | S | S | S | -Si(CH₃)₂(n-C₁₂H₂₅) | -Si(CH₃)₂(n-C₁₂H₂₅) |

| | | | | | |
|---|---|---|---|---|---|
| Dashed line indicates a chemical bond. | | | | | |

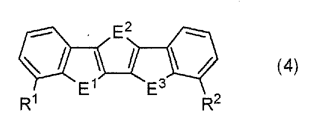

| Table 23 | | | | | |
|---|---|---|---|---|---|
| Compound No. | E¹ | E² | E³ | R¹ | R² |
| 400 | S | S | S | n-C₄H₉ | n-C₄H₉ |
| 401 | S | S | S | n-C₅H₁₁ | n-C₅H₁₁ |
| 402 | S | S | S | 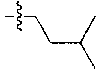 | 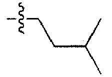 |
| 403 | S | S | S | n-C₆H₁₃ | n-C₆H₁₃ |
| 404 | S | S | S | 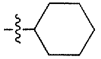 | 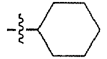 |
| 405 | S | S | S | 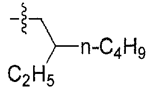 | 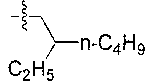 |
| 406 | S | S | S | n-C₈H₁₇ | n-C₈H₁₇ |
| 407 | S | S | S | 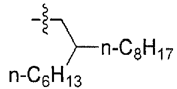 | 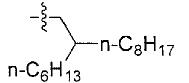 |
| 408 | S | S | S | n-C₁₂H₂₅ | n-C₁₂H₂₅ |
| 409 | S | S | S | n-C₁₃H₂₇ | n-C₁₃H₂₇ |
| 410 | S | S | S | n-C₁₆H₃₃ | n-C₁₆H₃₃ |
| 411 | S | S | S | n-C₁₈H₃₇ | n-C₁₈H₃₇ |
| 412 | S | S | S | n-C₂₀H₄₁ | n-C₂₀H₄₁ |
| 413 | S | S | S | n-C₂₅H₅₁ | n-C₂₅H₅₁ |
| 414 | S | S | S | n-C₃₀H₆₁ | n-C₃₀H₆₁ |
| 415 | S | S | S | n-C₆F₁₃ | n-C₆F₁₃ |
| 416 | S | Se | S | n-C₆H₁₃ | n-C₆H₁₃ |
| 417 | Se | S | Se | n-C₈H₁₇ | n-C₈H₁₇ |
| 418 | S | S | S | n-C₆H₁₃ | n-C₁₂H₂₅ |
| 420 | S | S | S | O(n-C₆H₁₃) | O(n-C₆H₁₃) |
| 423 | S | S | S | O(n-C₈H₁₇) | O(n-C₈H₁₇) |
| 425 | S | S | S | O(n-C₁₂H₂₅) | O(n-C₁₂H₂₅) |

| | | | | | |
|---|---|---|---|---|---|
| Dashed line indicates a chemical bond. | | | | | |

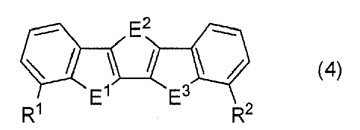

| Table 24 | | | | | |
|---|---|---|---|---|---|
| Compound No. | E¹ | E² | E³ | R¹ | R² |
| 426 | S | S | S | O(n-C₈F₁₇) | O(n-C₈F₁₇) |
| 427 | S | Se | S | O(n-C₆H₁₃) | O(n-C₆H₁₃) |
| 428 | S | S | S | O(n-C₈H₁₇) | O(n-C₁₂H₂₅) |
| 429 | S | S | S | 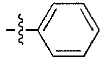 | 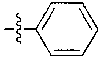 |
| 431 | S | S | S | 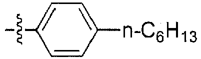 | 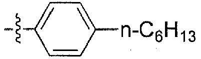 |
| 433 | S | S | S | 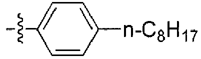 | 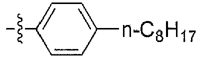 |
| 434 | S | S | S | 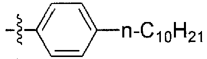 | 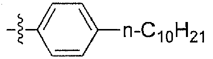 |
| 436 | S | S | S | 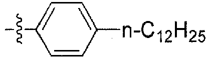 | 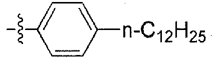 |
| 437 | S | S | S | 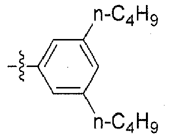 | 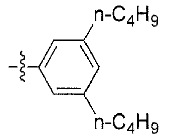 |
| 439 | S | S | S | 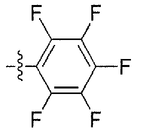 | 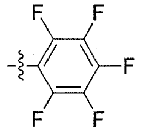 |
| 440 | S | S | S | 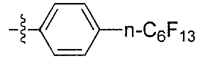 | 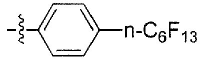 |
| 441 | S | S | S | 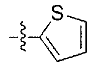 | 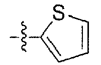 |

| | | | | | |
|---|---|---|---|---|---|
| Dashed line indicates a chemical bond. | | | | | |

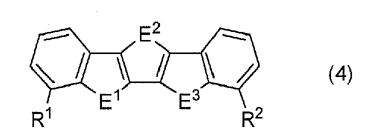

| Table 25 | | | | | |
|---|---|---|---|---|---|
| Compound No. | E¹ | E² | E³ | R¹ | R² |
| 442 | S | S | S | 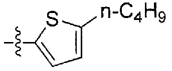 | 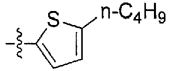 |
| 443 | S | S | S | 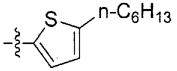 | 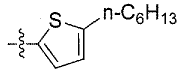 |
| 445 | S | S | S | 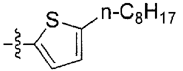 | 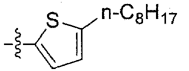 |
| 446 | S | S | S | 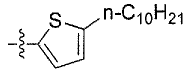 | 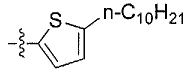 |
| 448 | S | S | S | 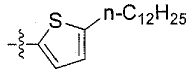 | 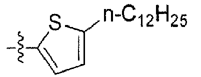 |
| 449 | S | S | S | 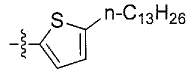 | 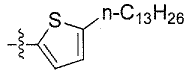 |
| 450 | S | S | S | 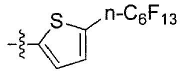 | 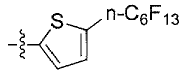 |
| 451 | S | S | S | 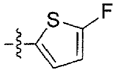 | 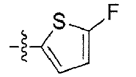 |
| 452 | S | S | S | 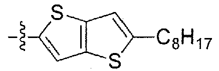 | 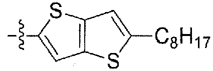 |
| 456 | S | S | S | 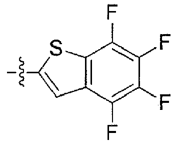 | 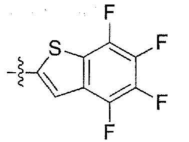 |

| | | | | | |
|---|---|---|---|---|---|
| Dashed line indicates a chemical bond. | | | | | |

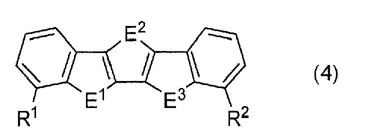

| Table 26 | | | | | |
|---|---|---|---|---|---|
| Compound No. | E¹ | E² | E³ | R¹ | R² |
| 459 | S | S | S | 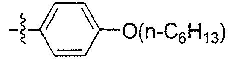 | 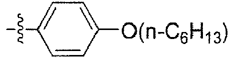 |
| 461 | S | S | S | 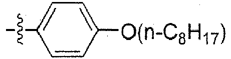 | 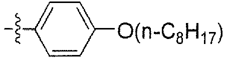 |
| 463 | S | S | S | 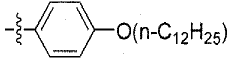 | 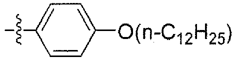 |
| 464 | S | S | S | 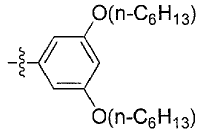 | 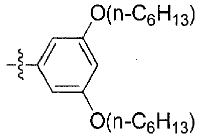 |
| 466 | S | S | S | 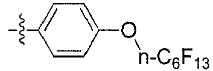 | 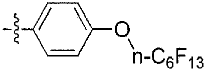 |
| 467 | S | S | S | 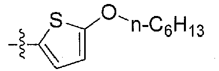 | 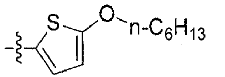 |
| 469 | S | S | S | 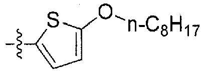 | 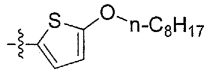 |
| 470 | S | S | S | 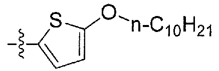 | 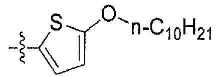 |

| | | | | | |
|---|---|---|---|---|---|
| Dashed line indicates a chemical bond. | | | | | |

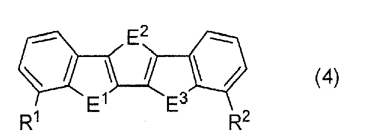

| Table 27 | | | | | |
|---|---|---|---|---|---|
| Compound No. | E¹ | E² | E³ | R¹ | R² |
| 472 | S | S | S | 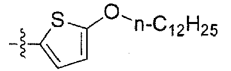 | 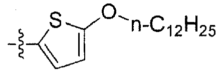 |
| 473 | S | S | S | 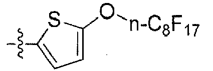 | 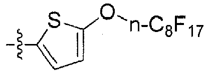 |
| 474 | S | Se | S | 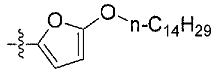 | 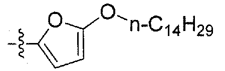 |
| 475 | S | S | S | 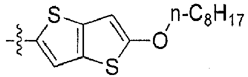 | 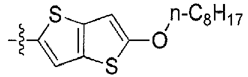 |
| 479 | S | S | S | 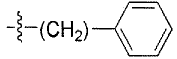 | 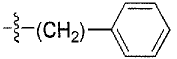 |
| 480 | S | S | S | 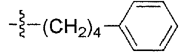 | 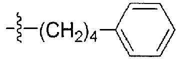 |
| 481 | S | S | S | 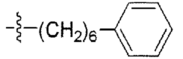 | 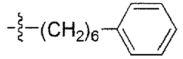 |
| 482 | S | S | S | 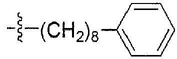 | 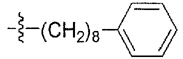 |
| 483 | S | S | S | 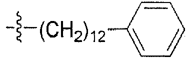 | 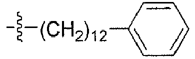 |
| 484 | S | S | S | 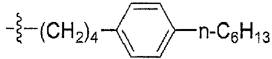 | 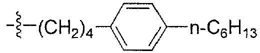 |
| 485 | S | S | S | 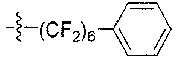 | 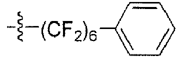 |

| | | | | | |
|---|---|---|---|---|---|
| Dashed line indicates a chemical bond. | | | | | |

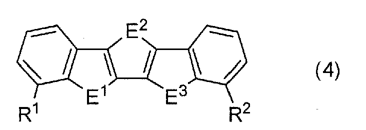

| Table 28 | | | | | |
|---|---|---|---|---|---|
| Compound No. | E¹ | E² | E³ | R¹ | R² |
| 486 | Se | S | Se | 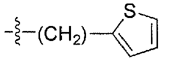 | 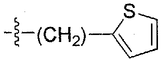 |
| 487 | S | S | S | 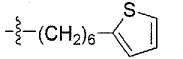 | 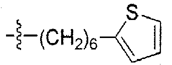 |
| 488 | S | S | S | 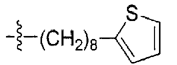 | 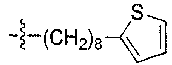 |
| 489 | S | S | S | 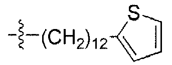 | 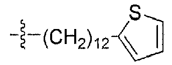 |
| 490 | S | S | S | 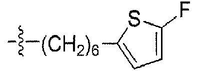 | 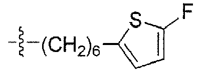 |
| 491 | S | S | S | 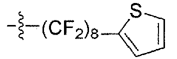 | 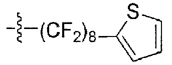 |
| 492 | S | S | S | 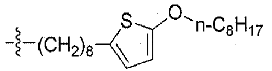 | 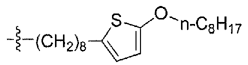 |
| 493 | S | S | S | 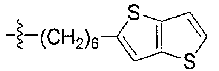 | 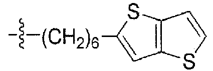 |
| 494 | S | S | S | 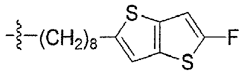 | 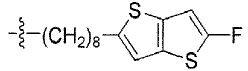 |
| 495 | S | S | S | 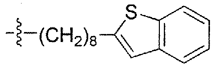 | 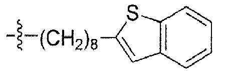 |
| 496 | S | S | S | -Si(CH₃)₃ | -Si(CH₃)₃ |

| | | | | | |
|---|---|---|---|---|---|
| Dashed line indicates a chemical bond. | | | | | |

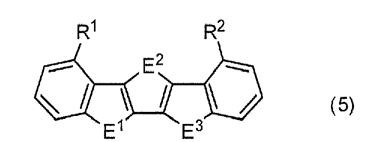

| Table 29 | | | | | |
|---|---|---|---|---|---|
| Compound No. | E¹ | E² | E³ | R¹ | R² |
| 497 | S | S | S | n-C₆H₁₃ | n-C₆H₁₃ |
| 498 | S | S | S | n-C₁₂H₂₅ | n-C₁₂H₂₅ |
| 499 | S | S | S | n-C₁₃H₂₇ | n-C₁₃H₂₇ |
| 500 | S | S | S | n-C₆F₁₃ | n-C₆F₁₃ |
| 501 | S | S | S | O(n-C₆H₁₃) | O(n-C₆H₁₃) |
| 502 | S | S | S | 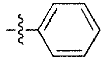 | 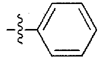 |
| 503 | S | S | S | 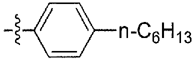 | 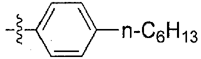 |
| 504 | S | S | S | 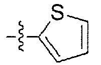 | 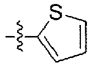 |
| 505 | S | S | S | 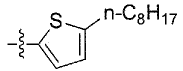 | 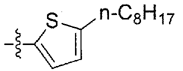 |
| 509 | S | S | S | 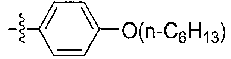 | 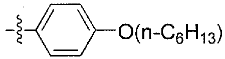 |
| 513 | S | S | S | 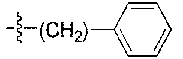 | 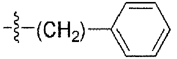 |
| 514 | S | S | S | 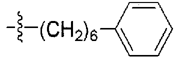 | 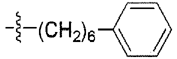 |

| | | | | | |
|---|---|---|---|---|---|
| Dashed line indicates a chemical bond. | | | | | |

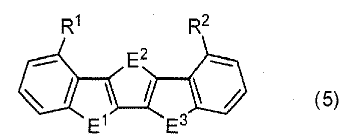

| Table 30 | | | | | |
|---|---|---|---|---|---|
| Compound No. | E¹ | E² | E³ | R¹ | R² |
| 515 | Se | S | Se | 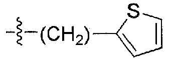 | 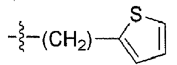 |
| 516 | S | S | S | 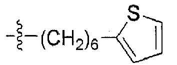 | 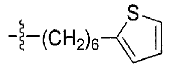 |
| 517 | S | S | S | 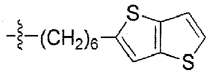 | 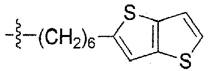 |
| 518 | S | S | S | 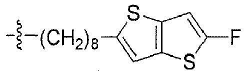 | 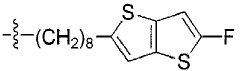 |
| 519 | S | S | S | 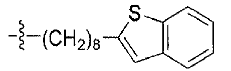 | 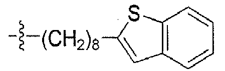 |
| 520 | S | S | S | -Si(CH₃)₃ | -Si(CH₃)₃ |

| | | | | | |
|---|---|---|---|---|---|
| Dashed line indicates a chemical bond. | | | | | |

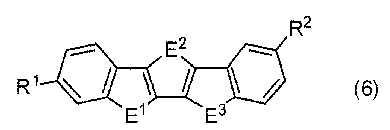

| Table 31 | | | | | |
|---|---|---|---|---|---|
| Compound No. | E¹ | E² | E³ | R¹ | R² |
| 521 | S | S | S | n-C₆H₁₃ | n-C₆H₁₃ |
| 522 | S | S | S | O(n-C₆H₁₃) | O(n-C₆H₁₃) |
| 523 | S | S | S | 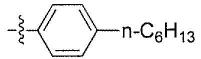 | 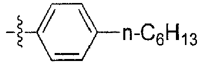 |
| 524 | S | S | S | 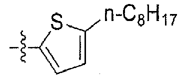 | 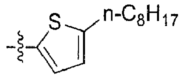 |

| | | | | | |
|---|---|---|---|---|---|
| Dashed line indicates a chemical bond. | | | | | |

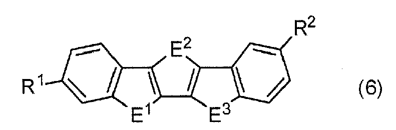

| Table 32 | | | | | |
|---|---|---|---|---|---|
| Compound No. | E¹ | E² | E³ | R¹ | R² |
| 530 | S | S | S | 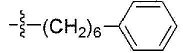 | 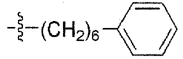 |
| 531 | S | S | S | 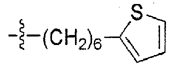 | 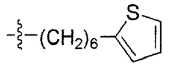 |
| 532 | S | S | S | 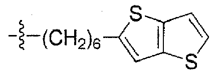 | 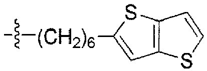 |
| 533 | S | S | S | 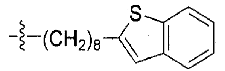 | 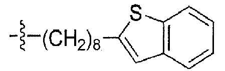 |
| 534 | S | S | S | -Si(CH₃)₃ | -Si(CH₃)₃ |

| | | | | | |
|---|---|---|---|---|---|
| Dashed line indicates a chemical bond. | | | | | |

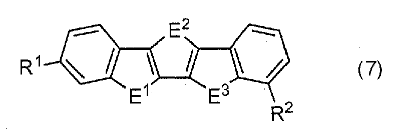

| Table 33 | | | | | |
|---|---|---|---|---|---|
| Compound No. | E¹ | E² | E³ | R¹ | R² |
| 535 | S | S | S | n-C₆H₁₃ | n-C₆H₁₃) |
| 536 | S | S | S | O(n-C₆H₁₃) | O(n-C₆H₁₃) |
| 537 | S | S | S | 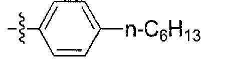 | 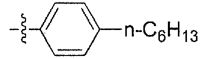 |
| 538 | S | S | S | 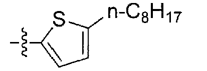 | 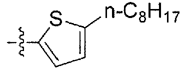 |

| | | | | | |
|---|---|---|---|---|---|
| Dashed line indicates a chemical bond. | | | | | |

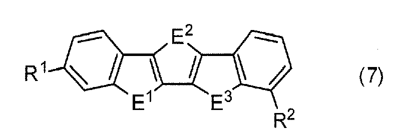

| Table 34 | | | | | |
|---|---|---|---|---|---|
| Compound No. | E¹ | E² | E³ | R¹ | R² |
| 545 | S | S | S | 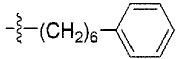 | 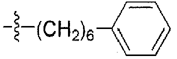 |
| 546 | S | S | S | 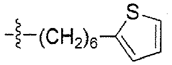 | 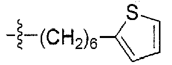 |
| 547 | S | S | S | 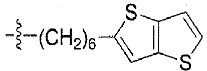 | 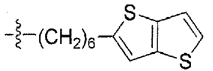 |
| 548 | S | S | S | 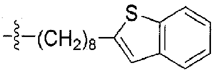 | 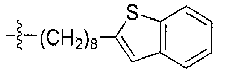 |
| 549 | S | S | S | -Si(CH₃)₃ | -Si(CH₃)₃ |

| | | | | | |
|---|---|---|---|---|---|
| Dashed line indicates a chemical bond. | | | | | |

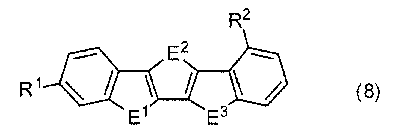

| Table 35 | | | | | |
|---|---|---|---|---|---|
| Compound No. | E¹ | E² | E³ | R¹ | R² |
| 550 | S | S | S | n-C₆H₁₃ | n-C₆H₁₃ |
| 551 | S | S | S | 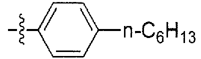 | 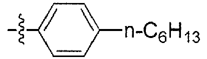 |
| 554 | S | S | S | 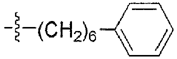 | 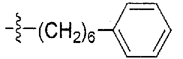 |
| 555 | S | S | S | 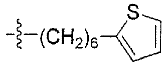 | 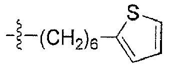 |
| 556 | S | S | S | -Si(CH₃)₃ | -Si(CH₃)₃ |

| | | | | | |
|---|---|---|---|---|---|
| Dashed line indicates a chemical bond. | | | | | |

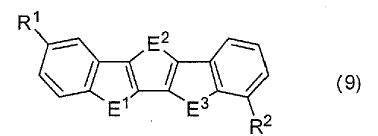

| Table 36 | | | | | |
|---|---|---|---|---|---|
| Compound No. | E¹ | E² | E³ | R¹ | R² |
| 557 | S | S | S | n-C₆H₁₃ | n-C₆H₁₃ |
| 558 | S | S | S | 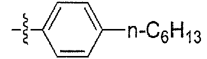 | 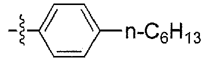 |
| 561 | S | S | S | 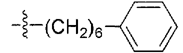 | 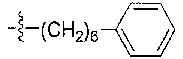 |
| 562 | S | S | S | 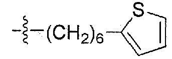 | 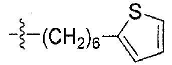 |
| 563 | S | S | S | -Si(CH₃)₃ | -Si(CH₃)₃ |

| | | | | | |
|---|---|---|---|---|---|
| Dashed line indicates a chemical bond. | | | | | |

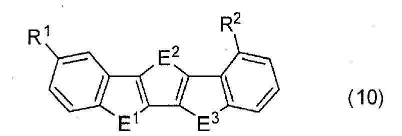

| Table 37 | | | | | |
|---|---|---|---|---|---|
| Compound No. | E¹ | E² | E³ | R¹ | R² |
| 564 | S | S | S | n-C₆H₁₃ | n-C₆H₁₃ |
| 565 | S | S | S | 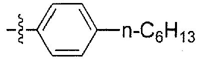 | 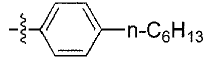 |
| 568 | S | S | S | 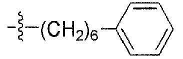 | 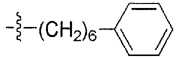 |
| 569 | S | S | S | 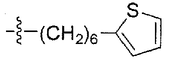 | 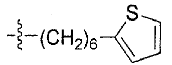 |
| 570 | S | S | S | -Si(CH₃)₃ | -Si(CH₃)₃ |

| | | | | | |
|---|---|---|---|---|---|
| Dashed line indicates a chemical bond. | | | | | |

Among the substituted benzochalcogenoacene compounds (1), a compound is preferable in which three of E's in the benzochalcogenoacene compound (1) are all sulfur atoms.
Especially, the substituted benzochalcogenoacene compounds (1) having the following numbers in the above tables are preferably exemplified:
1, 3, 4, 5, 6, 7, 8, 9, 10, 11, 12, 13, 14, 15, 16, 17, 18, 19, 20, 21, 22, 33, 34, 35, 38, 39, 40, 41, 42, 43, 44, 46, 47, 48, 49, 51, 52, 53, 54, 55, 56, 57, 58, 59, 60, 76, 77, 78, 79, 80, 81, 82, 84, 86, 87, 89, 90, 91, 94, 95, 96, 97, 99, 100, 102, 103, 104, 105, 106, 108, 117, 118, 119, 120, 122, 124, 125, 127, 128, 129, 131, 132, 133, 135, 136, 138, 139, 142, 144, 152, 153, 154, 155, 156, 157, 158, 159, 160, 161, 162, 163, 164, 165, 166, 167, 168, 169, 170, 176, 180, 181, 182, 183, 184, 185, 186, 187, 188, 189, 202, 203, 204, 205, 206; 208, 209, 210, 211, 212, 213, 214, 215, 216, 217, 218, 219,220, 221, 222, 223, 224, 225, 226, 227, 228, 229, 230, 231, 232, 233, 234, 235, 246, 247, 248, 252, 253, 254, 256, 259, 260, 261, 262, 264, 265, 266, 267, 268, 269, 270, 271, 272, 273, 284, 285, 286, 291, 292, 293, 294, 295, 297, 299, 300, 302, 306, 307, 308, 309, 310, 312, 313, 315, 3I6, 317, 319, 321, 327, 330, 331, 332, 333, 335, 337, 338, 340, 341, 342, 344, 345, 346, 348, 349, 351, 352, 353, 354, 357, 365, 366, 367, 368, 369, 370, 371, 372, 373, 374, 375, 379, 380, 381, 382, 383, 384, 385, 386, 387, 388, 389, 390, 391, 392, 393, 394, 395, 396, 397, 398 and 399.

The substituted benzochalcogenoacene compounds (1) having the following numbers in the above tables are exemplified as more preferable:
1, 3, 4, 5, 6, 7, 8, 9, 10, 11, 12, 15, 16, 17, 18, 38, 39, 40, 41, 42, 43, 46, 47, 48, 49, 51, 52, 53, 54, 55, 56, 76, 78, 80, 81, 82, 84, 86, 87, 89, 90, 91, 95, 96, 97, 99, 100, 102, 103, 108, 118, 119, 120, 122, 124, 125, 127, 128, 129, 132, 133, 135, 136, 138, 139, 144, 152, 153, 154, 155, 156, 157, 158, 159, 160, 161, 176, 180, 181, 182, 183, 184, 185, 186, 187, 188, 189, 202, 203, 205, 206, 208, 209, 211, 214, 215, 216, 217, 218, 219, 220, 221, 222, 223, 224, 225, 226, 227, 228, 229, 230, 231, 252, 253, 254, 256, 259, 260, 261, 262, 264, 265, 266, 267, 268, 269, 291, 293, 294, 295, 297, 299, 300, 302, 308, 309, 310, 312, 313, 315, 316, 317, 321, 327, 330, 331, 332, 333, 335, 337, 338, 340, 341, 344, 345, 346, 348, 349, 351, 352, 353, 354, 357, 365, 366, 367, 368, 369, 370, 371, 380, 381, 382, 383, 384, 385, 386, 389, 390, 392, 394, 395, 396 and 397.

The substituted benzochalcogenoacene compound (1) of the present invention is excellent in the solubility in the organic solvent, therefore, its handling is easy and its purification is easily carried out.
A thin film can be also formed by dissolving the substituted benzochalcogenoacene compound (1) in the organic solvent, applying the solution and drying it. The thin film can be easily formed by the applying and film-forming process to be described hereinafter, since the substituted benzochalcogenoacene compound (1) is excellent in the solubility.
In addition, the substituted benzochalcogenoacene compound (1) can provide a thin film showing high carrier mobility.

A process for producing the substituted benzochalcogenoacene compound (1) is described below.
In the process for producing the compound (1), firstly a diacetylene compound is provided which is represented, for example, by the formula (5-1) (hereinafter optionally described as a "compound (5-1)"): 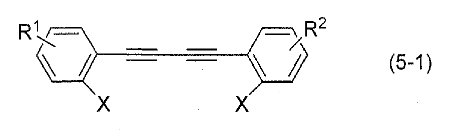 (wherein R¹ and R² represent the same meanings as described above, and X represents a halogen atom, preferably, a bromine atom), and subsequently, after dimetallation by a halogen-metal exchange reaction using an organometallic base (hereinafter, called a "present 1st reaction"), a dichalcogen-ene compound (optionally described as a "compound (4-1)") represented by the formula (4-1): 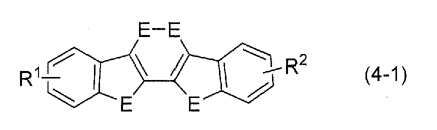 (wherein E, R¹ and R² represent the same meanings as described above) is obtained by working of sulfur or selenium (hereinafter, optionally described as a "present 2nd reaction").
Then, a mixture of the obtained compound (4-1) and a platinum compound such as biscyclooctadienyl platinum (Pt(COD)₂) or a copper compound such as a copper powder is heated in the absence of a solvent (hereinafter, optionally described as a "3rd-1 reaction"), or a mixture of the obtained compound (4-1), a nickel compound such as biscyclooctadienyl nickel (Ni(COD)₂) and a phosphine compound is heated and stirred in the presence of a solvent (optionally described as a "3rd-2 reaction").

The organometallic bases used in the present 1st reaction are exemplified by organolithium compounds such as methyllithium (MeLi), n-butyllithium (n-BuLi), sec-butyllithium (sec-BuLi) and tert-butyllithium (t-BuLi) and an organomagnesium compound such as an alkylgrignard compound. From the view point of a better reactivity in the halogen-metal exchange reaction, the organolithium compound is preferable as the organometallic base. For example, butyllithium (BuLi) can be used, and more preferably, t-butyl lithium (t-BuLi) can be used. (An expression in equivalent tends to be understood as unclear. Therefore, an expression based on the amount (moles) of the compound is also described side by side as below.)
The amount of the organometallic base to be used based on 1 mole of the compound (5-1) is, for example, in the range of 4 to 20 moles (in the range of 2 to 10 equivalents to 1 equivalent of a halogen atom), preferably in the range of 6 to 14 moles (in the range of 3 to 7 equivalents to 1 equivalent of a halogen atom), more preferably, in the range of 7 to 10 moles (in the range of 3.5 to 5 equivalents to 1 equivalent of the halogen atom). When the amount of the organometallic base used is 4 moles or more, the unreacted amount of the compound (5-1) is reduced and the yield of the obtained compound (4-1) tends to increase. When the amount used is 20 moles or less, a progress of a side reaction is suppressed and a purification of the compound (4-1) tends to become easy.

The present 1st reaction and the succeeding 2nd reaction are preferable to be carried out in the presence of a solvent.
The solvent used is selected from those which do not remarkably prevent the present 1st and 2nd reactions. For example, aliphatic hydrocarbon solvents such as pentane, hexane and heptane, aromatic solvents such as benzene, toluene and xylene, ether solvents such as diethyl ether and tetrahydrofuran (THF) and the mixtures of 2 or more selected among them are used. A preferable solvent is the ether solvent.

The present 1st reaction is carried out at temperatures of, for example, -20°C or lower, preferably, -40°C or lower, more preferably, -60°C or lower. The reaction time of the present 1st reaction can be controlled by the kind of organometallic bases or the solvents or by the reaction temperature, and the reaction time is in the range around from 10 minutes to 5 hours. Following the present 1st reaction, the present second reaction is carried out. A sulfur (or selenium) may be used as purchased or may be added as a solution or a suspension dissolved or suspended in the solvent used in the present 1st reaction. After addition of the sulfur (or selenium), the reaction temperature may be kept at a similar temperature to the present 1st reaction or may be heated in the temperature range which does not exceed the boiling point of the solvent used. Preferably, heating is carried out to reach the temperature range of 0 to 40°C, and subsequently, the temperature is kept in the same range. The reaction time is, for example, from 30 minutes to 72 hours.

A crystalline, powder or colloidal sulfur (or selenium) can be used as the sulfur or selenium to be used in the present 2nd reaction. The amount of the sulfur or selenium used may be, for example, in the range of 4 to 20 moles, preferably, in the range of 6 to 14 moles, more preferably, in the range of 7 to 10 moles based on 1 mole of the compound (1-5). It is preferable to use the sulfur (or selenium) in the amount of 4 moles or more, since the yield of the compound (4-1) tends to increase in the molar range. It is also preferable to use the sulfur (or selenium) in the amount of 20 moles or less, since, in the molar range, a progress of a side reaction can be inhibited and a purification of the compound (4-1) tends to become easy.

After completing the present 2nd reaction, a solvent in the reaction mixture is optionally evaporated. To the obtained reaction mixture, an alkaline water solution such as a sodium hydroxide water solution or a potassium hydroxide water solution is added, and the obtained compound (4-1) is extracted. When the solvent used in the present 1st and 2nd reactions is water, the solvent can be used directly as an extraction solvent. However, it is preferable to use a halogenated hydrocarbon solvent such as dichloromethane or chloroform as the extraction solvent. After obtaining two phases consisting of an organic phase and a water phase, the water phase is separated, then, to the water phase, a water solution of a hexacyanoferrate (III) salt such as potassium ferrycyanide is added, and subsequently, the compound (4-1) is extracted from the water phase by using an organic solvent such as said extraction solvent. The compound (4-1) thus obtained may be optionally purified further by the processes such as chromatography and recrystallization.

In the present 3rd-1 reaction, a copper compound or a platinum compound can be used in an amount of, for example, 0.5 to 20 moles, preferably 1 to 10 moles, more preferably 2 to 7 moles based on 1 mole of the compound (4-1). An example of the copper compound is a copper powder and an example of the platinum compound is biscyclooctadienyl platinum (Pt(COD)₂).
A reaction temperature of the present 3rd-1 reaction is, for example, from 150 to 400°C, preferably, from 200 to 370°C. A reaction temperature of the present 3rd-1 reaction is within 1 hour, preferably, within 30 minutes. After completing the reaction, the reaction temperature is lowered to room temperature, and insoluble impurities are filtered off by using an organic solvent such as chloroform or dichloromethane which can dissolve the substituted benzochalcogenoacene compound (1). The filtrate is concentrated and optionally followed by application of column chromatography, recrystallization, etc. to result in the production of the substituted benzochalcogenoacene compound (1).

A zero valent nickel compound such as biscyclooctadienyl nickel (Ni(COD)₂) is preferable as a nickel compound used in the present 3rd-1 reaction. The zero valent nickel compound may be formed in-situ by reduction of a two valent nickel compound such as bisacetylacetonato nickel (Ni(acac)₂) with a reducing agent such as diisobutylaluminum hydride.
The amount of the nickel compound used is, for example, in a range of 0.5-5 moles, and preferably in a range of 0.7-3 moles based on 1 mole of the compound (4-1).
Examples of a phosphine compound include triphenylphosphine, tricyclohexylphosphine, tri(o-tolyl)phosphine, trimethylphosphine, tri-t-butylphosphine, 1,2-(diphenylphosphino)ethane, 1,3-(diphenylphosphino)propane, 1,4-(diphenylphosphino)butane and 1,1-bis(diphenylphosphino)ferrocene. Among them, triphenylphosphine is preferable.
The amount of the phosphine compound used is, for example, in a range of 0.5-20 moles of the phosphine compound, preferably, in a range of 0.7-10 moles based on 1 mole of the nickel compound.

Examples of a solvent used in the present 3rd-2 reaction include an aliphatic hydrocarbon solvent such as pentane, hexane and heptane, an aromatic hydrocarbon solvent such as benzene, toluene and xylene, and a halogenated hydrocarbon solvent such as dichloromethane and chloroform. These solvents can be use alone or in a mixture of 2 or more of them. The aromatic hydrocarbon solvent is preferable, and toluene is more preferable as the solvent.
The reaction temperature of the present 3rd-2 reaction is, for example, in a range from 10°C to a boiling point or lower of the solvent.
The reaction time of the present 3rd-2 reaction is preferably within 72 hours depending on the reaction temperature.
After completing the present 3rd-2 reaction, insoluble impurities are filtered off optionally under heating. The filtrate is concentrated and optionally followed by purification using column chromatography, recrystallization, etc. to result in the production of the substituted benzochalcogenoacene compound (1).

Specific examples of the compound (5-1) used in the present 1st reaction are described in the following tables.

| Table 38 | | | | |
|---|---|---|---|---|
| 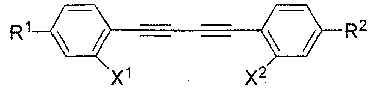 | | | | |

| Compound No. | X¹ | X² | R¹ | R² |
|---|---|---|---|---|
| 1141 | Br | Br | n-C₄H₉ | n-C₄H₉ |
| 1142 | Br | Br | S-C₄H₉ | s-C₄H₉ |
| 1143 | Br | Br | n-C₅H₁₁ | n-C₅H₁₁ |
| 1144 | Br | Br | 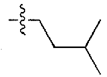 | 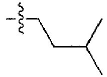 |
| 1145 | Br | Br | n-C₆H₁₃ | n-C₆H₁₃ |
| 1146 | Br | Br | 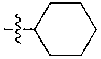 | 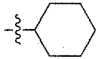 |
| 1147 | Br | Br | 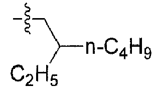 | 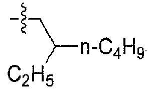 |
| 1148 | Br | Br | n-C₇H₁₅ | n-C₇H₁₅ |
| 1149 | Br | Br | n-C₈H₁₇ | n-C₈H₁₇ |
| 1150 | Br | Br | n-C₉H₁₉ | n-C₉H₁₉ |
| 1151 | Br | Br | n-C₁₀H₂₁ | n-C₁₀H₂₁ |
| 1152 | Br | Br | 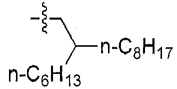 | 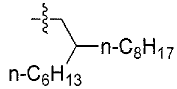 |
| 1153 | Br | Br | n-C₁₁H₂₃ | n-C₁₁H₂₃ |
| 1154 | Br | Br | n-C₁₂H₂₅ | n-C₁₂H₂₅ |
| 1155 | Br | Br | n-C₁₃H₂₇ | n-C₁₃H₂₇ |

| | | | | |
|---|---|---|---|---|
| Dashed line indicates a chemical bond. | | | | |

| Table 39 | | | | |
|---|---|---|---|---|
| 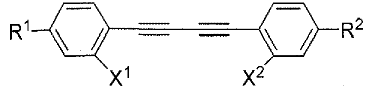 | | | | |

| Compound No. | X¹ | X² | R¹ | R² |
|---|---|---|---|---|
| 1156 | Br | Br | n-C₁₄H₂₉ | n-C₁₄H₂₉ |
| 1157 | Br | Br | n-C₁₅H₃₁ | n-C₁₅H₃₁ |
| 1158 | Br | Br | n-C₁₆H₃₃ | n-C₁₆H₃₃ |
| 1159 | Br | Br | n-C₁₇H₃₅ | n-C₁₇H₃₅ |
| 1160 | Br | Br | n-C₁₈H₃₇ | n-C₁₈H₃₇ |
| 1161 | Br | Br | n-C₁₉H₃₉ | n-C₁₉H₃₉ |
| 1162 | Br | Br | n-C₂₀H₄₁ | n-C₂₀H₄₁ |
| 1163 | Br | Br | n-C₂₁H₄₃ | n-C₂₁H₄₃ |
| 1164 | Br | Br | n-C₂₂H₄₅ | n-C₂₂H₄₅ |
| 1165 | Br | Br | n-C₂₃H₄₇ | n-C₂₃H₄₇ |
| 1166 | Br | Br | n-C₂₄H₄₉ | n-C₂₄H₄₉ |
| 1167 | Br | Br | n-C₂₅H₅₁ | n-C₂₅H₅₁ |
| 1168 | Br | Br | n-C₂₆H₅₃ | n-C₂₆H₅₃ |
| 1169 | Br | Br | n-C₂₇H₅₅ | n-C₂₇H₅₅ |
| 1170 | Br | Br | n-C₂₈H₅₇ | n-C₂₈H₅₇ |
| 1171 | Br | Br | n-C₂₉H₅₉ | n-C₂₉H₅₉ |
| 1172 | Br | Br | n-C₃₀H₆₁ | n-C₃₀H₆₁ |
| 1173 | Br | Br | n-C₆F₁₃ | n-C₆F₁₃ |
| 1174 | Br | Br | n-C₈F₁₇ | n-C₈F₁₇ |
| 1175 | Br | Br | n-C₁₂F₂₅ | n-C₁₂F₂₅ |
| 1176 | Br | Br | n-C₁₆F₃₃ | n-C₁₆F₃₃ |
| 1177 | I | I | n-C₆H₁₃ | n-C₆H₁₃ |
| 1178 | Br | Br | n-C₈H₁₇ | H |
| 1179 | Br | Br | n-C₆H₁₃ | n-C₁₂H₂₅ |
| 1180 | Br | Br | O(n-C₄H₉) | O(n-C₄H₉) |
| 1181 | Br | Br | O(n-C₅H₁₁) | O(n-C₅H₁₁) |
| 1183 | Br | Br | O(n-C₆H₁₃) | O(n-C₆H₁₃) |
| 1186 | Br | Br | O(n-C₇H₁₅) | O(n-C₇H₁₅) |
| 1187 | Br | Br | O(n-C₈H₁₇) | O(n-C₈H₁₇) |
| 1188 | Br | Br | O(n-C₉H₁₉) | O(n-C₉H₁₉) |
| 1189 | Br | Br | O(n-C₁₀H₂₁) | O(n-C₁₀H₂₁) |

| | | | | |
|---|---|---|---|---|
| Dashed line indicates a chemical bond. | | | | |

| Table 40 | | | | |
|---|---|---|---|---|
| 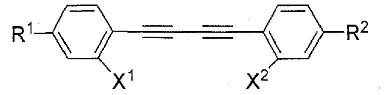 | | | | |

| Compound No. | X¹ | X² | R¹ | R² |
|---|---|---|---|---|
| 1191 | Br | Br | O(n-C₁₁H₂₃) | O(n-G₁₁H₂₃) |
| 1192 | Br | Br | O(n-C₁₂H₂₅) | O(n-C₁₂H₂₅) |
| 1193 | Br | Br | O(n-C₁₃H₂₇) | O(n-C₁₃H₂₇) |
| 1194 | Br | Br | O(n-C₁₄H₂₉) | O(n-C₁₄H₂₉) |
| 1195 | Br | Br | O(n-C₁₅H₃₁) | O(n-C₁₅H₃₁) |
| 1196 | Br | Br | O(n-C₁₆H₃₃) | O(n-C₁₆H₃₃) |
| 1197 | Br | Br | O(n-C₁₇H₃₅) | O(n-C₁₇H₃₅) |
| 1198 | Br | Br | O(n-C₁₈H₃₇) | O(n-C₁₈H₃₇) |
| 1199 | Br | Br | O(n-C₁₉H₃₉) | O(n-C₁₉H₃₉) |
| 1200 | Br | Br | O(n-C₂₀H₄₁) | O(n-C₂₀H₄₁) |
| 1201 | Br | Br | O(n-C₂₁H₄₃) | O(n-C₂₁H₄₃) |
| 1202 | Br | Br | O(n-C₂₂H₄₅) | O(n-C₂₂H₄₅) |
| 1203 | Br | Br | O(n-C₂₃H₄₇) | O(n-C₂₃H₄₇) |
| 1204 | Br | Br | O(n-C₂₄H₄₉) | O(n-C₂₄H₄₉) |
| 1205 | Br | Br | O(n-C₂₅H₅₁) | O(n-C₂₅H₅₁) |
| 1206 | Br | Br | O(n-C₂₆H₅₃) | O(n-C₂₆H₅₃) |
| 1207 | Br | Br | O(n-C₂₇H₅₅) | O(n-C₂₇H₅₅) |
| 1208 | Br | Br | O(n-C₂₈H₅₇) | O(n-C₂₈H₅₇) |
| 1209 | Br | Br | O(n-C₂₉H₅₉) | O(n-C₂₉H₅₉) |
| 1210 | Br | Br | O(n-C₃₀H₆₁) | O(n-C₃₀H₆₁) |
| 1211 | Br | Br | O(n-C₆F₁₃) | O(n-C₆F₁₃) |
| 1212 | Br | Br | O(n-C₈F₁₇) | O(n-C₈F₁₇) |
| 1213 | Br | Br | O(n-C₁₂F₂₅) | O(n-C₁₂F₂₅) |
| 1214 | Br | Br | O(n-C₁₆H₁₃) | O(n-C₁₆F₃₃) |
| 1215 | Br | I | O(n-C₆H₁₃) | O(n-C₆H₁₃) |
| 1216 | I | I | O(n-C₈H₁₇) | O(n-C₈H₁₇) |
| 1217 | Br | Br | O(n-C₈H₁₇) | O(n-C₁₂H₂₅) |
| 1218 | Br | Br | 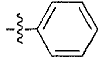 | 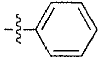 |
| 1219 | Br | Br | 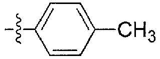 | 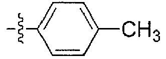 |

| | | | | |
|---|---|---|---|---|
| Dashed line indicates a chemical bond. | | | | |

| Table 41 | | | | |
|---|---|---|---|---|
| 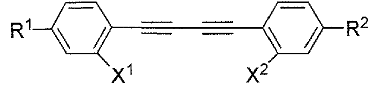 | | | | |

| Compound No. | X¹ | X² | R¹ | R² |
|---|---|---|---|---|
| 1220 | Br | Br | 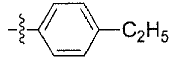 | 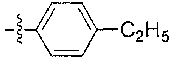 |
| 1221 | Br | Br | 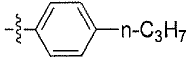 | 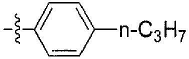 |
| 1222 | Br | Br | 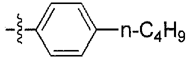 | 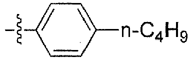 |
| 1224 | Br | Br | 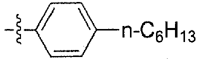 | 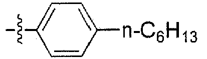 |
| 1226 | Br | Br | 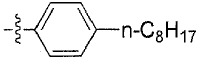 | 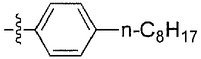 |
| 1227 | Br | Br | 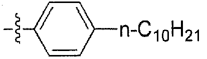 | 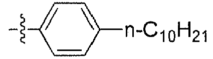 |
| 1229 | Br | Br | 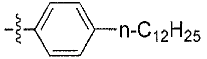 | 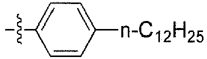 |
| 1230 | Br | Br | 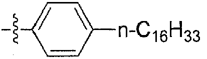 | 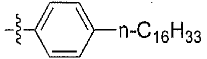 |
| 1231 | Br | Br | 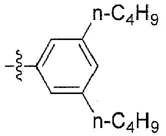 | 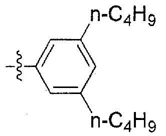 |
| 1233 | Br | Br | 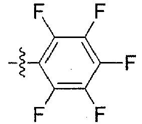 | 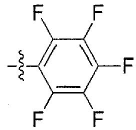 |
| 1234 | Br | Br | 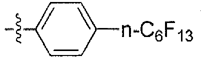 | 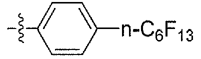 |
| 1235 | Br | Br | 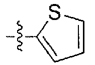 | 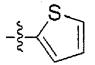 |

| | | | | |
|---|---|---|---|---|
| Dashed line indicates a chemical bond. | | | | |

| Table 42 | | | | |
|---|---|---|---|---|
| 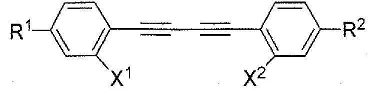 | | | | |

| Compound No. | X¹ | X² | R¹ | R² |
|---|---|---|---|---|
| 1236 | Br | Br | 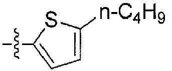 | 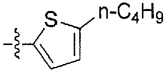 |
| 1237 | Br | Br | 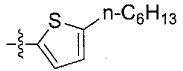 | 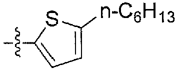 |
| 1239 | Br | Br | 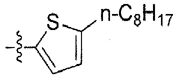 | 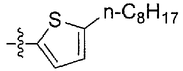 |
| 1240 | Br | Br | 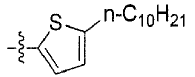 | 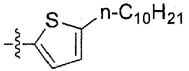 |
| 1242 | Br | Br | 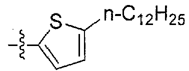 | 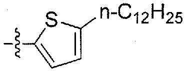 |
| 1243 | Br | Br | 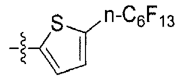 | 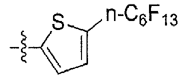 |
| 1244 | Br | Br | 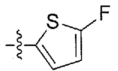 | 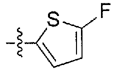 |
| 1245 | Br | Br | 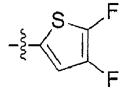 | 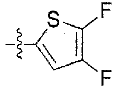 |
| 1246 | Br | Br | 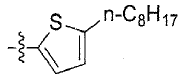 | 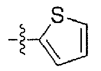 |
| 1247 | Br | Br | 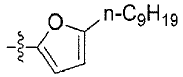 | 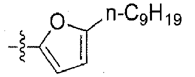 |
| 1248 | Br | Br | 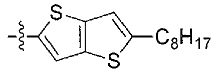 | 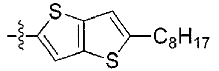 |

| | | | | |
|---|---|---|---|---|
| Dashed line indicates a chemical bond. | | | | |

| Table 43 | | | | |
|---|---|---|---|---|
| 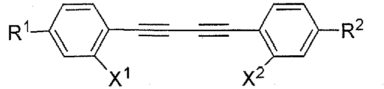 | | | | |

| Compound No. | X¹ | X² | R¹ | R² |
|---|---|---|---|---|
| 1254 | Br | Br | 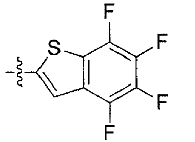 | 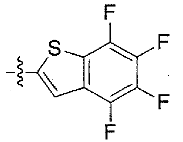 |
| 1257 | Br | Br | 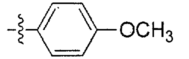 | 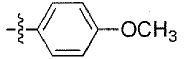 |
| 1258 | Br | Br | 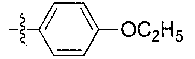 | 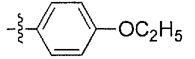 |
| 1259 | Br | Br | 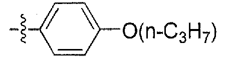 | 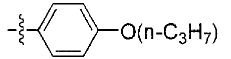 |
| 1260 | Br | Br | 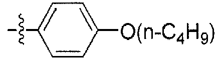 | 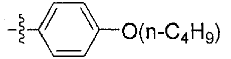 |
| 1262 | Br | Br | 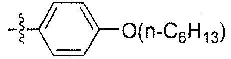 | 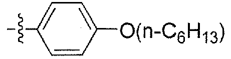 |
| 1264 | Br | Br | 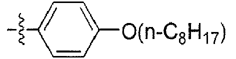 | 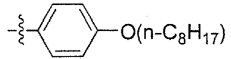 |

| | | | | |
|---|---|---|---|---|
| Dashed line indicates a chemical bond. | | | | |

| Table 44 | | | | |
|---|---|---|---|---|
| 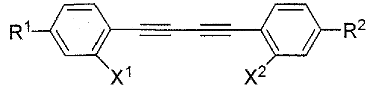 | | | | |

| Compound No. | X¹ | X² | R¹ | R² |
|---|---|---|---|---|
| 1265 | Br | Br | 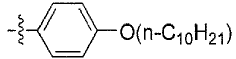 | 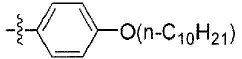 |
| 1267 | Br | Br | 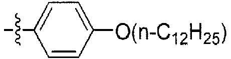 | 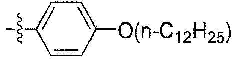 |
| 1268 | Br | Br | 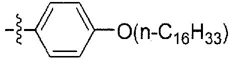 | 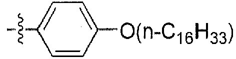 |
| 1269 | Br | Br | 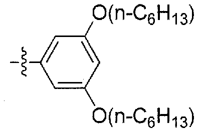 | 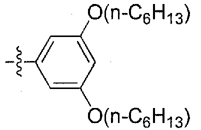 |
| 1271 | Br | Br | 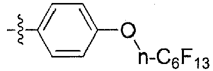 | 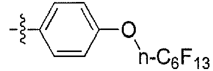 |
| 1272 | Br | Br | 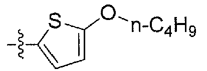 | 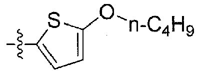 |
| 1273 | Br | Br | 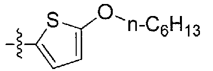 | 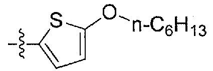 |
| 1275 | Br | Br | 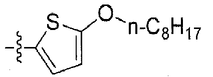 | 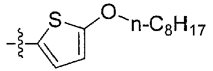 |
| 1276 | Br | Br | 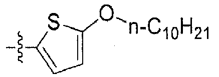 | 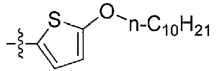 |
| 1278 | Br | Br | 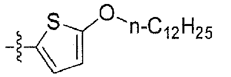 | 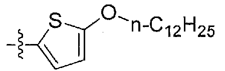 |
| 1279 | Br | Br | 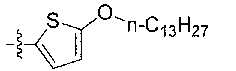 | 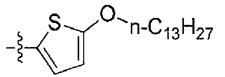 |

| | | | | |
|---|---|---|---|---|
| Dashed line indicates a chemical bond. | | | | |

| Table 45 | | | | |
|---|---|---|---|---|
| 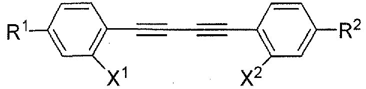 | | | | |

| Compound No. | X¹ | X² | R¹ | R² |
|---|---|---|---|---|
| 1280 | Br | Br | 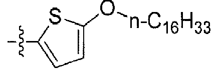 | 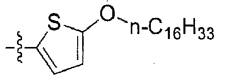 |
| 1281 | Br | Br | 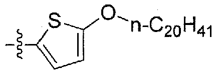 | 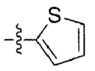 |
| 1282 | Br | Br | 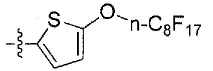 | 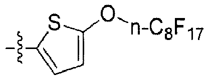 |
| 1283 | Br | Br | 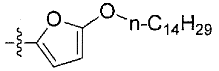 | 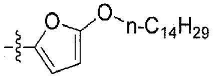 |
| 1284 | Br | Br | 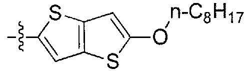 | 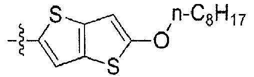 |

| | | | | |
|---|---|---|---|---|
| Dashed line indicates a chemical bond. | | | | |

| Table 46 | | | | |
|---|---|---|---|---|
| 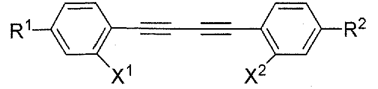 | | | | |

| Compound No. | X¹ | X² | R¹ | R² |
|---|---|---|---|---|
| 1292 | Br | Br | 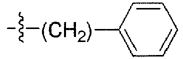 | 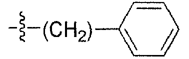 |
| 1293 | Br | Br | 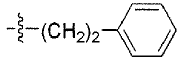 | 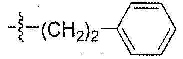 |
| 1294 | Br | Br | 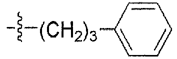 | 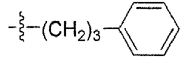 |
| 1295 | Br | Br | 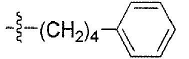 | 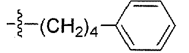 |
| 1296 | Br | Br | 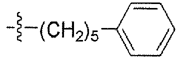 | 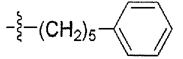 |
| 1297 | Br | Br | 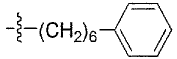 | 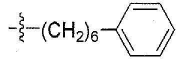 |
| 1298 | Br | Br | 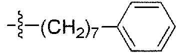 | 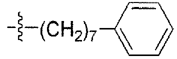 |
| 1299 | Br | Br | 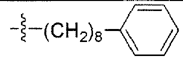 | 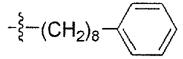 |
| 1300 | Br | Br | 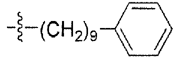 | 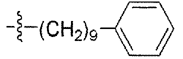 |
| 1301 | Br | Br | 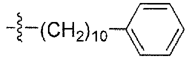 | 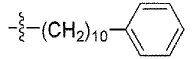 |
| 1302 | Br | Br | 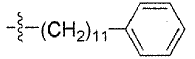 | 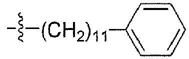 |
| 1303 | Br | Br | 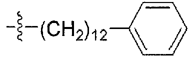 | 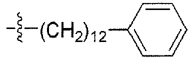 |
| 1304 | Br | Br | 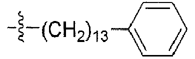 | 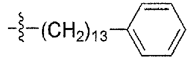 |
| 1305 | Br | Br | 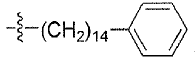 | 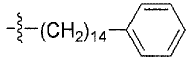 |

| | | | | |
|---|---|---|---|---|
| Dashed line indicates a chemical bond. | | | | |

| Table 47-1 | | | | |
|---|---|---|---|---|
| 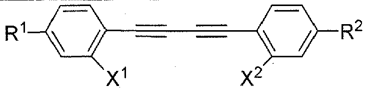 | | | | |

| Compound No. | X¹ | X² | R¹ | R² |
|---|---|---|---|---|
| 1306 | Br | Br | 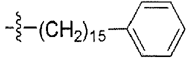 | 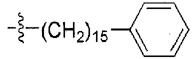 |
| 1307 | Br | Br | 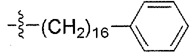 | 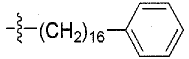 |
| 1308 | Br | Br | 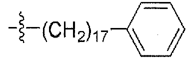 | 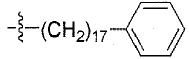 |
| 1309 | Br | Br | 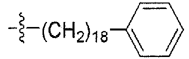 | 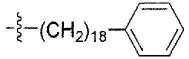 |
| 1310 | Br | Br | 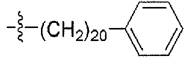 | 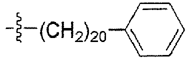 |
| 1311 | Br | Br | 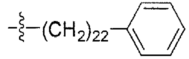 | 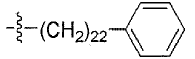 |
| 1312 | Br | Br | 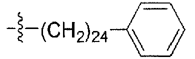 | 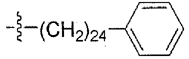 |
| 1313 | Br | Br | 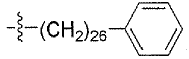 | 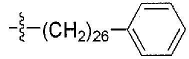 |
| 1314 | Br | Br | 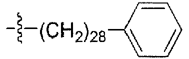 | 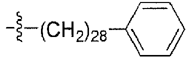 |
| 1315 | Br | Br | 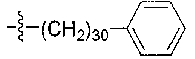 | 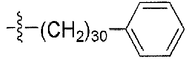 |

| | | | | |
|---|---|---|---|---|
| Dashed line indicates a chemical bond. | | | | |

| Table 47-2 | | | | |
|---|---|---|---|---|
| 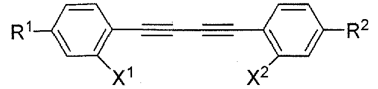 | | | | |

| Compound No. | X¹ | X² | R¹ | R² |
|---|---|---|---|---|
| 1316 | Br | Br | 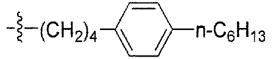 | 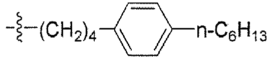 |
| 1317 | Br | Br | 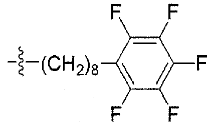 | 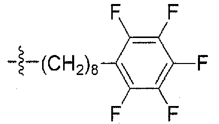 |
| 1318 | Br | Br | 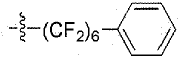 | 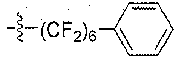 |
| 1319 | Br | Br | 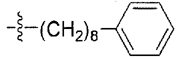 | n-C₆H₁₃ |
| 1320 | Br | Br | 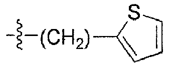 | 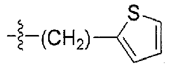 |
| 1321 | Br | Br | 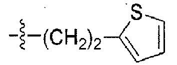 | 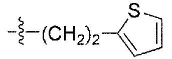 |
| 1322 | Br | Br | 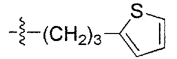 | 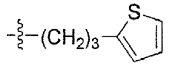 |
| 1323 | Br | Br | 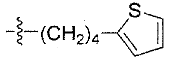 | 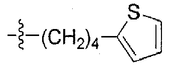 |
| 1324 | Br | Br | 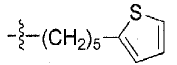 | 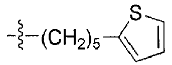 |

| | | | | |
|---|---|---|---|---|
| Dashed line indicates a chemical bond. | | | | |

| Table 48-1 | | | | |
|---|---|---|---|---|
| 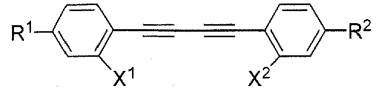 | | | | |

| Compound No. | X¹ | X² | R¹ | R² |
|---|---|---|---|---|
| 1325 | Br | Br | 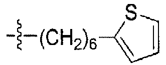 | 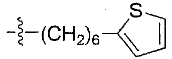 |
| 1326 | Br | Br | 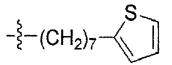 | 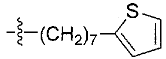 |
| 1327 | Br | Br | 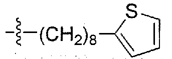 | 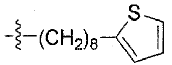 |
| 1328 | Br | Br | 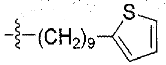 | 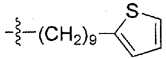 |
| 1329 | Br | Br | 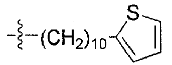 | 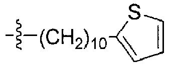 |
| 1330 | Br | Br | 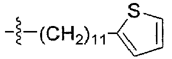 | 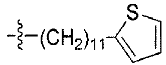 |
| 1331 | Br | Br | 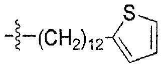 | 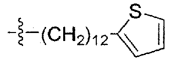 |
| 1332 | Br | Br | 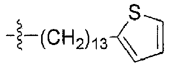 | 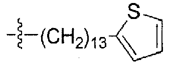 |
| 1333 | Br | Br | 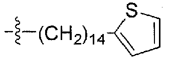 | 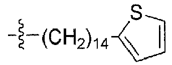 |
| 1334 | Br | Br | 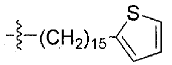 | 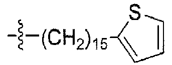 |

| | | | | |
|---|---|---|---|---|
| Dashed line indicates a chemical bond. | | | | |

| Table 48-2 | | | | |
|---|---|---|---|---|
| 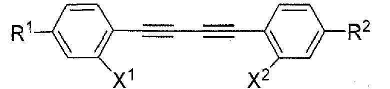 | | | | |

| Compound No. | X¹ | X² | R¹ | R² |
|---|---|---|---|---|
| 1335 | Br | Br | 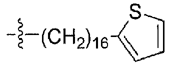 | 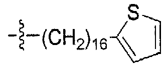 |
| 1336 | Br | Br | 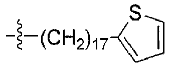 | 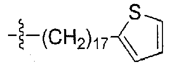 |
| 1337 | Br | Br | 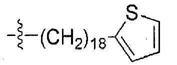 | 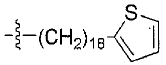 |
| 1338 | Br | Br | 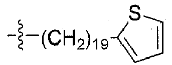 | 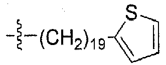 |
| 1339 | Br | Br | 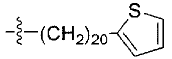 | 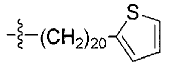 |
| 1340 | Br | Br | 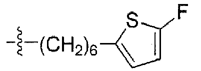 | 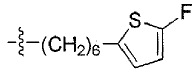 |
| 1341 | Br | Br | 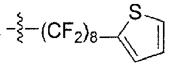 | 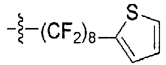 |
| 1342 | Br | Br | 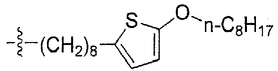 | 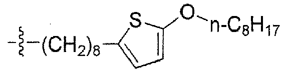 |
| 1343 | Br | Br | 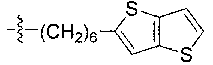 | 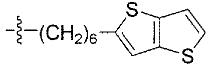 |

| | | | | |
|---|---|---|---|---|
| Dashed line indicates a chemical bond. | | | | |

| Table 49 | | | | |
|---|---|---|---|---|
| 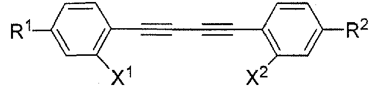 | | | | |

| Compound No. | X¹ | X² | R¹ | R² |
|---|---|---|---|---|
| 1344 | Br | Br | 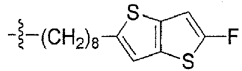 | 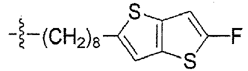 |
| 1345 | Br | Br | 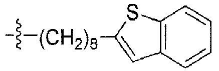 | 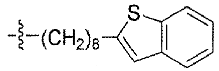 |
| 1346 | Br | Br | 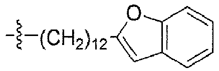 | 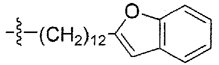 |
| 1347 | Br | Br | 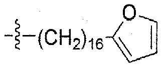 | 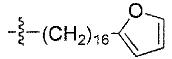 |
| 1348 | Br | Br | -Si(CH₃)₃ | -Si(CH₃)₃ |
| 1349 | Br | Br | -Si(C₂H₅)₃ | -Si(C₂H₅)₃ |
| 1350 | Br | Br | -Si(i-C₃H₇)₃ | -Si(i-C₃H₇)₃ |
| 1351 | Br | Br | -Si(CH₃)₂(t-C₄H₉) | -Si(CH₃)₂(t-C₄H₉) |
| 1352 | Br | Br | -Si(CH₃)₂(n-C₆H₁₃) | -Si(CH₃)₂(n-C₆H₁₃) |
| 1353 | Br | Br | -Si(CH₃)₂(n-C₁₂H₂₅) | -Si(CH₃)₂(n-C₁₂H₂₅) |

| | | | | |
|---|---|---|---|---|
| Dashed line indicates a chemical bond. | | | | |

| Table 50 | | | | |
|---|---|---|---|---|
| 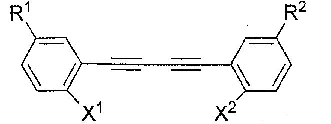 | | | | |

| Compound No. | X¹ | X² | R¹ | R² |
|---|---|---|---|---|
| 1354 | Br | Br | n-C₄H₉ | n-C₄H₉ |
| 1355 | Br | Br | s-C₄H₉ | s-C₄H₉ |
| 1356 | Br | Br | n-C₅H₁₁ | n-C₅H₁₁ |
| 1357 | Br | Br | 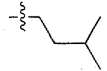 | 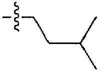 |
| 1358 | Br | Br | n-C₆H₁₃ | n-C₆H₁₃ |
| 1359 | Br | Br | 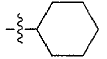 | 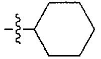 |
| 1360 | Br | Br | 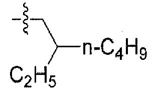 | 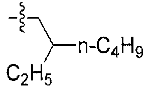 |
| 1361 | Br | Br | n-C₇H₁₅ | n-C₇H₁₅ |
| 1362 | Br | Br | n-C₈H₁₇ | n-C₈H₁₇ |
| 1363 | Br | Br | n-C₉H₁₉ | n-C₉H₁₉ |
| 1364 | Br | Br | n-C₁₀H₂₁ | n-C₁₀H₂₁ |

| | | | | |
|---|---|---|---|---|
| Dashed line indicates a chemical bond. | | | | |

| Table 51 | | | | |
|---|---|---|---|---|
| 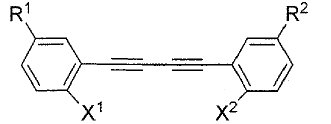 | | | | |

| Compound No. | X¹ | X² | R¹ | R² |
|---|---|---|---|---|
| 1365 | Br | Br | 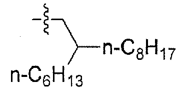 | 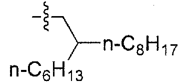 |
| 1366 | Br | Br | n-C₁₁H₂₃ | n-C₁₁H₂₃ |
| 1367 | Br | Br | n-C₁₂H₂₅ | n-C₁₂H₂₅ |
| 1368 | Br | Br | n-C₁₃H₂₇ | n-C₁₃H₂₇ |
| 1369 | Br | Br | n-C₁₄H₂₉ | n-C₁₄H₂₉ |
| 1370 | Br | Br | n-C₁₅H₃₁ | n-C₁₅H₃₁ |
| 1371 | Br | Br | n-C₁₆H₃₃ | n-C₁₆H₃₃ |
| 1372 | Br | Br | n-C₁₇H₃₅ | n-C₁₇H₃₅ |
| 1373 | Br | Br | n-C₁₈H₃₇ | n-C₁₈H₃₇ |
| 1374 | Br | Br | n-C₁₉H₃₉ | n-C₁₉H₃₉ |
| 1375 | Br | Br | n-C₂₀H₄₁ | n-C₂₀H₄₁ |
| 1376 | Br | Br | n-C₂₁H₄₃ | n-C₂₁H₄₃ |
| 1377 | Br | Br | n-C₂₂H₄₅ | n-C₂₂H₄₅ |
| 1378 | Br | Br | n-C₂₃H₄₇ | n-C₂₃H₄₇ |
| 1379 | Br | Br | n-C₂₄H₄₉ | n-C₂₄H₄₉ |
| 1380 | Br | Br | n-C₂₅H₅₁ | n-C₂₅H₅₁ |
| 1381 | Br | Br | n-C₂₆H₅₃ | n-C₂₆H₅₃ |
| 1382 | Br | Br | n-C₂₇H₅₅ | n-C₂₇H₅₅ |
| 1383 | Br | Br | n-C₂₈H₅₇ | n-C₂₈H₅₇ |
| 1384 | Br | Br | n-C₂₉H₅₉ | n-C₂₉H₅₉ |
| 1385 | Br | Br | n-C₃₀H₆₁ | n-C₃₀H₆₁ |
| 1386 | Br | Br | n-C₆F₁₃ | n-C₆F₁₃ |
| 1387 | Br | Br | n-C₈F₁₇ | n-C₈F₁₇ |
| 1388 | Br | Br | n-C₁₂F₂₅ | n-C₁₂F₂₅ |
| 1389 | Br | Br | n-C₁₆F₃₃ | n-C₁₆F₃₃ |
| 1390 | I | I | n-C₆H₁₃ | n-C₆H₁₃ |
| 1391 | Br | Br | n-C₈H₁₇ | n-C₈H₁₇ |
| 1392 | Br | Br | n-C₆H₁₃ | n-C₁₂H₂₅ |
| 1393 | Br | Br | O(n-C₄H₉) | O(n-C₄H₉) |
| 1394 | Br | Br | O(n-C₅H₁₁) | O(n-C₅H₁₁) |

| | | | | |
|---|---|---|---|---|
| Dashed line indicates a chemical bond. | | | | |

| Table 52 | | | | |
|---|---|---|---|---|
| 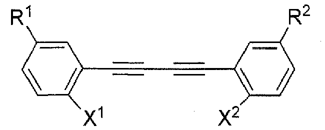 | | | | |

| Compound No. | X¹ | X² | R¹ | R² |
|---|---|---|---|---|
| 1396 | Br | Br | O(n-C₆H₁₃) | O(n-C₆H₁₃) |
| 1399 | Br | Br | O(n-C₇H₁₅) | O(n-C₇H₁₅) |
| 1400 | Br | Br | O(n-C₈H₁₇) | O(n-C₈H₁₇) |
| 1401 | Br | Br | O(n-C₉H₁₉) | O(n-C₉H₁₉) |
| 1402 | Br | Br | O(n-C₁₀H₂₁) | O(n-C₁₀H₂₁) |
| 1404 | Br | Br | O(n-C₁₁H₂₃) | O(n-C₁₁H₂₃) |
| 1405 | Br | Br | O(n-C₁₂H₂₅) | O(n-C₁₂H₂₅) |
| 1406 | Br | Br | O(n-C₁₃H₂₇) | O(n-C₁₃H₂₇) |
| 1407 | Br | Br | O(n-C₁₄H₂₉) | O(n-C₁₄H₂₉) |
| 1408 | Br | Br | O(n-C₁₅H₃₁) | O(n-C₁₅H₃₁) |
| 1409 | Br | Br | O(n-C₁₆H₃₃) | O(n-C₁₆H₃₃) |
| 1410 | Br | Br | O(n-C₁₇H₃₅) | O(n-C₁₇H₃₅) |
| 1411 | Br | Br | O(n-C₁₈H₃₇) | O(n-C₁₈H₃₇) |
| 1412 | Br | Br | O(n-C₁₉H₃₉) | O(n-C₁₉H₃₉) |
| 1413 | Br | Br | O(n-C₂₀H₄₁) | O(n-C₂₀H₄₁) |
| 1414 | Br | Br | O(n-C₂₁H₄₃) | O(n-C₂₁H₄₃) |
| 1415 | Br | Br | O(n-C₂₂H₄₅) | O(n-C₂₂H₄₅) |
| 1416 | Br | Br | O(n-C₂₃H₄₇) | O(n-C₂₃H₄₇) |
| 1417 | Br | Br | O(n-C₂₄H₄₉) | O(n-C₂₄H₄₉) |
| 1418 | Br | Br | O(n-C₂₅H₅₁) | O(n-C₂₅H₅₁) |
| 1419 | Br | Br | O(n-C₂₆H₅₃) | O(n-C₂₆H₅₃) |
| 1420 | Br | Br | O(n-C₂₇H₅₅) | O(n-C₂₇H₅₅) |
| 1421 | Br | Br | O(n-C₂₈H₅₇) | O(n-C₂₈H₅₇) |
| 1422 | Br | Br | O(n-C₂₉H₅₉) | O(n-C₂₉H₅₉) |
| 1423 | Br | Br | O(n-C₃₀H₆₁) | O(n-C₃₀H₆₁) |
| 1424 | Br | Br | O(n-C₆F₁₃) | O(n-C₆F₁₃) |
| 1425 | Br | Br | O(n-C₈F₁₇) | O(n-C₈F₁₇) |
| 1426 | Br | Br | O(n-C₁₂F₂₅) | O(n-C₁₂F₂₅) |
| 1427 | Br | Br | O(n-C₁₆F₃₃) | O(n-C₁₆F₃₃) |
| 1428 | I | I | O(n-C₆H₁₃) | O(n-C₆H₁₃) |
| 1429 | Cl | Cl | O(n-C₈H₁₇) | O(n-C₈H₁₇) |

| | | | | |
|---|---|---|---|---|
| Dashed line indicates a chemical bond. | | | | |

| Table 53 | | | | |
|---|---|---|---|---|
| 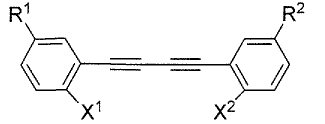 | | | | |

| Compound No. | X¹ | X² | R¹ | R² |
|---|---|---|---|---|
| 1430 | Br | Br | O(n-C₈H₁₇) | O(n-C₁₂H₂₅) |
| 1431 | Br | Br | 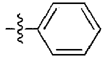 | 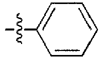 |
| 1432 | Br | Br | 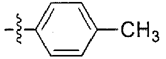 | 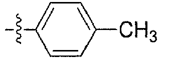 |
| 1433 | Br | Br | 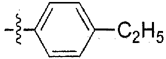 | 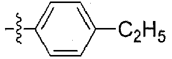 |
| 1434 | Br | Br | 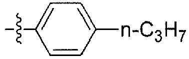 | 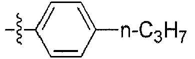 |
| 1435 | Br | Br | 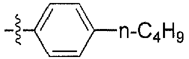 | 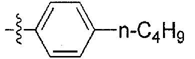 |
| 1437 | Br | Br | 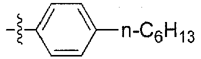 | 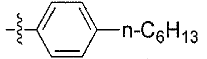 |
| 1439 | Br | Br | 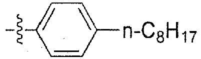 | 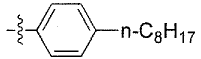 |
| 1440 | Br | Br | 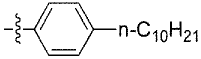 | 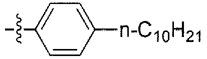 |
| 1442 | Br | Br | 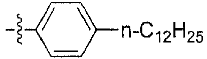 | 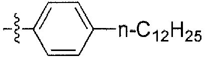 |
| 1443 | Br | Br | 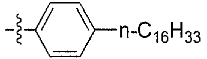 | 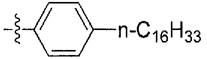 |
| 1444 | Br | Br | 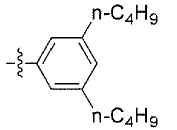 | 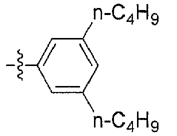 |

| | | | | |
|---|---|---|---|---|
| Dashed line indicates a chemical bond. | | | | |

| Table 54-1 | | | | |
|---|---|---|---|---|
| 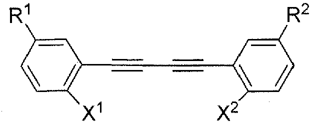 | | | | |

| Compound No. | X¹ | X² | R¹ | R² |
|---|---|---|---|---|
| 1446 | Br | Br | 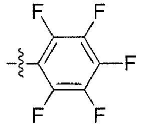 | 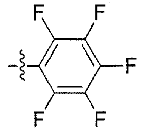 |
| 1447 | Br | Br | 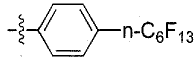 | 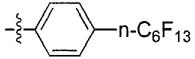 |
| 1448 | Br | Br | 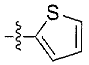 | 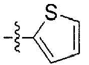 |
| 1449 | Br | Br | 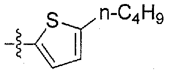 | 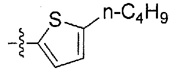 |
| 1450 | Br | Br | 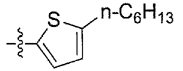 | 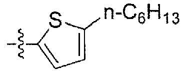 |
| 1452 | Br | Br | 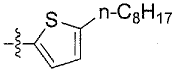 | 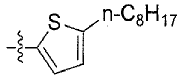 |
| 1453 | Br | Br | 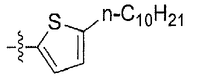 | 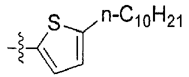 |

| | | | | |
|---|---|---|---|---|
| Dashed line indicates a chemical bond. | | | | |

| Table 54-2 | | | | |
|---|---|---|---|---|
| 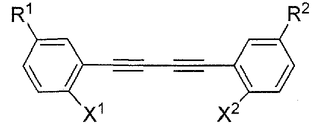 | | | | |

| Compound No. | X¹ | X² | R¹ | R² |
|---|---|---|---|---|
| 1455 | Br | Br | 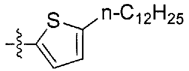 | 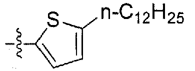 |
| 1456 | Br | Br | 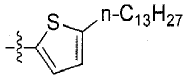 | 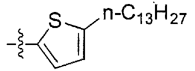 |
| 1457 | Br | Br | 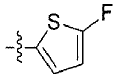 | 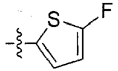 |
| 1458 | Br | Br | 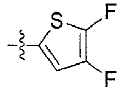 | 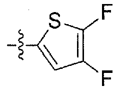 |
| 1459 | Br | Br | 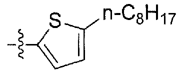 | 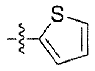 |
| 1460 | Br | Br | 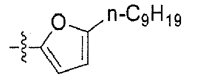 | 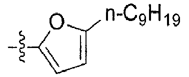 |

| | | | | |
|---|---|---|---|---|
| Dashed line indicates a chemical bond. | | | | |

| Table 55 | | | | |
|---|---|---|---|---|
| 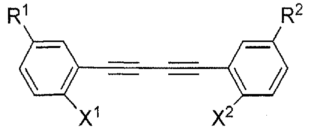 | | | | |

| Compound No. | X¹ | X² | R¹ | R² |
|---|---|---|---|---|
| 1461 | Br | Br | 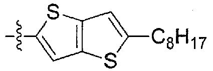 | 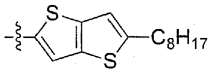 |
| 1467 | Br | Br | 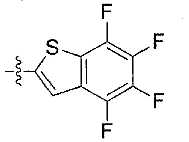 | 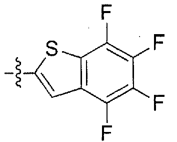 |
| 1470 | Br | Br | 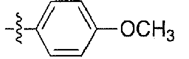 | 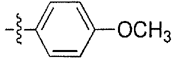 |
| 1471 | Br | Br | 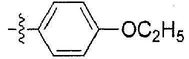 | 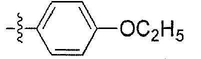 |
| 1472 | Br | Br | 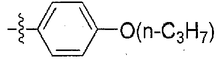 | 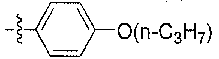 |
| 1473 | Br | Br | 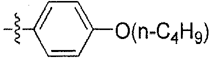 | 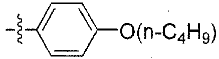 |
| 1475 | Br | Br | 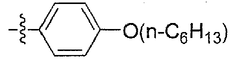 | 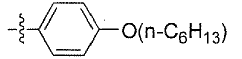 |

| | | | | |
|---|---|---|---|---|
| Dashed line indicates a chemical bond. | | | | |

| Table 56 | | | | |
|---|---|---|---|---|
| 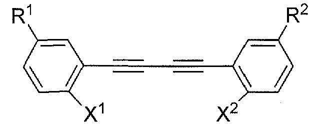 | | | | |

| Compound No. | X¹ | X² | R¹ | R² |
|---|---|---|---|---|
| 1477 | Br | Br | 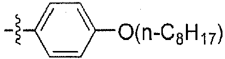 | 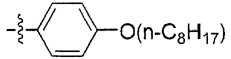 |
| 1478 | Br | Br | 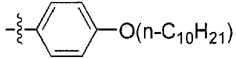 | 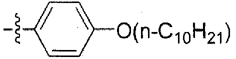 |
| 1480 | Br | Br | 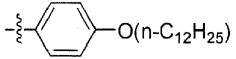 | 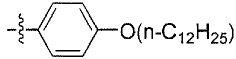 |
| 1481 | Br | Br | 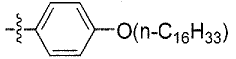 | 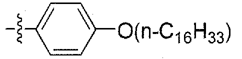 |
| 1482 | Br | Br | 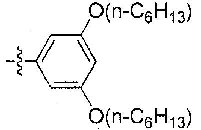 | 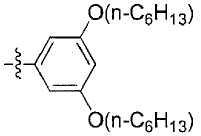 |
| 1484 | Br | Br | 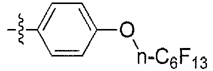 | 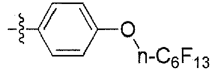 |
| 1485 | Br | Br | 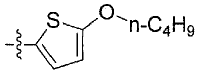 | 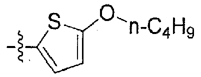 |
| 1486 | Br | Br | 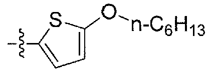 | 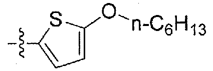 |
| 1488 | Br | Br | 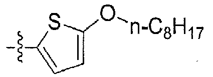 | 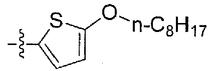 |
| 1489 | Br | Br | 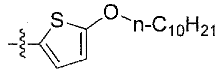 | 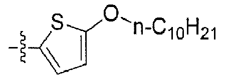 |

| | | | | |
|---|---|---|---|---|
| Dashed line indicates a chemical bond. | | | | |

| Table 57 | | | | |
|---|---|---|---|---|
| 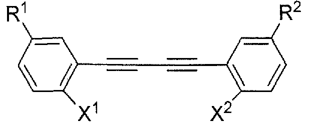 | | | | |

| Compound No. | X¹ | X² | R¹ | R² |
|---|---|---|---|---|
| 1491 | Br | Br | 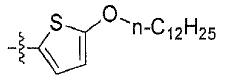 | 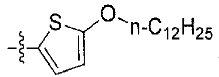 |
| 1492 | Br | Br | 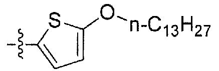 | 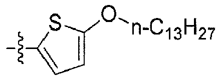 |
| 1493 | Br | Br | 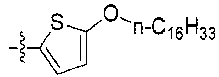 | 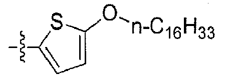 |
| 1494 | Br | Br | 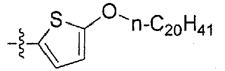 | 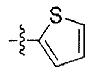 |
| 1495 | Br | Br | 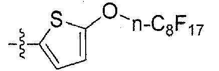 | 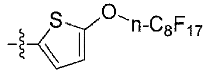 |
| 1496 | Br | Br | 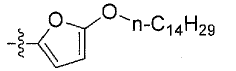 | 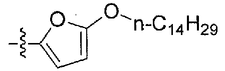 |
| 1497 | Br | Br | 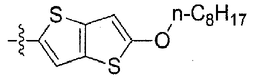 | 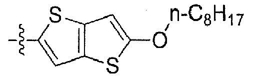 |

| | | | | |
|---|---|---|---|---|
| Dashed line indicates a chemical bond. | | | | |

| Table 58-1 | | | | |
|---|---|---|---|---|
| 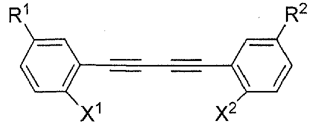 | | | | |

| Compound No. | X¹ | X² | R¹ | R² |
|---|---|---|---|---|
| 1505 | Br | Br | 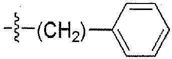 | 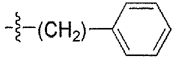 |
| 1506 | Br | Br | 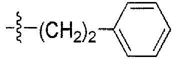 | 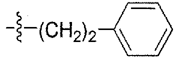 |
| 1507 | Br | Br | 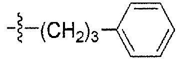 | 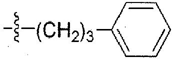 |
| 1508 | Br | Br | 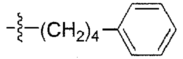 | 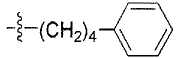 |
| 1509 | Br | Br | 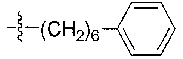 | 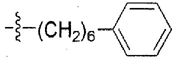 |
| 1510 | Br | Br | 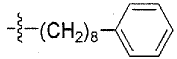 | 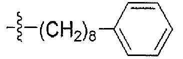 |
| 1511 | Br | Br | 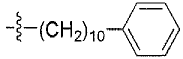 | 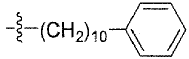 |
| 1512 | Br | Br | 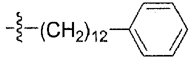 | 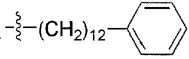 |
| 1513 | Br | Br | 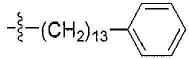 | 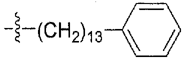 |

| | | | | |
|---|---|---|---|---|
| Dashed line indicates a chemical bond. | | | | |

| Table 58-2 | | | | |
|---|---|---|---|---|
| 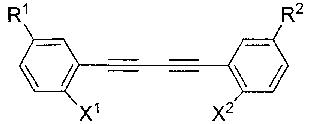 | | | | |

| Compound No. | X¹ | X² | R¹ | R² |
|---|---|---|---|---|
| 1514 | Br | Br | 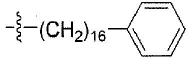 | 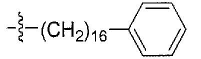 |
| 1515 | Br | Br | 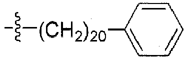 | 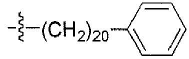 |
| 1516 | Br | Br | 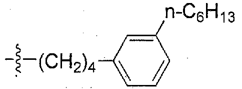 | 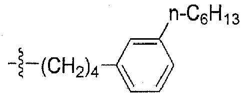 |
| 1517 | Br | Br | 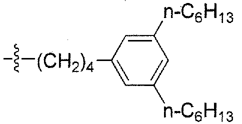 | 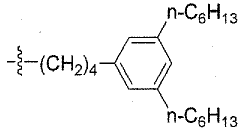 |
| 1518 | Br | Br | 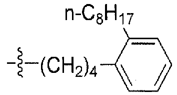 | 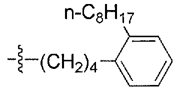 |
| 1519 | Br | Br | 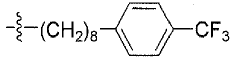 | 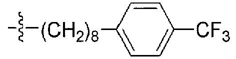 |
| 1520 | Br | Br | 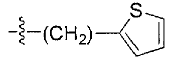 | 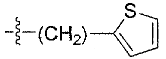 |
| 1521 | Br | Br | 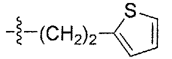 | 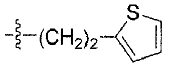 |

| | | | | |
|---|---|---|---|---|
| Dashed line indicates a chemical bond. | | | | |

| Table 59-1 | | | | |
|---|---|---|---|---|
| 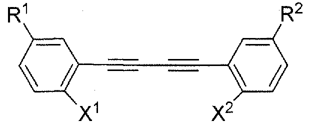 | | | | |

| Compound No. | X¹ | X² | R¹ | R² |
|---|---|---|---|---|
| 1522 | Br | Br | 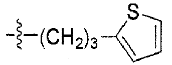 | 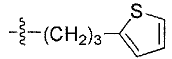 |
| 1523 | Br | Br | 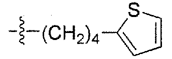 | 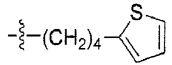 |
| 1524 | Br | Br | 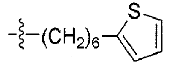 | 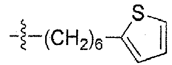 |
| 1525 | Br | Br | 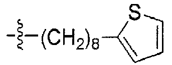 | 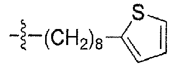 |
| 1526 | Br | Br | 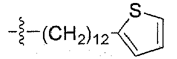 | 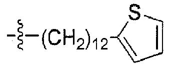 |
| 1527 | Br | Br | 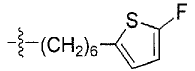 | 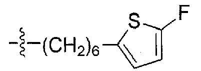 |
| 1528 | Br | Br | 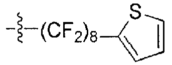 | 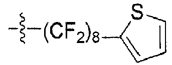 |
| 1529 | Br | Br | 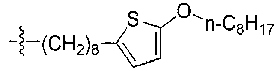 | 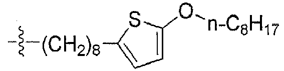 |
| 1530 | Br | Br | 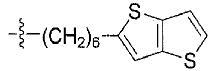 | 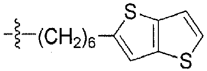 |

| | | | | |
|---|---|---|---|---|
| Dashed line indicates a chemical bond. | | | | |

| Table 59-2 | | | | |
|---|---|---|---|---|
| 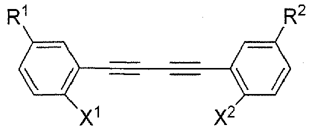 | | | | |

| Compound No. | X¹ | X² | R¹ | R² |
|---|---|---|---|---|
| 1531 | Br | Br | 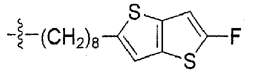 | 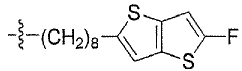 |
| 1532 | Br | Br | 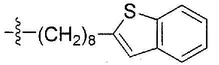 | 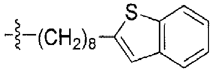 |
| 1533 | Br | Br | 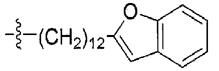 | 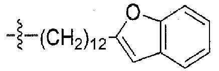 |
| 1534 | Br | Br | -Si(CH₃)₃ | -Si(CH₃)₃ |
| 1535 | Br | Br | -Si(C₂Hs)₃ | -Si(C₂Hs)₃ |
| 1536 | Br | Br | -Si(i-C₃H₇)₃ | -Si(i-C₃H₇)₃ |
| 1537 | Br | Br | -Si(CH₃)₂(t-C₄H₉) | -Si(CH₃)₂(t-C₄H₉) |
| 1538 | Br | Br | -Si(CH₃)₂(n-C₆H₁₃) | -Si(CH₃)₂(n-C₆H₁₃) |
| 1539 | Br | Br | -Si(CH₃)₂(n-C₁₂H₂₅) | -Sᵢ(CH₃)₂(n-C₁₂H₂₅) |

| | | | | |
|---|---|---|---|---|
| Dashed line indicates a chemical bond. | | | | |

| Table 60 | | | | |
|---|---|---|---|---|
| 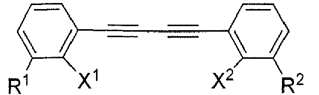 | | | | |

| Compound No. | X¹ | X² | R¹ | R² |
|---|---|---|---|---|
| 1540 | Br | Br | n-C₄H₉ | n-C₄H₉ |
| 1541 | Br | Br | n-C₅H₁₁ | n-C₅H₁₁ |
| 1542 | Br | Br | 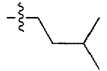 | 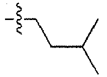 |
| 1543 | Br | Br | n-C₆H₁₃ | n-C₆H₁₃ |
| 1544 | Br | Br | 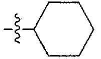 | 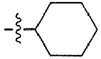 |
| 1545 | Br | Br | 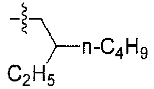 | 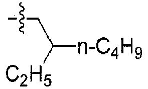 |
| 1546 | Br | Br | n-C₈H₁₇ | n-C₈H₁₇ |
| 1547 | Br | Br | 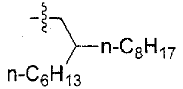 | 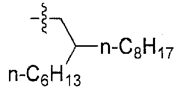 |
| 1548 | Br | Br | n-C₁₂H₂₅ | n-C₁₂H₂₅ |
| 1549 | Br | Br | n-C₁₃H₂₇ | n-C₁₃H₂₇ |
| 1550 | Br | Br | n-C₁₆H₃₃ | n-C₁₆H₃₃ |
| 1551 | Br | Br | n-C₁₈H₃₇ | n-C₁₈H₃₇ |
| 1552 | Br | Br | n-C₂₀H₄₁ | n-C₂₀H₄₁ |
| 1553 | Br | Br | n-C₂₅H₅₁ | n-C₂₅H₅₁ |
| 1554 | Br | Br | n-C₃₀H₆₁ | n-C₃₀H₆₁ |
| 1555 | Br | Br | n-C₆F₁₃ | n-C₆F₁₃ |
| 1556 | I | I | n-C₆H₁₃ | n-C₆H₁₃ |
| 1557 | Cl | Cl | n-C₈H₁₇ | n-C₈H₁₇ |
| 1558 | Br | Br | n-C₆H₁₃ | n-C₁₂H₂₅ |
| 1560 | Br | Br | O(n-C₆H₁₃) | O(n-C₆H₁₃) |
| 1563 | Br | Br | O(n-C₈H₁₇) | O(n-C₈H₁₇) |
| 1565 | Br | Br | O(n-C₁₂H₂₅) | O(n-C₁₂H₂₅) |

| | | | | |
|---|---|---|---|---|
| Dashed line indicates a chemical bond. | | | | |

| Table 61 | | | | |
|---|---|---|---|---|
| 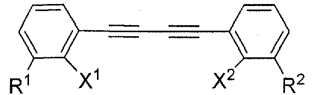 | | | | |

| Compound No. | X¹ | X² | R¹ | R² |
|---|---|---|---|---|
| 1566 | Br | Br | O(n-C₈F₁₇) | O(n-C₈F₁₇) |
| 1567 | I | I | O(n-C₆H₁₃) | O(n-C₆H₁₃) |
| 1568 | Br | Br | O(n-C₈H₁₇) | O(n-C₁₂H₂₅) |
| 1569 | Br | Br | 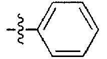 | 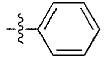 |
| 1571 | Br | Br | 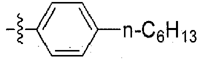 | 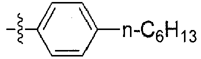 |
| 1573 | Br | Br | 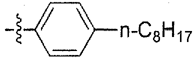 | 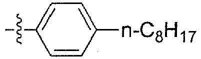 |
| 1574 | Br | Br | 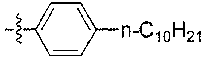 | 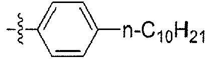 |
| 1576 | Br | Br | 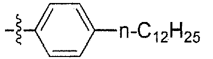 | 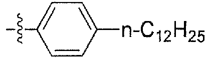 |
| 1577 | Br | Br | 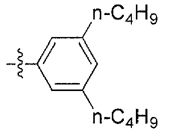 | 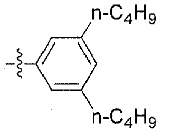 |
| 1579 | Br | Br | 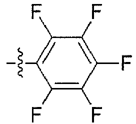 | 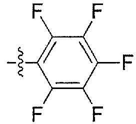 |
| 1580 | Br | Br | 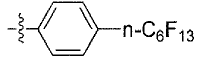 | 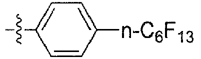 |
| 1581 | Br | Br | 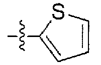 | 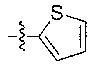 |

| | | | | |
|---|---|---|---|---|
| Dashed line indicates a chemical bond. | | | | |

| Table 62 | | | | |
|---|---|---|---|---|
| 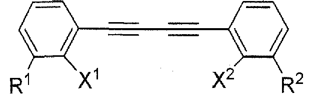 | | | | |

| Compound No. | X¹ | X² | R¹ | R² |
|---|---|---|---|---|
| 1582 | Br | Br | 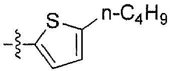 | 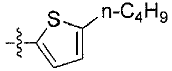 |
| 1583 | Br | Br | 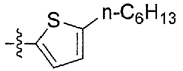 | 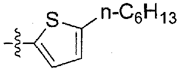 |
| 1585 | Br | Br | 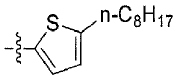 | 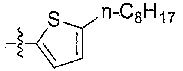 |
| 1586 | Br | Br | 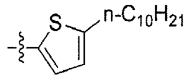 | 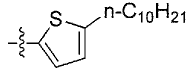 |
| 1588 | Br | Br | 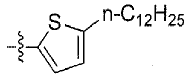 | 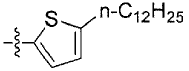 |
| 1589 | Br | Br | 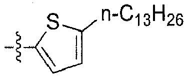 | 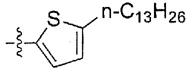 |
| 1590 | Br | Br | 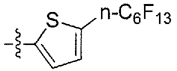 | 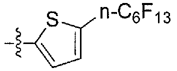 |
| 1591 | Br | Br | 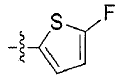 | 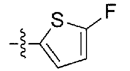 |
| 1592 | Br | Br | 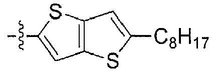 | 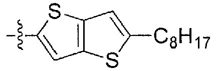 |
| 1596 | Br | Br | 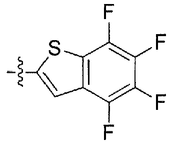 | 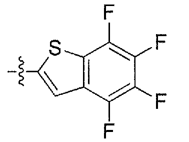 |

| | | | | |
|---|---|---|---|---|
| Dashed line indicates a chemical bond. | | | | |

| Table 63 | | | | |
|---|---|---|---|---|
| 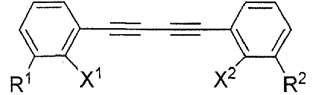 | | | | |

| Compound No. | X¹ | X² | R¹ | R² |
|---|---|---|---|---|
| 1599 | Br | Br | 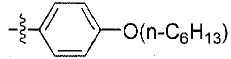 | 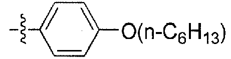 |
| 1601 | Br | Br | 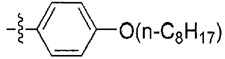 | 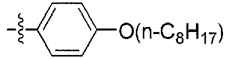 |
| 1603 | Br | Br | 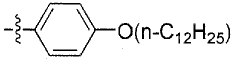 | 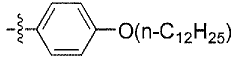 |
| 1604 | Br | Br | 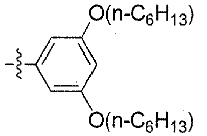 | 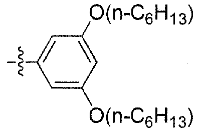 |
| 1606 | Br | Br | 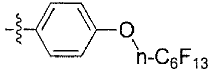 | 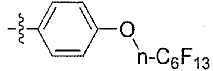 |
| 1607 | Br | Br | 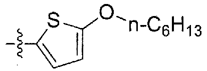 | 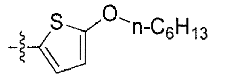 |
| 1609 | Br | Br | 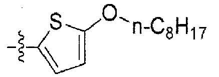 | 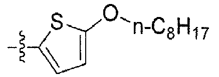 |
| 1610 | Br | Br | 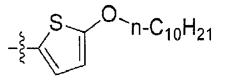 | 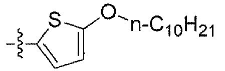 |

| | | | | |
|---|---|---|---|---|
| Dashed line indicates a chemical bond. | | | | |

| Table 64 | | | | |
|---|---|---|---|---|
| 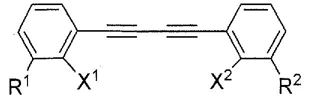 | | | | |

| Compound No. | X¹ | X² | R¹ | R² |
|---|---|---|---|---|
| 1612 | Br | Br | 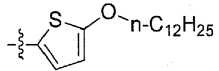 | 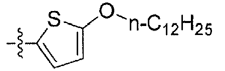 |
| 1613 | Br | Br | 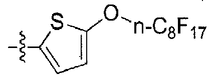 | 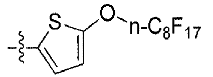 |
| 1614 | Br | Br | 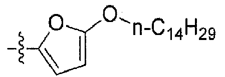 | 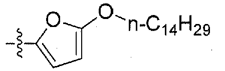 |
| 1615 | Br | Br | 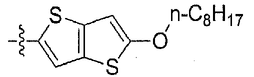 | 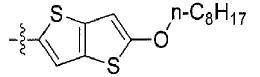 |
| 1619 | Br | Br | 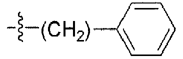 | 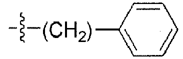 |
| 1620 | Br | Br | 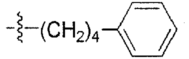 | 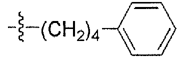 |
| 1621 | Br | Br | 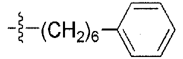 | 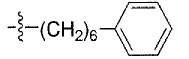 |
| 1622 | Br | Br | 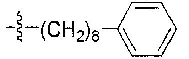 | 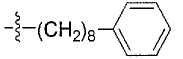 |
| 1623 | Br | Br | 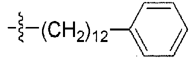 | 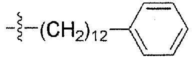 |
| 1624 | Br | Br | 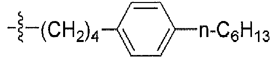 | 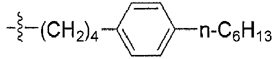 |
| 1625 | Br | Br | 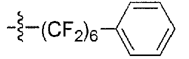 | 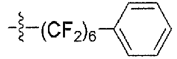 |

| | | | | |
|---|---|---|---|---|
| Dashed line indicates a chemical bond. | | | | |

| Table 65 | | | | |
|---|---|---|---|---|
| 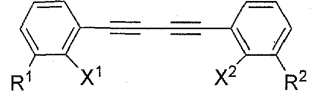 | | | | |

| Compound No. | X¹ | X² | R¹ | R² |
|---|---|---|---|---|
| 1626 | Br | Br | 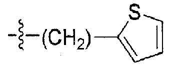 | 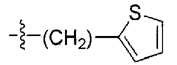 |
| 1627 | Br | Br | 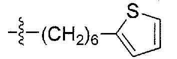 | 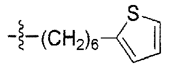 |
| 1628 | Br | Br | 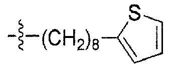 | 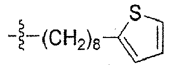 |
| 1629 | Br | Br | 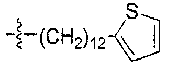 | 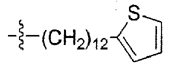 |
| 1630 | Br | Br | 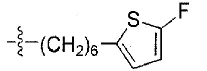 | 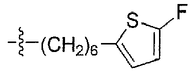 |
| 1631 | Br | Br | 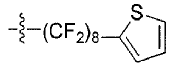 | 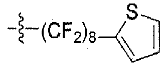 |
| 1632 | Br | Br | 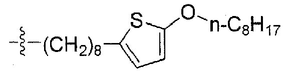 | 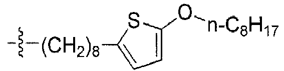 |
| 1633 | Br | Br | 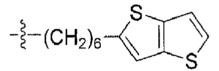 | 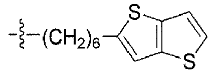 |
| 1634 | Br | Br | 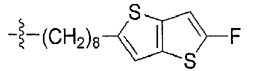 | 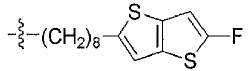 |
| 1635 | Br | Br | 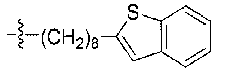 | 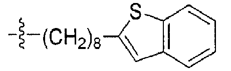 |
| 1636 | Br | Br | -Si(CH₃)₃ | -Si(CH₃)₃ |

| | | | | |
|---|---|---|---|---|
| Dashed line indicates a chemical bond. | | | | |

| Table 66 | | | | |
|---|---|---|---|---|
| 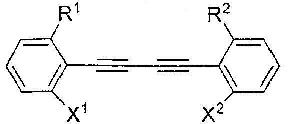 | | | | |

| Compound No. | X¹ | X² | R¹ | R² |
|---|---|---|---|---|
| 1637 | Br | Br | n-C₆H₁₃ | n-C₆H₁₃ |
| 1638 | Br | Br | n-C₁₂H₂₅ | n-C₁₂H₂₅ |
| 1639 | Br | Br | n-C₁₃H₂₇ | n-C₁₃H₂₇ |
| 1640 | Br | Br | n-C₆F₁₃ | n-C₆F₁₃ |
| 1641 | Br | Br | O(n-C₆H₁₃) | O(n-C₆H₁₃) |
| 1642 | Br | Br | 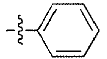 | 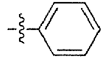 |
| 1643 | Br | Br | 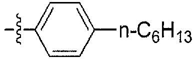 | 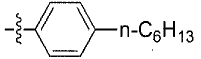 |
| 1644 | Br | Br | 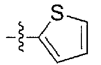 | 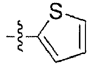 |
| 1645 | Br | Br | 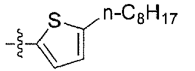 | 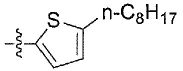 |
| 1649 | Br | Br | 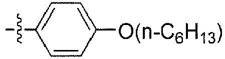 | 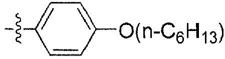 |
| 1653 | Br | Br | 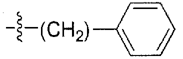 | 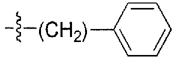 |
| 1654 | Br | Br | 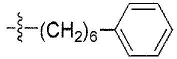 | 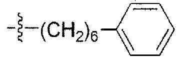 |

| | | | | |
|---|---|---|---|---|
| Dashed line indicates a chemical bond. | | | | |

| Table 67 | | | | |
|---|---|---|---|---|
| 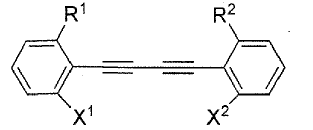 | | | | |

| Compound No. | X¹ | X² | R¹ | R² |
|---|---|---|---|---|
| 1655 | Br | Br | 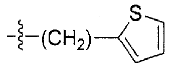 | 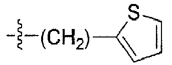 |
| 1656 | Br | Br | 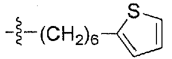 | 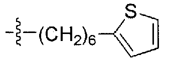 |
| 1657 | Br | Br | 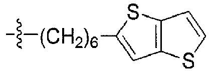 | 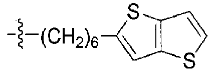 |
| 1658 | Br | Br | 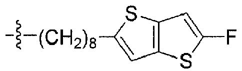 | 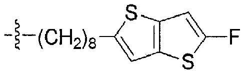 |
| 1659 | Br | Br | 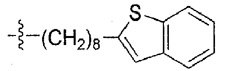 | 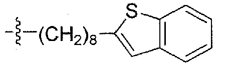 |
| 1660 | Br | Br | -Si(CH₃)₃ | -Si(CH₃)₃ |

| | | | | |
|---|---|---|---|---|
| Dashed line indicates a chemical bond. | | | | |

| Table 68 | | | | |
|---|---|---|---|---|
| 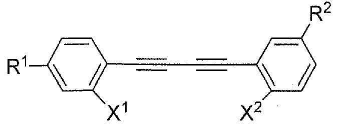 | | | | |

| Compound No. | X¹ | X² | R¹ | R² |
|---|---|---|---|---|
| 1661 | Br | Br | n-C₆H₁₃ | n-C₆H₁₃ |
| 1662 | Br | Br | O(n-C₆H₁₃) | O(n-C₆H₁₃) |
| 1663 | Br | Br | 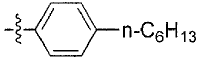 | 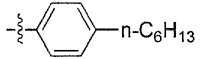 |
| 1664 | Br | Br | 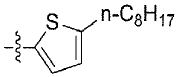 | 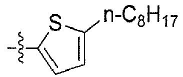 |

| | | | | |
|---|---|---|---|---|
| Dashed line indicates a chemical bond. | | | | |

| Table 69 | | | | |
|---|---|---|---|---|
| 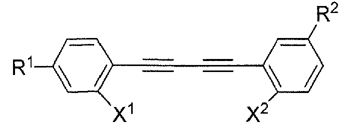 | | | | |

| Compound No. | X¹ | X² | R¹ | R² |
|---|---|---|---|---|
| 1670 | Br | Br | 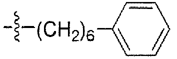 | 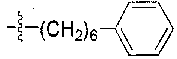 |
| 1671 | Br | Br | 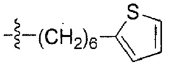 | 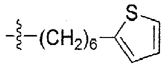 |
| 1672 | Br | Br | 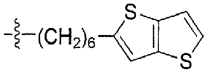 | 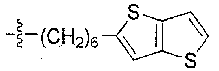 |
| 1673 | Br | Br | 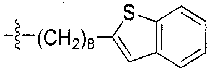 | 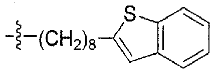 |
| 1674 | Br | Br | -Si(CH₃)₃ | -Si(CH₃)₃ |

| | | | | |
|---|---|---|---|---|
| Dashed line indicates a chemical bond. | | | | |

| Table 70 | | | | |
|---|---|---|---|---|
| 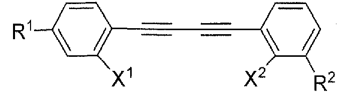 | | | | |

| Compound No. | X¹ | X² | R¹ | R² |
|---|---|---|---|---|
| 1675 | Br | Br | n-C₆H₁₃ | n-C₆H₁₃ |
| 1676 | Br | Br | O(n-C₆H₁₃) | O(n-C₆H₁₃) |
| 1677 | Br | Br | 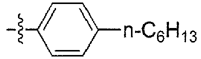 | 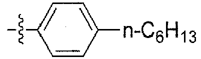 |
| 1678 | Br | Br | 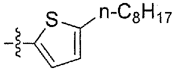 | 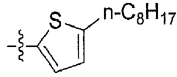 |

| | | | | |
|---|---|---|---|---|
| Dashed line indicates a chemical bond. | | | | |

| Table 71 | | | | |
|---|---|---|---|---|
| 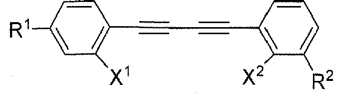 | | | | |

| Compound No. | X¹ | X² | R¹ | R² |
|---|---|---|---|---|
| 1685 | Br | Br | 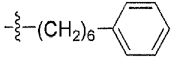 | 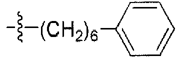 |
| 1686 | Br | Br | 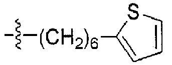 | 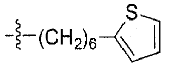 |
| 1687 | Br | Br | 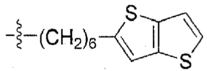 | 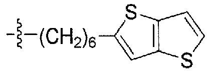 |
| 1688 | Br | Br | 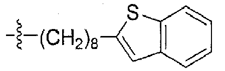 | 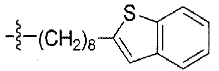 |
| 1689 | Br | Br | -Si(CH₃)₃ | -Si(CH₃)₃ |

| | | | | |
|---|---|---|---|---|
| Dashed line indicates a chemical bond. | | | | |

| Table 72 | | | | |
|---|---|---|---|---|
| 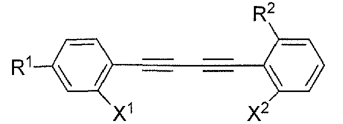 | | | | |

| Compound No. | X¹ | X² | R¹ | R² |
|---|---|---|---|---|
| 1690 | Br | Br | n-C₆H₁₃ | n-C₆H₁₃ |
| 1691 | Br | Br | 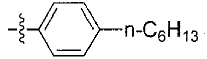 | 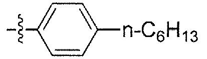 |
| 1694 | Br | Br | 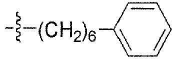 | 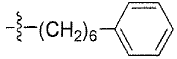 |
| 1695 | Br | Br | 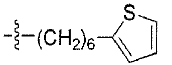 | 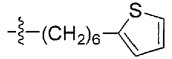 |
| 1696 | Br | Br | -Si(CH₃)₃ | -Si(CH₃)₃ |

| | | | | |
|---|---|---|---|---|
| Dashed line indicates a chemical bond. | | | | |

| Table 73 | | | | |
|---|---|---|---|---|
| 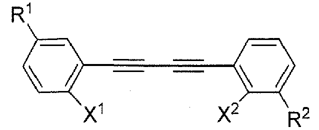 | | | | |

| Compound No. | X¹ | X² | R¹ | R2 |
|---|---|---|---|---|
| 1697 | Br | Br | n-C₆H₁₃ | n-C₆H₁₃ |
| 1698 | Br | Br | 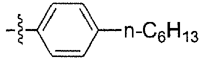 | 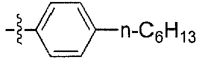 |
| 1701 | Br | Br | 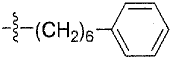 | 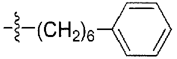 |
| 1702 | Br | Br | 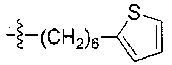 | 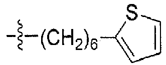 |
| 1703 | Br | Br | -Si(CH₃)₃ | -Si(CH₃)₃ |

| | | | | |
|---|---|---|---|---|
| Dashed line indicates a chemical bond. | | | | |

| Table 74 | | | | |
|---|---|---|---|---|
| 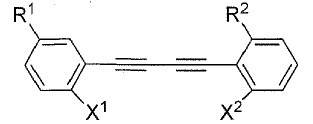 | | | | |

| Compound No. | X¹ | X² | R¹ | R² |
|---|---|---|---|---|
| 1704 | Br | Br | n-C₆H₁₃ | n-C₆H₁₃ |
| 1705 | Br | Br | 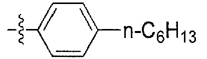 | 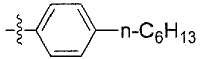 |
| 1708 | Br | Br | 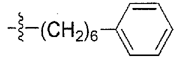 | 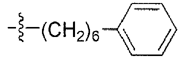 |
| 1709 | Br | Br | 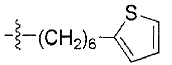 | 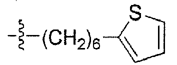 |
| 1710 | Br | Br | -Si(CH₃)₃ | -Si(CH₃)₃ |

| | | | | |
|---|---|---|---|---|
| Dashed line indicates a chemical bond. | | | | |

Specific examples of the compound (4-1) obtained in the present 2nd reaction are illustrated in the following tables.

| Table 75 | | | | | |
|---|---|---|---|---|---|
| 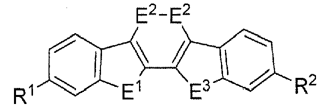 | | | | | |

| Compound No. | E¹ | E² | E³ | R¹ | R² |
|---|---|---|---|---|---|
| 571 | S | S | S | n-C₄H₉ | n-C₄H₉ |
| 572 | S | S | S | s-C₄H₉ | s-C4H₉ |
| 573 | S | S | S | n-C₅H₁₁ | n-C₅H₁₁ |
| 574 | S | S | S | 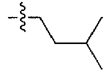 | 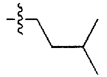 |
| 575 | S | S | S | n-C₆H₁₃ | n-C₆H₁₃ |
| 576 | S | S | S | 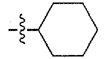 | 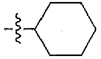 |
| 577 | S | S | S | 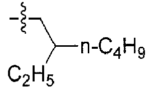 | 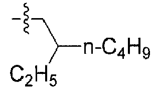 |
| 578 | S | S | S | n-C₇H₁₅ | n-C₇H₁₅ |
| 579 | S | S | S | n-C₈H₁₇ | n-C₈H₁₇ |
| 580 | S | S | S | n-C₉H₁₉ | n-C₉H₁₉ |
| 581 | S | S | S | n-C₁₀H₂₁ | n-C₁₀H₂₁ |
| 582 | S | S | S | 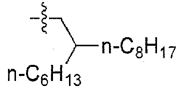 | 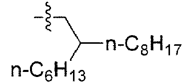 |
| 583 | S | S | S | n-C₁₁H₂₃ | n-C₁₁H₂₃ |
| 584 | S | S | S | n-C₁₂H₂₅ | n-C₁₂H₂₅ |
| 585 | S | S | S | n-C₁₃H₂₇ | n-C₁₃H₂₇ |

| | | | | | |
|---|---|---|---|---|---|
| Dashed line indicates a chemical bond. | | | | | |

| Table 76 | | | | | |
|---|---|---|---|---|---|
| 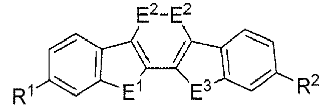 | | | | | |

| Compound No. | E¹ | E² | E³ | R¹ | R² |
|---|---|---|---|---|---|
| 586 | S | S | S | n-C₁₄H₂₉ | n-C₁₄H₂₉ |
| 587 | S | S | S | n-C₁₅H₃₁ | n-C₁₅H₃₁ |
| 588 | S | S | S | n-C₁₆H₃₃ | n-C₁₆H₃₃ |
| 589 | S | S | S | n-C₁₇H₃₅ | n-C₁₇H₃₅ |
| 590 | S | S | S | n-C₁₈H₃₇ | n-C₁₈H₃₇ |
| 591 | S | S | S | n-C₁₉H₃₉ | n-C₁₉H₃₉ |
| 592 | S | S | S | n-C₂₀H₄₁ | n-C₂₀H₄₁ |
| 593 | S | S | S | n-C₂₁H₄₃ | n-C₂₁H₄₃ |
| 594 | S | S | S | n-C₂₂H₄₅ | n-C₂₂H₄₅ |
| 595 | S | S | S | n-C₂₃H₄₇ | n-C₂₃H₄₇ |
| 596 | S | S | S | n-C₂₄H₄₉ | n-C₂₄H₄₉ |
| 597 | S | S | S | n-C₂₅H₅₁ | n-C₂₅H₅₁ |
| 598 | S | S | S | n-C₂₆H₅₃ | n-C₂₆H₅₃ |
| 599 | S | S | S | n-C₂₇H₅₅ | n-C₂₇H₅₅ |
| 600 | S | S | S | n-C₂₈H₅₇ | n-C₂₈H₅₇ |
| 601 | S | S | S | n-C₂₉H₅₉ | n-C₂₉H₅₉ |
| 602 | S | S | S | n-C₃₀H₆₁ | n-C₃₀H₆₁ |
| 603 | S | S | S | n-C₆F₁₃ | n-C₆F₁₃ |
| 604 | S | S | S | n-C₈F₁₇ | n-C₈F₁₇ |
| 605 | S | S | S | n-C₁₂F₂₅ | n-C₁₂F₂₅ |
| 606 | S | S | S | n-C₆H₁₃ | H |
| 607 | S | Se | S | n-C₆H₁₃ | n-C₆H₁₃ |
| 608 | Se | S | Se | n-C₈H₁₇ | n-C₈H₁₇ |
| 609 | S | S | S | n-C₆H₁₃ | n-C₁₂H₂₅ |
| 610 | S | S | S | O(n-C₄H₉) | O(n-C₄H₉) |
| 611 | S | S | S | O(n-C₅H₁₁) | O(n-C₅H₁₁) |
| 613 | S | S | S | O(n-C₆H₁₃) | O(n-C₆H₁₃) |
| 616 | S | S | S | O(n-C₇H₁₅) | O(n-C₇H₁₅) |
| 617 | S | S | S | O(n-C₈H₁₇) | O(n-C₈H₁₇) |
| 618 | S | S | S | O(n-C₉H₁₉) | O(n-C₉H₁₉) |
| 619 | S | S | S | O(n-C₁₀H₂₁) | O(n-C₁₀H₂₁) |

| | | | | | |
|---|---|---|---|---|---|
| Dashed line indicates a chemical bond. | | | | | |

| Table 77 | | | | | |
|---|---|---|---|---|---|
| 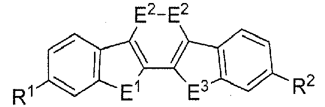 | | | | | |

| Compound No. | E¹ | E² | E³ | R¹ | R² |
|---|---|---|---|---|---|
| 621 | S | S | S | O(n-C₁₁H₂₃) | O(n-C₁₁H₂₃) |
| 622 | S | S | S | O(n-C₁₂H₂₅) | 0(n-C₁₂H₂₅) |
| 623 | S | S | S | O(n-C₁₃H₂₇) | O(n-C₁₃H₂₇) |
| 624 | S | S | S | O(n-C₁₄H₂₉) | O(n-C₁₄H₂₉) |
| 625 | S | S | S | O(n-C₁₅H₃₁) | O(n-C₁₅H₃₁) |
| 626 | S | S | S | O(n-C₁₆H₃₃) | O(n-C₁₆H₃₃) |
| 627 | S | S | S | O(n-C₁₇H₃₅) | O(n-C₁₇H₃₅) |
| 628 | S | S | S | O(n-C₁₈H₃₇) | O(n-C₁₈H₃₇) |
| 629 | S | S | S | O(n-C₁₉H₃₉) | O(n-C₁₉H₃₉) |
| 630 | S | S | S | O(n-C₂₀H₄₁) | O(n-C₂₀H₄₁) |
| 631 | S | S | S | O(n-C₂₁H₄₃) | O(n-C₂₁H₄₃) |
| 632 | S | S | S | O(n-C₂₂H₄₅) | O(n-C₂₂H₄₅) |
| 633 | S | S | S | O(n-C₂₃H₄₇) | O(n-C₂₃H₄₇) |
| 634 | S | S | S | O(n-C₂₄H₄₉) | O(n-C₂₄H₄₉) |
| 635 | S | S | S | O(n-C₂₅H₅₁) | O(n-C₂₅H₅₁) |
| 636 | S | S | S | O(n-C₂₆H₅₃) | O(n-C₂₆H₅₃) |
| 637 | S | S | S | O(n-C₂₇H₅₅) | O(n-C₂₇H₅₅) |
| 638 | S | S | S | O(n-C₂₈H₅₇) | O(n-C₂₈H₅₇) |
| 639 | S | S | S | O(n-C₂₉H₅₉) | O(n-C₂₉H₅₉) |
| 640 | S | S | S | O(n-C₃₀H₆₁) | O(n-C₃₀H₆₁) |
| 641 | S | S | S | O(n-C₆F₁₃) | O(n-C₆F₁₃) |
| 642 | S | S | S | O(n-C₈F₁₇) | O(n-C₈F₁₇) |
| 643 | S | S | S | O(n-C₁₂F₂₅) | O(n-C₁₂F₂₅) |
| 644 | S | S | S | O(n-C₈H₁₇) | H |
| 645 | S | Se | S | O(n-C₆H₁₃) | O(n-C₆H₁₃) |
| 646 | Se | S | Se | O(n-C₈H₁₇) | O(n-C₈H₁₇) |
| 647 | S | S | S | O(n-C₈H₁₇) | O(n-C₁₂H₂₅) |
| 648 | S | S | S | 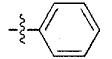 | 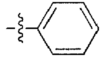 |
| 649 | S | S | S | 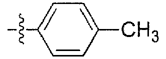 | 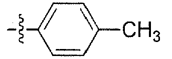 |

| | | | | | |
|---|---|---|---|---|---|
| Dashed line indicates a chemical bond. | | | | | |

| Table 78 | | | | | |
|---|---|---|---|---|---|
| 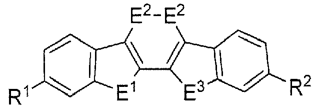 | | | | | |

| Compound No. | E¹ | E² | E³ | R¹ | R² |
|---|---|---|---|---|---|
| 650 | S | S | S | 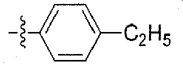 | 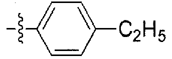 |
| 651 | S | S | S | 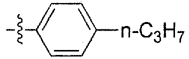 | 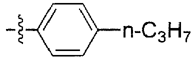 |
| 652 | S | S | S | 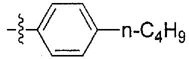 | 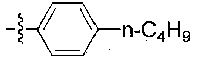 |
| 654 | S | S | S | 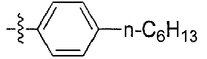 | 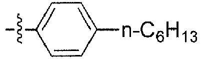 |
| 656 | S | S | S | 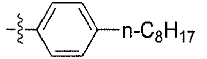 | 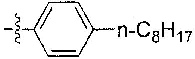 |
| 657 | S | S | S | 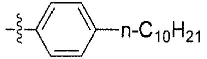 | 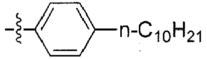 |
| 659 | S | S | S | 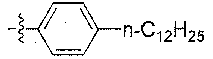 | 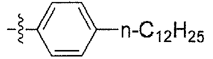 |
| 660 | S | S | S | 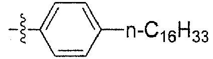 | 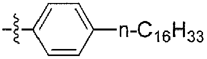 |
| 661 | S | S | S | 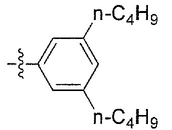 | 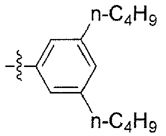 |
| 663 | S | S | S | 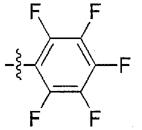 | 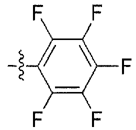 |
| 664 | S | S | S | 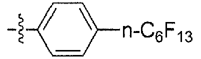 | 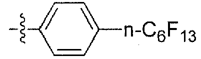 |
| 665 | S | S | S | 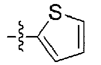 | 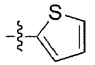 |

| | | | | | |
|---|---|---|---|---|---|
| Dashed line indicates a chemical bond. | | | | | |

| Table 79 | | | | | |
|---|---|---|---|---|---|
| 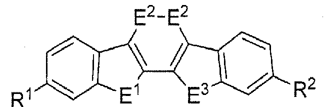 | | | | | |

| Compound No. | E¹ | E² | E³ | R¹ | R² |
|---|---|---|---|---|---|
| 666 | S | S | S | 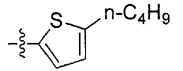 | 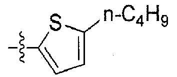 |
| 667 | S | S | S | 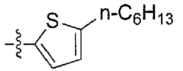 | 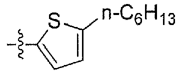 |
| 669 | S | S | S | 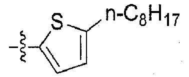 | 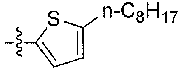 |
| 670 | S | S | S | 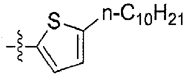 | 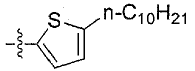 |
| 672 | S | S | S | 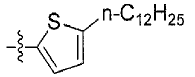 | 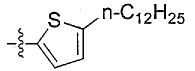 |
| 673 | S | S | S | 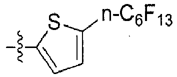 | 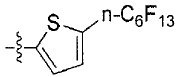 |
| 674 | S | S | S | 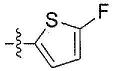 | 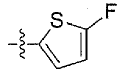 |
| 675 | S | S | S | 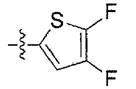 | 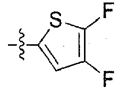 |
| 676 | S | S | S | 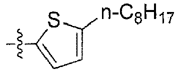 | 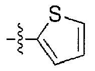 |
| 677 | S | Se | S | 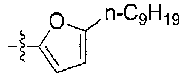 | 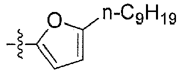 |
| 678 | S | S | S | 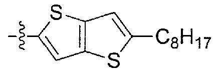 | 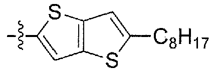 |

| | | | | | |
|---|---|---|---|---|---|
| Dashed line indicates a chemical bond. | | | | | |

| Table 80 | | | | | |
|---|---|---|---|---|---|
| 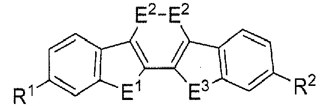 | | | | | |

| Compound No. | E¹ | E² | E³ | R¹ | R² |
|---|---|---|---|---|---|
| 684 | S | S | S | 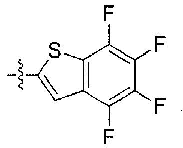 | 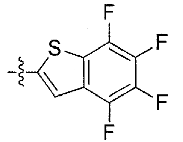 |
| 687 | S | S | S | 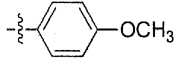 | 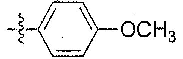 |
| 688 | S | S | S | 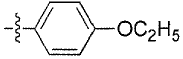 | 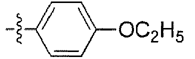 |
| 689 | S | S | S | 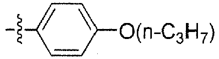 | 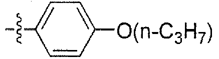 |
| 690 | S | S | S | 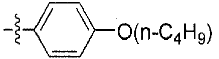 | 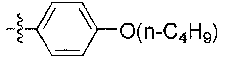 |
| 692 | S | S | S | 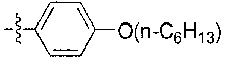 | 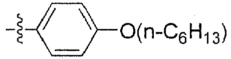 |
| 694 | S | S | S | 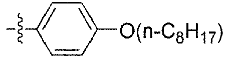 | 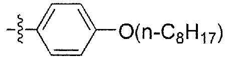 |

| | | | | | |
|---|---|---|---|---|---|
| Dashed line indicates a chemical bond. | | | | | |

| Table 81 | | | | | |
|---|---|---|---|---|---|
| 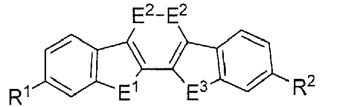 | | | | | |

| Compound No. | E¹ | E² | E³ | R¹ | R² |
|---|---|---|---|---|---|
| 695 | S | S | S | 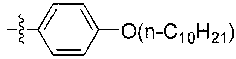 | 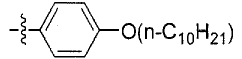 |
| 697 | S | S | S | 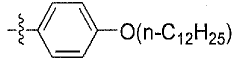 | 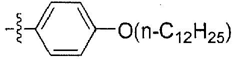 |
| 698 | S | S | S | 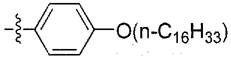 | 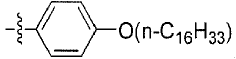 |
| 699 | S | S | S | 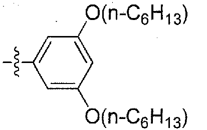 | 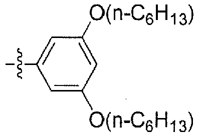 |
| 701 | S | S | S | 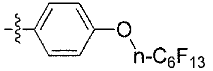 | 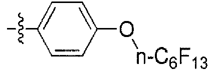 |
| 702 | S | S | S | 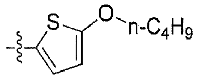 | 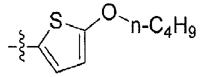 |
| 703 | S | S | S | 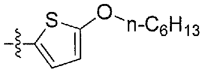 | 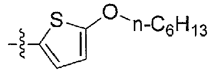 |
| 705 | S | S | S | 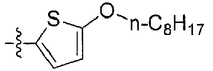 | 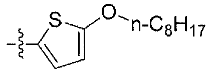 |
| 706 | S | S | S | 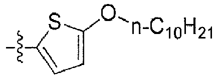 | 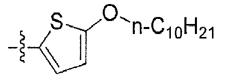 |
| 708 | S | S | S | 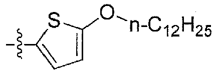 | 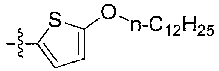 |
| 709 | S | S | S | 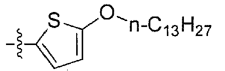 | 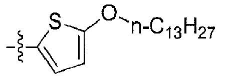 |

| | | | | | |
|---|---|---|---|---|---|
| Dashed line indicates a chemical bond. | | | | | |

| Table 82 | | | | | |
|---|---|---|---|---|---|
| 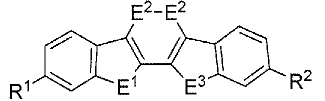 | | | | | |

| Compound No. | E¹ | E² | E³ | R¹ | R² |
|---|---|---|---|---|---|
| 710 | S | Se | S | 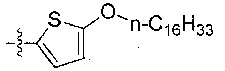 | 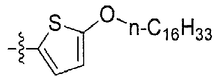 |
| 711 | Se | S | Se | 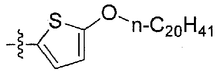 | 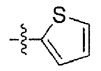 |
| 712 | S | S | S | 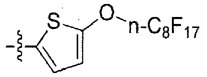 | 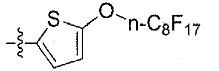 |
| 713 | S | Se | S | 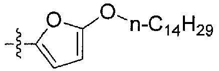 | 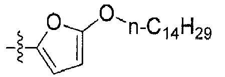 |
| 714 | S | S | S | 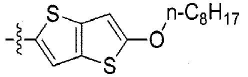 | 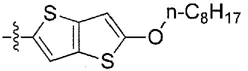 |

| | | | | | |
|---|---|---|---|---|---|
| Dashed line indicates a chemical bond. | | | | | |

| Table 83 | | | | | |
|---|---|---|---|---|---|
| 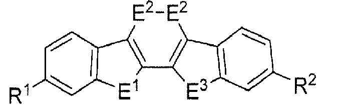 | | | | | |

| Compound No. | E¹ | E² | E³ | R¹ | R² |
|---|---|---|---|---|---|
| 722 | S | S | S | 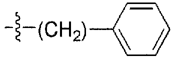 | 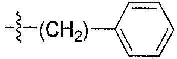 |
| 723 | S | S | S | 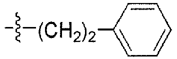 | 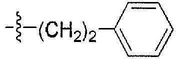 |
| 724 | S | S | S | 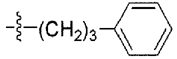 | 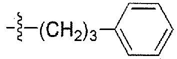 |
| 725 | S | S | S | 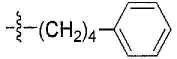 | 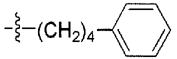 |
| 726 | S | S | S | 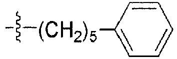 | 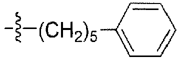 |
| 727 | S | S | S | 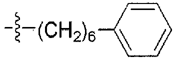 | 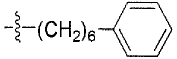 |
| 728 | S | S | S | 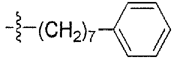 | 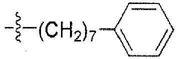 |
| 729 | S | S | S | 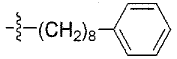 | 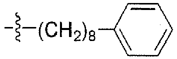 |
| 730 | S | S | S | 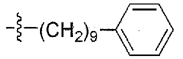 | 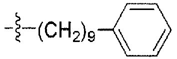 |
| 731 | S | S | S | 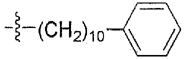 | 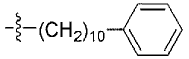 |
| 732 | S | S | S | 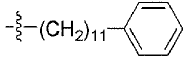 | 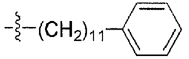 |
| 733 | S | S | S | 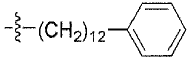 | 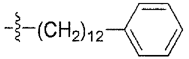 |
| 734 | S | S | S | 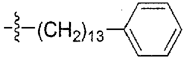 | 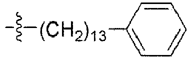 |
| 735 | S | S | S | 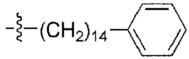 | 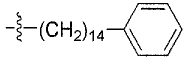 |

| | | | | | |
|---|---|---|---|---|---|
| Dashed line indicates a chemical bond. | | | | | |

| Table 84-1 | | | | | |
|---|---|---|---|---|---|
| 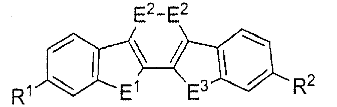 | | | | | |

| Compound No. | E¹ | E² | E³ | R¹ | R² |
|---|---|---|---|---|---|
| 736 | S | S | S | 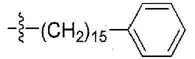 | 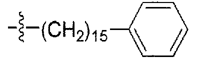 |
| 737 | S | S | S | 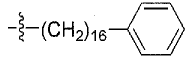 | 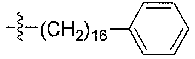 |
| 738 | S | S | S | 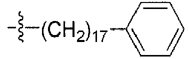 | 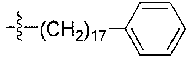 |
| 739 | S | S | S | 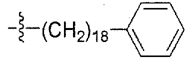 | 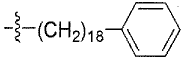 |
| 740 | S | S | S | 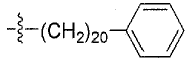 | 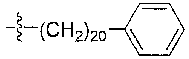 |
| 741 | S | S | S | 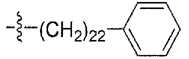 | 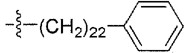 |
| 742 | S | S | S | 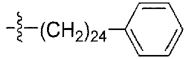 | 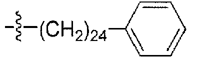 |
| 743 | S | S | S | 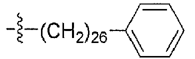 | 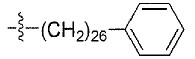 |
| 744 | S | S | S | 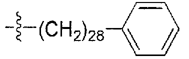 | 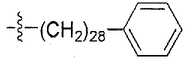 |
| 745 | S | S | S | 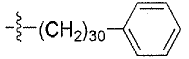 | 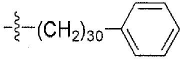 |

| | | | | | |
|---|---|---|---|---|---|
| Dashed line indicates a chemical bond. | | | | | |

| Table 84-2 | | | | | |
|---|---|---|---|---|---|
| 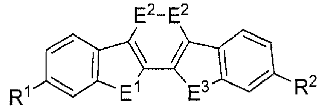 | | | | | |

| Compound No. | E¹ | E² | E³ | R¹ | R² |
|---|---|---|---|---|---|
| 746 | S | S | S | 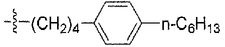 | 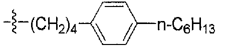 |
| 747 | S | S | S | 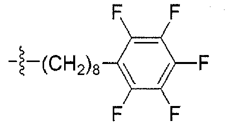 | 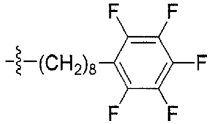 |
| 748 | S | S | S | 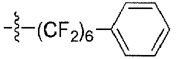 | 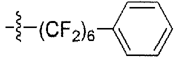 |
| 749 | Se | Se | Se | 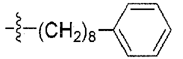 | n-C₆H₁₃ |
| 750 | S | S | S | 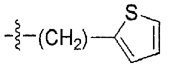 | 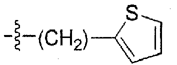 |
| 751 | S | S | S | 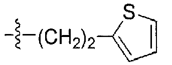 | 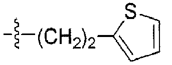 |
| 752 | S | Se | S | 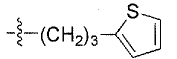 | 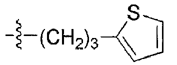 |
| 753 | S | Se | S | 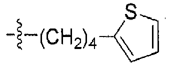 | 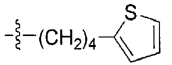 |
| 754 | Se | S | Se | 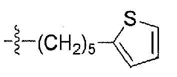 | 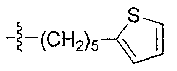 |

| | | | | | |
|---|---|---|---|---|---|
| Dashed line indicates a chemical bond. | | | | | |

| Table 85-1 | | | | | |
|---|---|---|---|---|---|
| 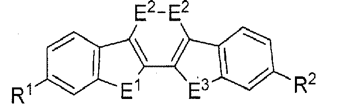 | | | | | |

| Compound No. | E¹ | E² | E³ | R¹ | R² |
|---|---|---|---|---|---|
| 755 | S | S | S | 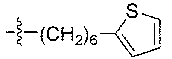 | 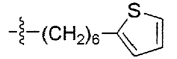 |
| 756 | S | S | S | 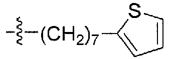 | 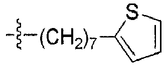 |
| 757 | S | S | S | 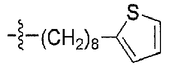 | 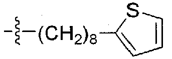 |
| 758 | S | S | S | 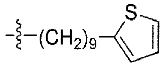 | 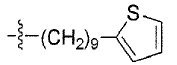 |
| 759 | S | S | S | 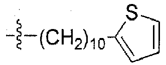 | 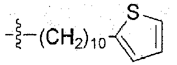 |
| 760 | S | S | S | 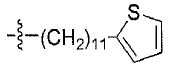 | 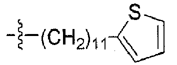 |
| 761 | S | S | S | 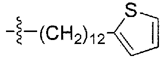 | 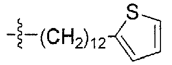 |
| 762 | S | S | S | 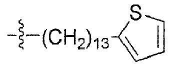 | 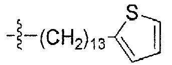 |
| 763 | S | S | S | 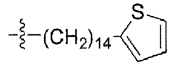 | 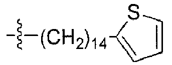 |
| 764 | S | S | S | 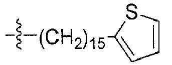 | 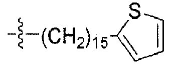 |

| | | | | | |
|---|---|---|---|---|---|
| Dashed line indicates a chemical bond. | | | | | |

| Table 85-2 | | | | | |
|---|---|---|---|---|---|
| 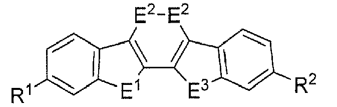 | | | | | |

| Compound No. | E¹ | E² | E³ | R¹ | R² |
|---|---|---|---|---|---|
| 765 | S | S | S | 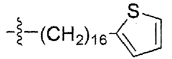 | 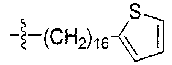 |
| 766 | S | S | S | 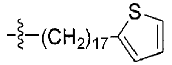 | 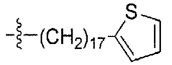 |
| 767 | S | S | S | 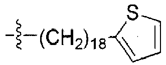 | 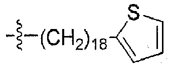 |
| 768 | S | S | S | 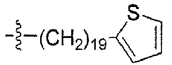 | 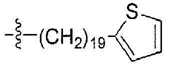 |
| 769 | S | S | S | 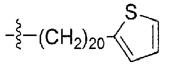 | 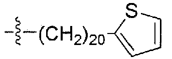 |
| 770 | S | S | S | 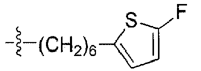 | 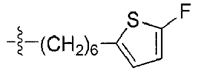 |
| 771 | S | S | S | 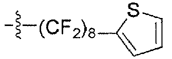 | 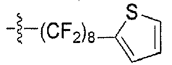 |
| 772 | S | S | S | 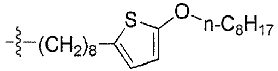 | 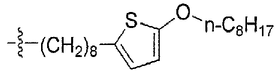 |
| 773 | S | S | S | 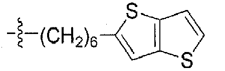 | 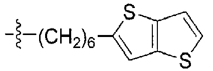 |

| | | | | | |
|---|---|---|---|---|---|
| Dashed line indicates a chemical bond. | | | | | |

| Table 86 | | | | | |
|---|---|---|---|---|---|
| 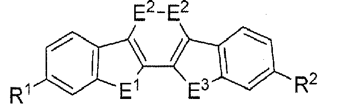 | | | | | |

| Compound No. | E¹ | E² | E³ | R¹ | R² |
|---|---|---|---|---|---|
| 774 | S | S | S | 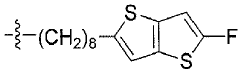 | 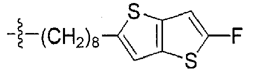 |
| 775 | S | S | S | 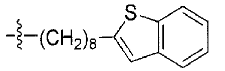 | 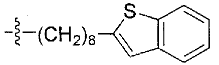 |
| 776 | Se | Se | Se | 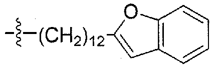 | 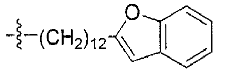 |
| 777 | S | S | S | 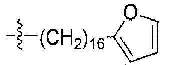 | 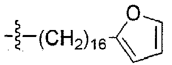 |
| 778 | S | S | S | -Si(CH₃)₃ | -Si(CH₃)₃ |
| 779 | S | S | S | -Si(C₂H₅)₃ | -Si(C₂H₅)₃ |
| 780 | S | S | S | -Si(i-C₃H₇)₃ | -Si(i-C₃H₇)₃ |
| 781 | S | S | S | -Si(CH₃)₂(t-C₄H₉) | -Si(CH₃)₂(t-C₄H₉) |
| 782 | S | S | S | -Si(CH₃)₂(n-C₆Hₗ₃) | -Si(CH₃)₂(n-C₆H₁₃) |
| 783 | S | S | S | -Si(CH₃)₂(n-C₁₂H₂₅) | -Si(CH₃)₂(n-C₁₂H₂₅) |

| | | | | | |
|---|---|---|---|---|---|
| Dashed line indicates a chemical bond. | | | | | |

| Table 87 | | | | | |
|---|---|---|---|---|---|
| 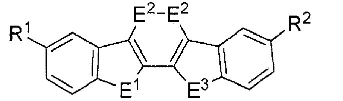 | | | | | |

| Compound No. | E¹ | E² | E³ | R¹ | R² |
|---|---|---|---|---|---|
| 784 | S | S | S | n-C₄H₉ | n-C₄H₉ |
| 785 | S | S | S | s-C₄H₉ | s-C₄H₉ |
| 786 | S | S | S | n-C₅H₁₁ | n-C₅H₁₁ |
| 787 | S | S | S | 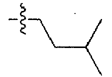 | 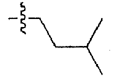 |
| 788 | S | S | S | n-C₆H₁₃ | n-C₆H₁₃ |
| 789 | S | S | S | 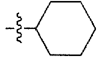 | 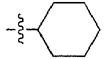 |
| 790 | S | S | S | 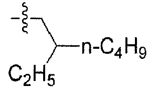 | 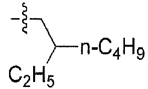 |
| 791 | S | S | S | n-C₇H₁₅ | n-C₇H₁₅ |
| 792 | S | S | S | n-C₈H₁₇ | n-C₈H₁₇ |
| 793 | S | S | S | n-C₉H₁₉ | n-C₉H₁₉ |
| 794 | S | S | S | n-C₁₀H₂₁ | n-C₁₀H₂₁ |

| | | | | | |
|---|---|---|---|---|---|
| Dashed line indicates a chemical bond. | | | | | |

| Table 88 | | | | | |
|---|---|---|---|---|---|
| 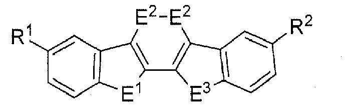 | | | | | |

| Compound No. | E¹ | E² | E³ | R¹ | R² |
|---|---|---|---|---|---|
| 795 | S | S | S | 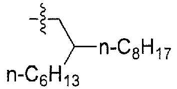 | 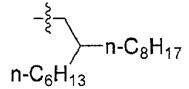 |
| 796 | S | S | S | n-C₁₁H₂₃ | n-C₁₁H₂₃ |
| 797 | S | S | S | n-C₁₂H₂₅ | n-C₁₂H₂₅ |
| 798 | S | S | S | n-C₁₃H₂₇ | n-C₁₃H₂₇ |
| 799 | S | S | S | n-C₁₄H₂₉ | n-C₁₄H₂₉ |
| 800 | S | S | S | n-C₁₅H₃₁ | n-C₁₅H₃₁ |
| 801 | S | S | S | n-C₁₆H₃₃ | n-C₁₆H₃₃ |
| 802 | S | S | S | n-C₁₇H₃₅ | n-C₁₇H₃₅ |
| 803 | S | S | S | n-C₁₈H₃₇ | n-C₁₈H₃₇ |
| 804 | S | S | S | n-C₁₉H₃₉ | n-C₁₉H₃₉ |
| 805 | S | S | S | n-C₂₀H₄₁ | n-C₂₀H₄₁ |
| 806 | S | S | S | n-C₂₁H₄₃ | n-C₂₁H₄₃ |
| 807 | S | S | S | n-C₂₂H₄₅ | n-C₂₂H₄₅ |
| 808 | S | S | S | n-C₂₃H₄₇ | n-C₂₃H₄₇ |
| 809 | S | S | S | n-C₂₄H₄₉ | n-C₂₄H₄₉ |
| 810 | S | S | S | n-C₂₅H₅₁ | n-C₂₅H₅₁ |
| 811 | S | S | S | n-C₂₆H₅₃ | n-C₂₆H₅₃ |
| 812 | S | S | S | n-C₂₇H₅₅ | n-C₂₇H₅₅ |
| 813 | S | S | S | n-C₂₈H₅₇ | n-C₂₈H₅₇ |
| 814 | S | S | S | n-C₂₉H₅₉ | n-C₂₉H₅₉ |
| 815 | S | S | S | n-C₃₀H₆₁ | n-C₃₀H₆₁ |
| 816 | S | S | S | n-C₆F₁₃ | n-C₆F₁₃ |
| 817 | S | S | S | n-C₈F₁₇ | n-C₈F₁₇ |
| 818 | S | S | S | n-C₁₂F₂₅ | n-C₁₂F₂₅ |
| 819 | S | S | S | n-C₆H₁₃ | H |
| 820 | S | Se | S | n-C₆H₁₃ | n-C₆H₁₃ |
| 821 | Se | S | Se | n-C₈H₁₇ | n-C₈H₁₇ |
| 822 | S | S | S | n-C₆H₁₃ | n-C₁₂H₂₅ |
| 823 | S | S | S | O(n-C₄H₉) | O(n-C₄H₉) |
| 824 | S | S | S | O(n-C₅H₁₁) | O(n-CsH₁₁) |

| | | | | | |
|---|---|---|---|---|---|
| Dashed line indicates a chemical bond. | | | | | |

| Table 89 | | | | | |
|---|---|---|---|---|---|
| 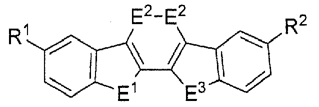 | | | | | |

| Compound No. | E¹ | E² | E³ | R¹ | R² |
|---|---|---|---|---|---|
| 826 | S | S | S | O(n-C₆H₁₃) | O(n-C₆H₁₃) |
| 829 | S | S | S | O(n-C₇H₁₅) | O(n-C₇H₁₅) |
| 830 | S | S | S | O(n-C₈H₁₇) | O(n-C₈H₁₇) |
| 831 | S | S | S | O(n-C₉H₁₉) | O(n-C₉H₁₉) |
| 832 | S | S | S | O(n-C₁₀H₂₁) | O(n-C₁₀H₂₁) |
| 834 | S | S | S | O(n-C₁₁H₂₃) | O(n-C₁₁H₂₃) |
| 835 | S | S | S | O(n-C₁₂H₂₅) | O(n-C₁₂H₂₅) |
| 836 | S | S | S | O(n-C₁₃H₂₇) | O(n-C₁₃H₂₇) |
| 837 | S | S | S | O(n-C₁₄H₂₉) | O(n-C₁₄H₂₉) |
| 838 | S | S | S | O(n-C₁₅H₃₁) | O(n-C₁₅H₃₁) |
| 839 | S | S | S | O(n-C₁₆H₃₃) | O(n-C₁₆H₃₃) |
| 840 | S | S | S | O(n-C₁₇H₃₅) | O(n-C₁₇H₃₅) |
| 841 | S | S | S | O(n-C₁₈H₃₇) | O(n-C₁₈H₃₇) |
| 842 | S | S | S | O(n-C₁₉H₃₉) | O(n-C₁₉H₃₉) |
| 843 | S | S | S | O(n-C₂₀H₄₁) | O(n-C₂₀H₄₁) |
| 844 | S | S | S | O(n-C₂₁H₄₃) | O(n-C₂₁H₄₃) |
| 845 | S | S | S | O(n-C₂₂H₄₅) | O(n-C₂₂H₄₅) |
| 846 | S | S | S | O(n-C₂₃H₄₇) | O(n-C₂₃H₄₇) |
| 847 | S | S | S | O(n-C₂₄H₄₉) | O(n-C₂₄H₄₉) |
| 848 | S | S | S | O(n-C₂₅H₅₁) | O(n-C₂₅H₅₁) |
| 849 | S | S | S | O(n-C₂₆H₅₃) | O(n-C₂₆H₅₃) |
| 850 | S | S | S | O(n-C₂₇H₅₅) | O(n-C₂₇H₅₅) |
| 851 | S | S | S | O(n-C₂₈H₅₇) | O(n-C₂₈H₅₇) |
| 852 | S | S | S | O(n-C₂₉H₅₉) | O(n-C₂₉Hs₉) |
| 853 | S | S | S | O(n-C₃₀H₆₁) | O(n-C₃₀H₆₁) |
| 854 | S | S | S | O(n-C₆F₁₃) | O(n-C₆F₁₃) |
| 855 | S | S | S | O(n-C₈F₁₇) | O(n-C₈F₁₇) |
| 856 | S | S | S | O(n-C₁₂F₂₅) | O(n-C₁₂F₂₅) |
| 857 | S | S | S | O(n-C₁₆F₃₃) | O(n-C₁₆F₃₃) |
| 858 | S | Se | S | O(n-C₆H₁₃) | O(n-C₆H₁₃) |
| 859 | Se | S | Se | O(n-C₈H₁₇) | O(n-C₈H₁₇) |

| | | | | | |
|---|---|---|---|---|---|
| Dashed line indicates a chemical bond. | | | | | |

| Table 90 | | | | | |
|---|---|---|---|---|---|
| 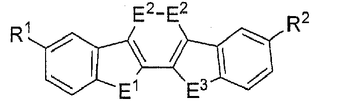 | | | | | |

| Compound No. | E¹ | E² | E³ | R¹ | R² |
|---|---|---|---|---|---|
| 860 | S | S | S | O(n-C₈H₁₇) | O(n-C₁₂H₂₅) |
| 861 | S | S | S | 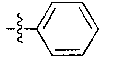 | 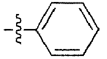 |
| 862 | S | S | S | 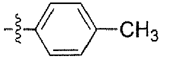 | 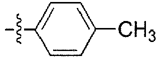 |
| 863 | S | S | S | 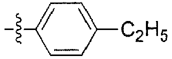 | 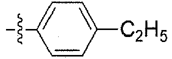 |
| 864 | S | S | S | 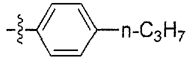 | 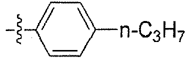 |
| 865 | S | S | S | 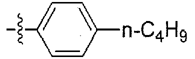 | 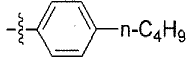 |
| 867 | S | S | S | 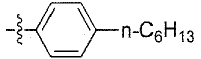 | 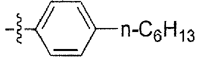 |
| 869 | S | S | S | 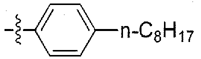 | 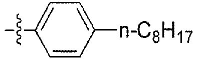 |
| 870 | S | S | S | 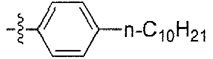 | 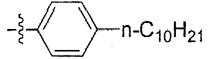 |
| 872 | S | S | S | 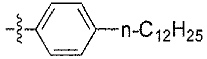 | 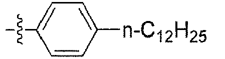 |
| 873 | S | S | S | 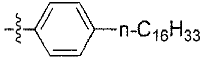 | 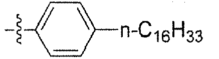 |
| 874 | S | S | S | 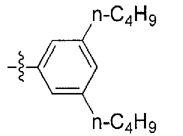 | 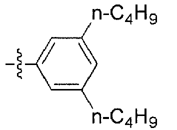 |

| | | | | | |
|---|---|---|---|---|---|
| Dashed line indicates a chemical bond. | | | | | |

| Table 91 | | | | | |
|---|---|---|---|---|---|
| 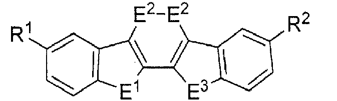 | | | | | |

| Compound No. | E¹ | E² | E³ | R¹ | R² |
|---|---|---|---|---|---|
| 876 | S | S | S | 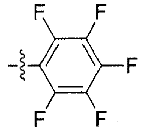 | 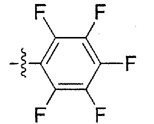 |
| 877 | S | S | S | 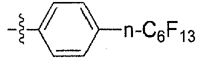 | 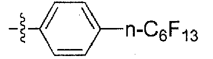 |
| 878 | S | S | S | 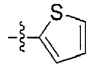 | 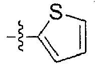 |
| 879 | S | S | S | 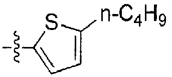 | 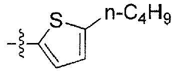 |
| 880 | S | S | S | 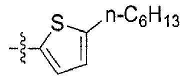 | 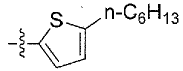 |
| 882 | S | S | S | 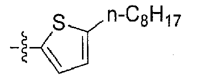 | 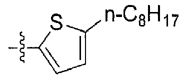 |
| 883 | S | S | S | 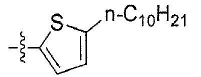 | 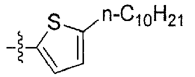 |
| 885 | S | S | S | 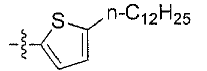 | 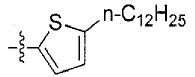 |
| 886 | S | S | S | 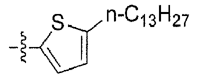 | 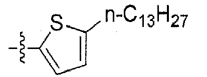 |
| 887 | S | S | S | 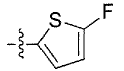 | 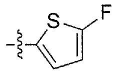 |
| 888 | S | S | S | 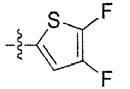 | 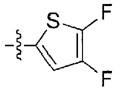 |
| 889 | S | S | S | 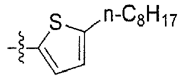 | 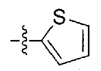 |
| 890 | S | Se | S | 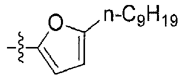 | 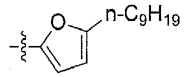 |

| | | | | | |
|---|---|---|---|---|---|
| Dashed line indicates a chemical bond. | | | | | |

| Table 92 | | | | | |
|---|---|---|---|---|---|
| 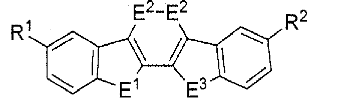 | | | | | |

| Compound No. | E¹ | E² | E³ | R¹ | R² |
|---|---|---|---|---|---|
| 891 | S | S | S | 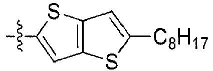 | 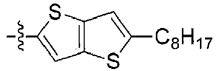 |
| 897 | S | S | S | 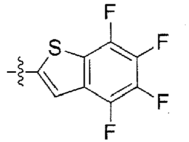 | 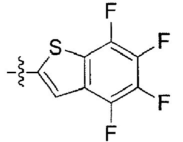 |
| 900 | S | S | S | 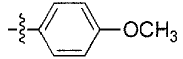 | 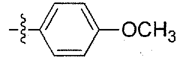 |
| 901 | S | S | S | 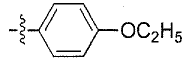 | 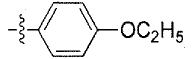 |
| 902 | S | S | S | 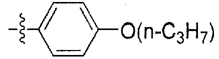 | 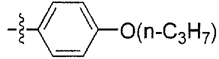 |
| 903 | S | S | S | 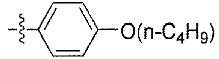 | 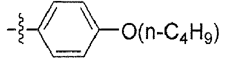 |
| 905 | S | S | S | 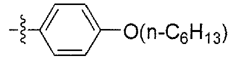 | 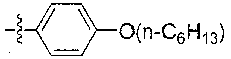 |

| | | | | | |
|---|---|---|---|---|---|
| Dashed line indicates a chemical bond. | | | | | |

| Table 93 | | | | | |
|---|---|---|---|---|---|
| 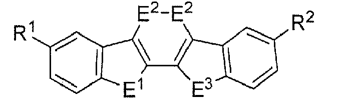 | | | | | |

| Compound No. | E¹ | E² | E³ | R¹ | R² |
|---|---|---|---|---|---|
| 907 | S | S | S | 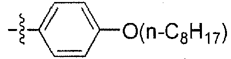 | 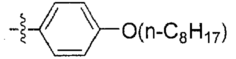 |
| 908 | S | S | S | 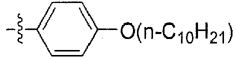 | 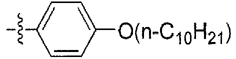 |
| 910 | S | S | S | 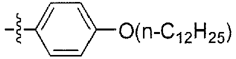 | 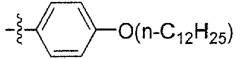 |
| 911 | S | S | S | 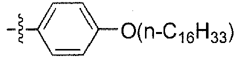 | 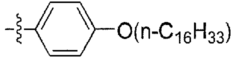 |
| 912 | S | S | S | 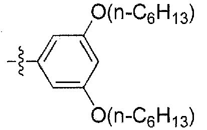 | 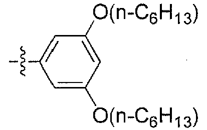 |
| 914 | S | S | S | 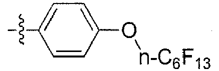 | 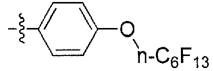 |
| 915 | S | S | S | 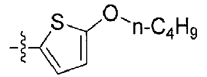 | 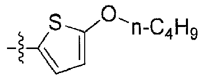 |
| 916 | S | S | S | 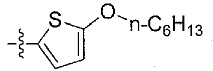 | 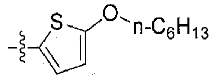 |
| 918 | S | S | S | 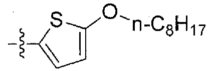 | 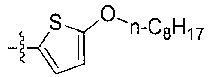 |
| 919 | S | S | S | 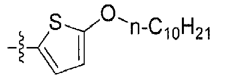 | 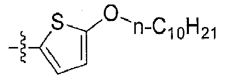 |

| | | | | | |
|---|---|---|---|---|---|
| Dashed line indicates a chemical bond. | | | | | |

| Table 94 | | | | | |
|---|---|---|---|---|---|
| 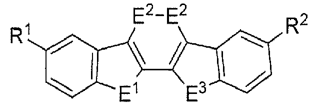 | | | | | |

| Compound No. | E¹ | E² | E³ | R¹ | R² |
|---|---|---|---|---|---|
| 921 | S | S | S | 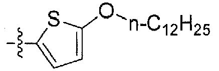 | 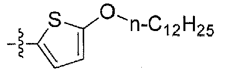 |
| 922 | S | S | S | 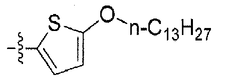 | 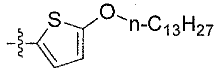 |
| 923 | S | Se | S | 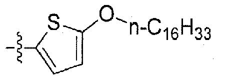 | 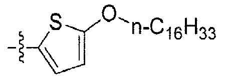 |
| 924 | Se | S | Se | 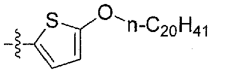 | 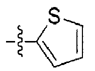 |
| 925 | S | S | S | 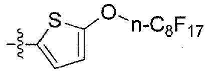 | 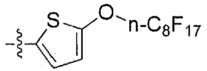 |
| 927 | S | Se | S | 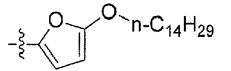 | 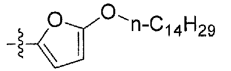 |
| 928 | S | S | S | 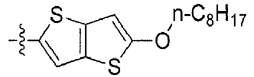 | 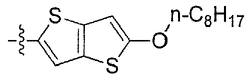 |

| | | | | | |
|---|---|---|---|---|---|
| Dashed line indicates a chemical bond. | | | | | |

| Table 95 | | | | | |
|---|---|---|---|---|---|
| 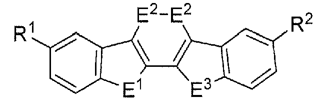 | | | | | |

| Compound No. | E¹ | E² | E³ | R¹ | R² |
|---|---|---|---|---|---|
| 936 | S | S | S | 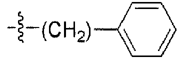 | 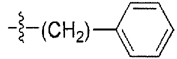 |
| 937 | S | S | S | 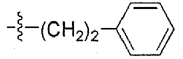 | 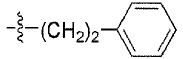 |
| 938 | S | S | S | 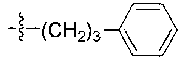 | 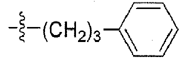 |
| 939 | S | S | S | 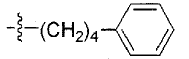 | 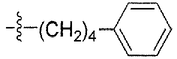 |
| 940 | S | S | S | 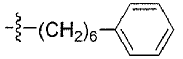 | 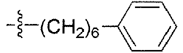 |
| 941 | S | S | S | 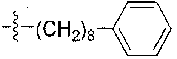 | 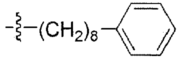 |
| 942 | S | S | S | 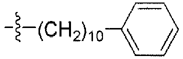 | 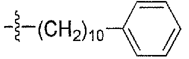 |
| 943 | S | S | S | 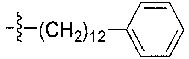 | 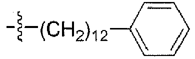 |
| 944 | S | S | S | 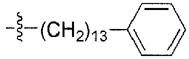 | 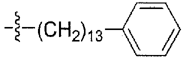 |
| 945 | S | S | S | 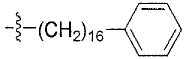 | 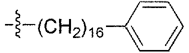 |
| 946 | S | S | S | 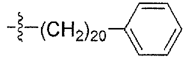 | 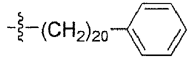 |
| 947 | S | S | S | 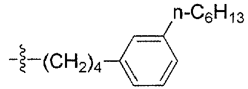 | 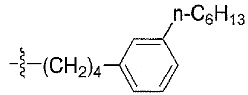 |
| 948 | S | S | S | 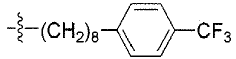 | 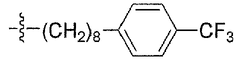 |
| 950 | Se | S | Se | 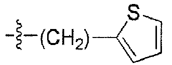 | 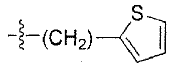 |
| 951 | S | S | S | 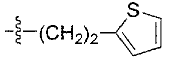 | 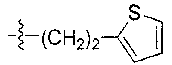 |

| | | | | | |
|---|---|---|---|---|---|
| Dashed line indicates a chemical bond. | | | | | |

| Table 96-1 | | | | | |
|---|---|---|---|---|---|
| 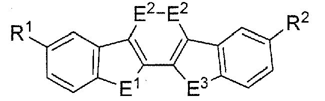 | | | | | |

| Compound No. | E¹ | E² | E³ | R¹ | R² |
|---|---|---|---|---|---|
| 952 | S | S | S | 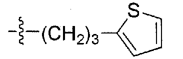 | 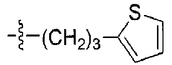 |
| 953 | S | Se | S | 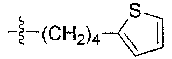 | 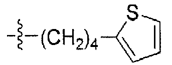 |
| 954 | S | S | S | 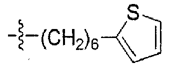 | 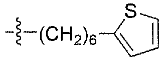 |
| 955 | S | S | S | 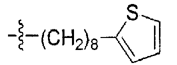 | 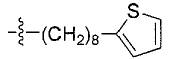 |
| 956 | S | S | S | 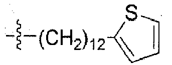 | 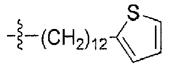 |
| 957 | S | S | S | 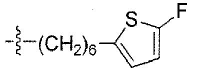 | 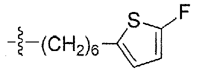 |
| 958 | S | S | S | 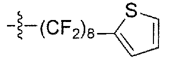 | 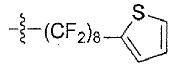 |
| 959 | S | S | S | 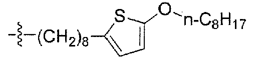 | 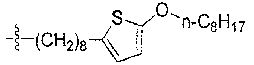 |
| 960 | S | S | S | 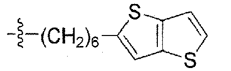 | 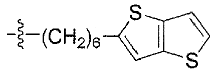 |

| | | | | | |
|---|---|---|---|---|---|
| Dashed line indicates a chemical bond. | | | | | |

| Table 96-2 | | | | | |
|---|---|---|---|---|---|
| 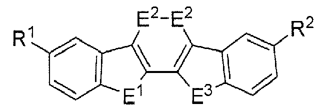 | | | | | |

| Compound No. | E¹ | E² | E³ | R¹ | R² |
|---|---|---|---|---|---|
| 961 | S | S | S | 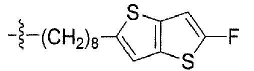 | 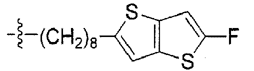 |
| 962 | S | S | S | 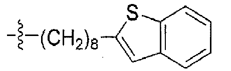 | 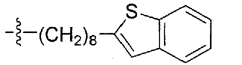 |
| 963 | Se | Se | Se | 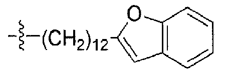 | 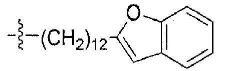 |
| 964 | S | S | S | -Si(CH₃)₃ | -Si(CH₃)₃ |
| 965 | S | S | S | -Si(C₂H₅)₃ | -Si(C₂H₅)₃ |
| 966 | S | S | S | -Si(i-C₃H₇)₃ | -Si(i-C₃H₇)₃ |
| 967 | S | S | S | -Si(CH₃)₂(t-C₄H₉) | -Si(CH₃)₂(t-C₄H₉) |
| 968 | S | S | S | -Si(CH₃)₂(n-C₆H₁₃) | -Si(CH₃)₂(n-C₆H₁₃) |
| 969 | S | S | S | -Si(CH₃)₂(n-C₁₂H₂₅) | -Si(CH₃)₂(n-C₁₂H₂₅) |

| | | | | | |
|---|---|---|---|---|---|
| Dashed line indicates a chemical bond. | | | | | |

| Table 97 | | | | | |
|---|---|---|---|---|---|
| 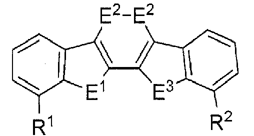 | | | | | |

| Compound No. | E¹ | E² | E³ | R¹ | R² |
|---|---|---|---|---|---|
| 970 | S | S | S | n-C₄H₉ | n-C₄H₉ |
| 971 | S | S | S | n-C₅H₁₁ | n-C₅H₁₁ |
| 972 | S | S | S | 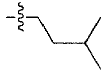 | 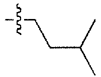 |
| 973 | S | S | S | n-C₆H₁₃ | n-C₆H₁₃ |
| 974 | S | S | S | 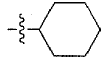 | 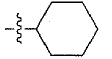 |
| 975 | S | S | S | 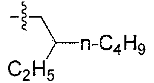 | 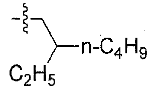 |
| 976 | S | S | S | n-C₈H₁₇ | n-C₈H₁₇ |
| 977 | S | S | S | 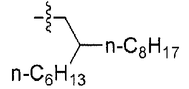 | 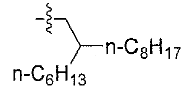 |
| 978 | S | S | S | n-C₁₂H₂₅ | n-C₁₂H₂₅ |
| 979 | S | S | S | n-C₁₃H₂₇ | n-C₁₃H₂₇ |
| 980 | S | S | S | n-C₁₆H₃₃ | n-C₁₆H₃₃ |
| 981 | S | S | S | n-C₁₈H₃₇ | n-C₁₈H₃₇ |
| 982 | S | S | S | n-C₂₀H₄₁ | n-C₂₀H₄₁ |
| 983 | S | S | S | n-C₂₅H₅₁ | n-C₂₅H₅₁ |
| 984 | S | S | S | n-C₃₀H₆₁ | n-C₃₀H₆₁ |
| 985 | S | S | S | n-C₆F₁₃ | n-C₆F₁₃ |
| 986 | S | Se | S | n-C₆H₁₃ | n-C₆H₁₃ |
| 987 | Se | S | Se | n-C₈H₁₇ | n-C₈H₁₇ |
| 988 | S | S | S | n-C₆H₁₃ | n-C₁₂H₂₅ |
| 990 | S | S | S | O(n-C₆H₁₃) | O(n-C₆H₁₃) |
| 993 | S | S | S | O(n-C₈H₁₇) | O(n-C₈H₁₇) |
| 995 | S | S | S | O(n-C₁₂H₂₅) | O(n-C₁₂H₂₅) |

| | | | | | |
|---|---|---|---|---|---|
| Dashed line indicates a chemical bond. | | | | | |

| Table 98 | | | | | |
|---|---|---|---|---|---|
| 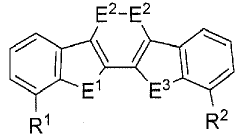 | | | | | |

| Compound No. | E¹ | E² | E³ | R¹ | R² |
|---|---|---|---|---|---|
| 996 | S | S | S | O(n-C₈F₁₇) | O(n-C₈F₁₇) |
| 997 | S | Se | S | O(n-C₆H₁₃) | O(n-C₆H₁₃) |
| 998 | S | S | S | O(n-C₈H₁₇) | O(n-C₁₂H₂₅) |
| 999 | S | S | S | 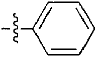 | 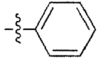 |
| 1001 | S | S | S | 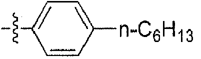 | 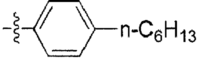 |
| 1003 | S | S | S | 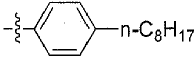 | 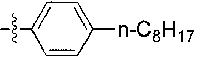 |
| 1004 | S | S | S | 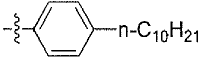 | 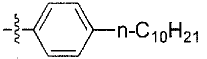 |
| 1006 | S | S | S | 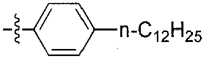 | 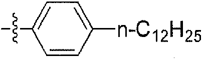 |
| 1007 | S | S | S | 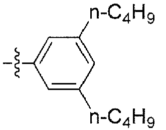 | 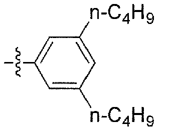 |
| 1009 | S | S | S | 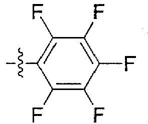 | 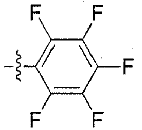 |
| 1010 | S | S | S | 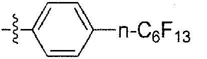 | 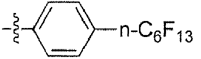 |
| 1011 | S | S | S | 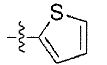 | 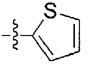 |

| | | | | | |
|---|---|---|---|---|---|
| Dashed line indicates a chemical bond. | | | | | |

| Table 99 | | | | | |
|---|---|---|---|---|---|
| 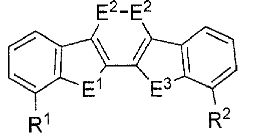 | | | | | |

| Compound No. | E¹ | E² | E³ | R¹ | R² |
|---|---|---|---|---|---|
| 1012 | S | S | S | 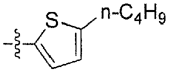 | 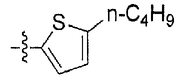 |
| 1013 | S | S | S | 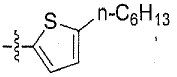 | 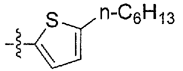 |
| 1015 | S | S | S | 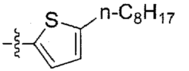 | 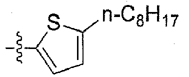 |
| 1016 | S | S | S | 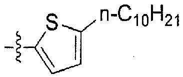 | 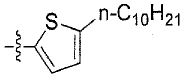 |
| 1018 | S | S | S | 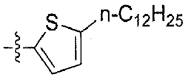 | 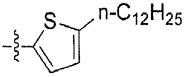 |
| 1019 | S | S | S | 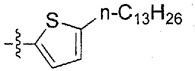 | 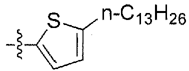 |
| 1020 | S | S | S | 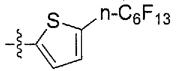 | 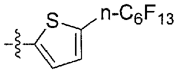 |
| 1021 | S | S | S | 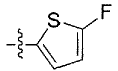 | 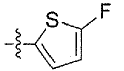 |
| 1022 | S | S | S | 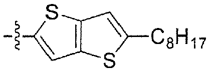 | 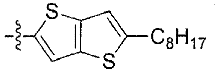 |
| 1026 | S | S | S | 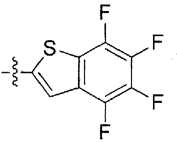 | 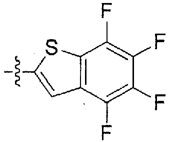 |

| | | | | | |
|---|---|---|---|---|---|
| Dashed line indicates a chemical bond. | | | | | |

| Table 100 | | | | | |
|---|---|---|---|---|---|
| 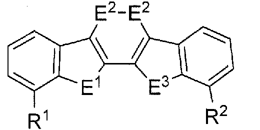 | | | | | |

| Compound No. | E¹ | E² | E³ | R¹ | R² |
|---|---|---|---|---|---|
| 1029 | S | S | S | 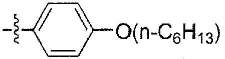 | 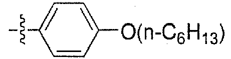 |
| 1031 | S | S | S | 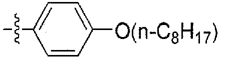 | 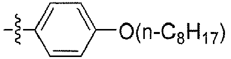 |
| 1033 | S | S | S | 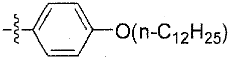 | 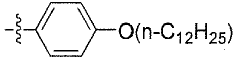 |
| 1034 | S | S | S | 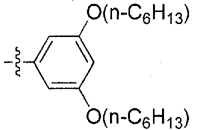 | 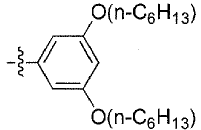 |
| 1036 | S | S | S | 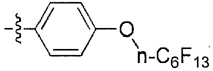 | 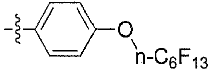 |
| 1037 | S | S | S | 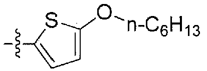 | 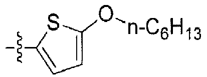 |
| 1039 | S | S | S | 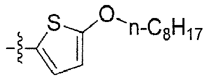 | 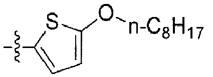 |
| 1040 | S | S | S | 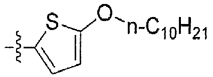 | 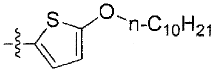 |

| | | | | | |
|---|---|---|---|---|---|
| Dashed line indicates a chemical bond. | | | | | |

| Table 101 | | | | | |
|---|---|---|---|---|---|
| 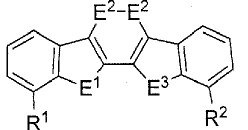 | | | | | |

| Compound No. | E¹ | E² | E³ | R¹ | R² |
|---|---|---|---|---|---|
| 1042 | S | S | S | 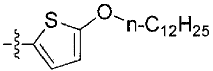 | 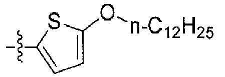 |
| 1043 | S | S | S | 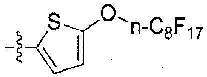 | 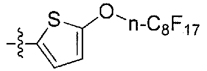 |
| 1044 | S | Se | S | 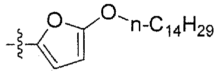 | 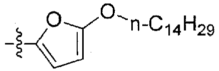 |
| 1045 | S | S | S | 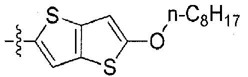 | 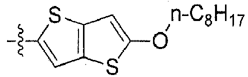 |
| 1049 | S | S | S | 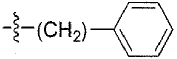 | 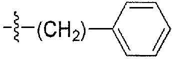 |
| 1050 | S | S | S | 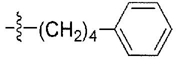 | 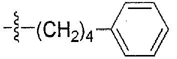 |
| 1051 | S | S | S | 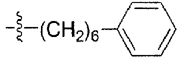 | 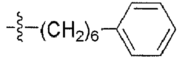 |
| 1052 | S | S | S | 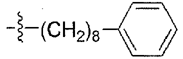 | 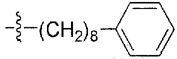 |
| 1053 | S | S | S | 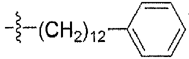 | 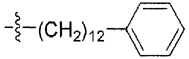 |
| 1054 | S | S | S | 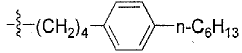 | 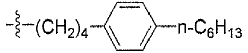 |
| 1055 | S | S | S | 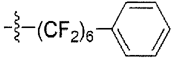 | 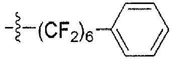 |

| | | | | | |
|---|---|---|---|---|---|
| Dashed line indicates a chemical bond. | | | | | |

| Table 102 | | | | | |
|---|---|---|---|---|---|
| 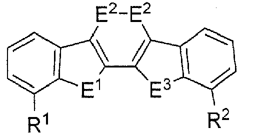 | | | | | |

| Compound No. | E¹ | E² | E³ | R¹ | R² |
|---|---|---|---|---|---|
| 1056 | Se | S | Se | 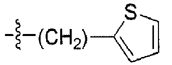 | 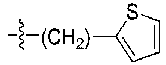 |
| 1057 | S | S | S | 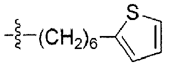 | 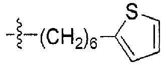 |
| 1058 | S | S | S | 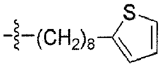 | 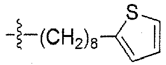 |
| 1059 | S | S | S | 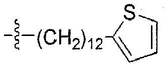 | 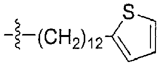 |
| 1060 | S | S | S | 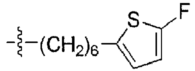 | 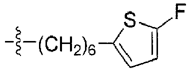 |
| 1061 | S | S | S | 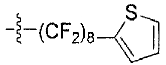 | 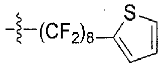 |
| 1062 | S | S | S | 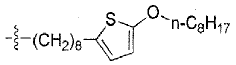 | 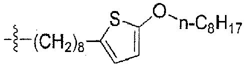 |
| 1063 | S | S | S | 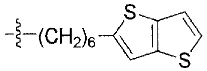 | 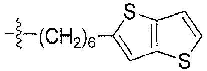 |
| 1064 | S | S | S | 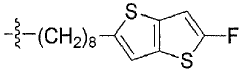 | 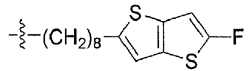 |
| 1065 | S | S | S | 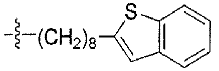 | 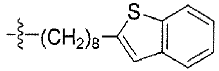 |
| 1066 | S | S | S | -Si(CH₃)₃ | -Si(CH₃)₃ |

| | | | | | |
|---|---|---|---|---|---|
| Dashed line indicates a chemical bond. | | | | | |

| Table 103 | | | | | |
|---|---|---|---|---|---|
| 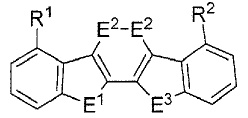 | | | | | |

| Compound No. | E¹ | E² | E³ | R¹ | R² |
|---|---|---|---|---|---|
| 1067 | S | S | S | n-C₆H₁₃ | n-C₆H₁₃ |
| 1068 | S | S | S | n-C₁₂H₂₅ | n-C₁₂H₂₅ |
| 1069 | S | S | S | n-C₁₃H₂₇ | n-C₁₃H₂₇ |
| 1070 | S | S | S | n-C₆F₁₃ | n-C₆F₁₃ |
| 1071 | S | S | S | O(n-C₆H₁₃) | O(n-C₆H₁₃) |
| 1072 | S | S | S | 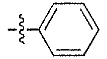 | 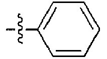 |
| 1073 | S | S | S | 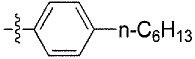 | 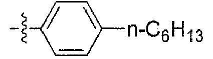 |
| 1074 | S | S | S | 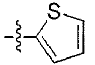 | 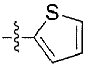 |
| 1075 | S | S | S | 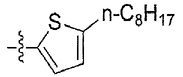 | 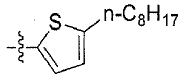 |
| 1079 | S | S | S | 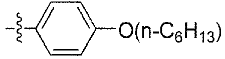 | 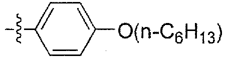 |
| 1083 | S | S | S | 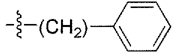 | 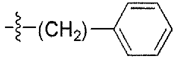 |
| 1084 | S | S | S | 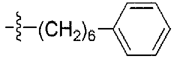 | 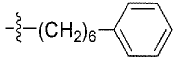 |

| | | | | | |
|---|---|---|---|---|---|
| Dashed line indicates a chemical bond. | | | | | |

| Table 104 | | | | | |
|---|---|---|---|---|---|
| 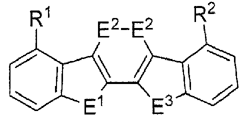 | | | | | |

| Compound No. | E¹ | E² | E³ | R¹ | R² |
|---|---|---|---|---|---|
| 1085 | Se | S | Se | 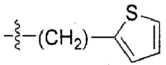 | 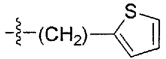 |
| 1086 | S | S | S | 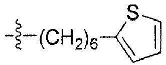 | 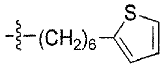 |
| 1087 | S | S | S | 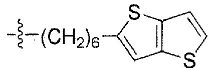 | 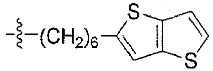 |
| 1088 | S | S | S | 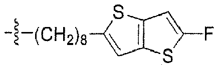 | 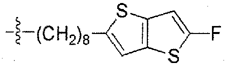 |
| 1089 | S | S | S | 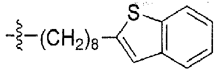 | 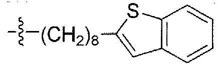 |
| 1090 | S | S | S | -Si(CH₃)₃ | -Si(CH₃)₃ |

| | | | | | |
|---|---|---|---|---|---|
| Dashed line indicates a chemical bond. | | | | | |

| Table 105 | | | | | |
|---|---|---|---|---|---|
| 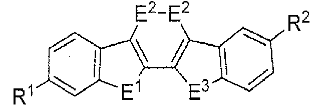 | | | | | |

| Compound No. | E¹ | E² | E³ | R¹ | R² |
|---|---|---|---|---|---|
| 1091 | S | S | S | n-C₆H₁₃ | n-C₆H₁₃ |
| 1092 | S | S | S | O(n-C₆H₁₃) | O(n-C₆H₁₃) |
| 1093 | S | S | S | 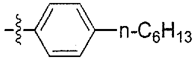 | 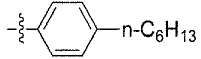 |
| 1094 | S | S | S | 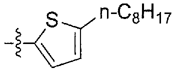 | 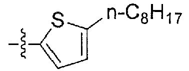 |

| | | | | | |
|---|---|---|---|---|---|
| Dashed line indicates a chemical bond. | | | | | |

| Table 106 | | | | | |
|---|---|---|---|---|---|
| 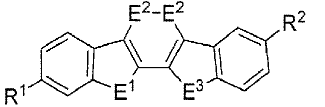 | | | | | |

| Compound No. | E¹ | E² | E³ | R¹ | R² |
|---|---|---|---|---|---|
| 1100 | S | S | S | 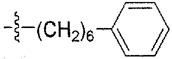 | 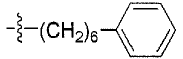 |
| 1101 | S | S | S | 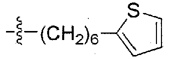 | 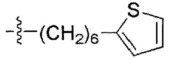 |
| 1102 | S | S | S | 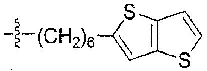 | 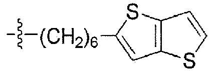 |
| 1103 | S | S | S | 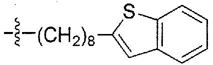 | 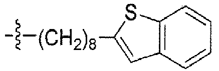 |
| 1104 | S | S | S | -Si(CH₃)₃ | -Si(CH₃)₃ |

| | | | | | |
|---|---|---|---|---|---|
| Dashed line indicates a chemical bond. | | | | | |

| Table 107 | | | | | |
|---|---|---|---|---|---|
| 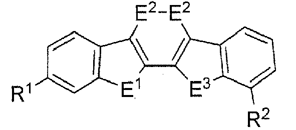 | | | | | |

| Compound No. | E¹ | E² | E³ | R¹ | R² |
|---|---|---|---|---|---|
| 1105 | S | S | S | n-C₆H₁₃ | n-C₆H₁₃ |
| 1106 | S | S | S | O(n-C₆H₁₃) | O(n-C₆H₁₃) |
| 1107 | S | S | S | 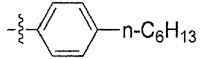 | 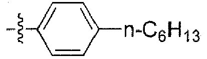 |
| 1108 | S | S | S | 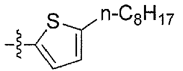 | 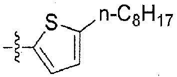 |

| | | | | | |
|---|---|---|---|---|---|
| Dashed line indicates a chemical bond. | | | | | |

| Table 108 | | | | | |
|---|---|---|---|---|---|
| 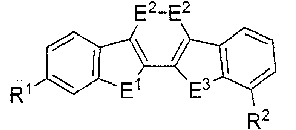 | | | | | |

| Compound No. | E¹ | E² | E³ | R¹ | R² |
|---|---|---|---|---|---|
| 1115 | S | S | S | 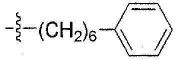 | 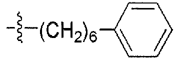 |
| 1116 | S | S | S | 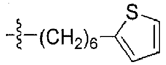 | 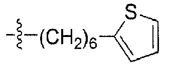 |
| 1117 | S | S | S | 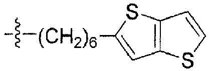 | 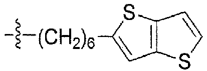 |
| 1118 | S | S | S | 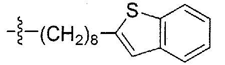 | 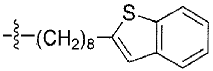 |
| 1119 | S | S | S | -Si(CH₃)₃ | -Si(CH₃)₃ |

| | | | | | |
|---|---|---|---|---|---|
| Dashed line indicates a chemical bond. | | | | | |

| Table 109 | | | | | |
|---|---|---|---|---|---|
| 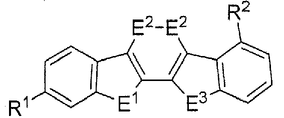 | | | | | |

| Compound No. | E¹ | E² | E³ | R¹ | R² |
|---|---|---|---|---|---|
| 1120 | S | S | S | n-C₆H₁₃ | n-C₆H₁₃ |
| 1121 | S | S | S | 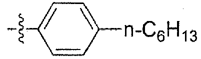 | 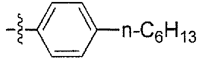 |
| 1124 | S | S | S | 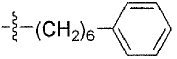 | 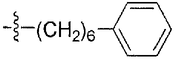 |
| 1125 | S | S | S | 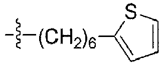 | 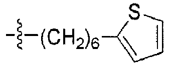 |
| 1126 | S | S | S | -Si(CH₃)₃ | -Si(CH₃)₃ |

| | | | | | |
|---|---|---|---|---|---|
| Dashed line indicates a chemical bond. | | | | | |

| Table 110 | | | | | |
|---|---|---|---|---|---|
| 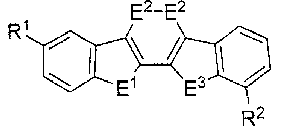 | | | | | |

| Compound No. | E¹ | E² | E³ | R¹ | R² |
|---|---|---|---|---|---|
| 1127 | S | S | S | n-C₆H₁₃ | n-C₆H₁₃ |
| 1128 | S | S | S | 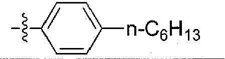 | 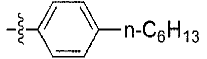 |
| 1131 | S | S | S | 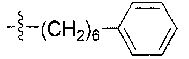 | 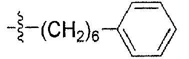 |
| 1132 | S | S | S | 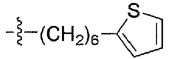 | 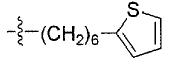 |
| 1133 | S | S | S | -Si(CH₃)₃ | -Si(CH₃)₃ |

| | | | | | |
|---|---|---|---|---|---|
| Dashed line indicates a chemical bond. | | | | | |

| Table 111 | | | | | |
|---|---|---|---|---|---|
| 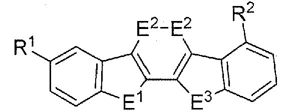 | | | | | |

| Compound No. | E¹ | E² | E³ | R¹ | R² |
|---|---|---|---|---|---|
| 1134 | S | S | S | n-C₆H₁₃ | n-C₆H₁₃ |
| 1135 | S | S | S | 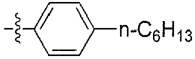 | 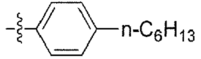 |
| 1138 | S | S | S | 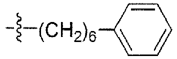 | 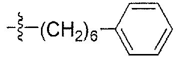 |
| 1139 | S | S | S | 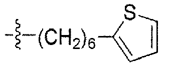 | 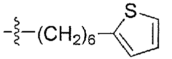 |
| 1140 | S | S | S | -Si(CH₃)₃ | -Si(CH₃)₃ |

| | | | | | |
|---|---|---|---|---|---|
| Dashed line indicates a chemical bond. | | | | | |

One example of a production process of the compound (5-1) used for the present 1st reaction is described for the case that R¹ and R² are the same kinds (hereinafter, optionally described as R) as follows. That is, the compound (5-1) can be produced by the so-called Glaser Reaction, the Eglinton Coupling or the Hay Coupling (preferably, the Hay Coupling utilizing a copper compound such as copper iodide) by using, for example, a compound represented by the formula (6-1): 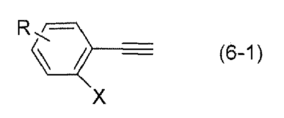 (wherein R represents the same meaning as R¹ and R², and X represents a halogen atom, preferably, a bromine atom).
The Hay Coupling can be carried out, for example, in the presence of N,N,N',N'-tetramethyethylnediamine (TMEDA) and a copper compound such as copper iodide according to the reaction formula below. 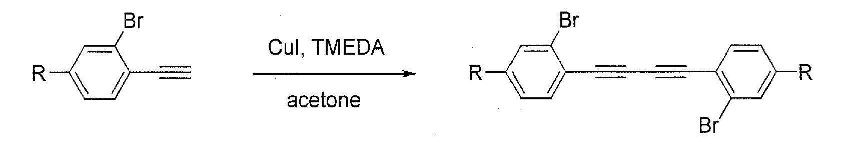 The compound of the formula (6-1) can be produced, for example, by the process comprising steps of:
brominating aniline having a substituent R with N-bromosuccinimide, etc. in the α-position of the amino group,
converting the amino group of thus obtained compound into an iodine group by the Sandmeyer Reaction, and
carrying out an ethynylation of the iodine group in thus obtained iodine-containing compound by the Sonogashira Cross-coupling Reaction, etc.

The specific examples of the compound represented by the formula (6-1) are illustrated in the following tables.

| Table 112 | | |
|---|---|---|
| 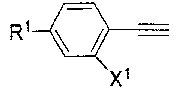 | | |

| Compound No. | X¹ | R¹ |
|---|---|---|
| 1711 | Br | n-C₄H₉ |
| 1712 | Br | s-C₄H₉ |
| 1713 | Br | n-C₅H₁₁ |
| 1714 | Br | 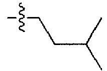 |
| 1715 | Br | n-C₆H₁₃ |
| 1716 | Br | 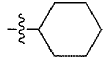 |
| 1717 | Br | 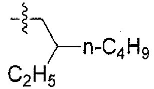 |
| 1718 | Br | n-C₇H₁₅ |
| 1719 | Br | n-C₈H₁₇ |
| 1720 | Br | n-C₉H₁₉ |
| 1721 | Br | n-C₁₀H₂₁ |
| 1722 | Br | 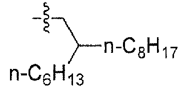 |
| 1723 | Br | n-C₁₁H₂₃ |
| 1724 | Br | n-C₁₂H₂₅ |
| 1725 | Br | n-C₁₃H₂₇ |

| | | |
|---|---|---|
| Dashed line indicates a chemical bond. | | |

| Table 113 | | |
|---|---|---|
| 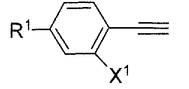 | | |

| Compound No. | X¹ | R¹ |
|---|---|---|
| 1726 | Br | n-C₁₄H₂₉ |
| 1727 | Br | n-C₁₅H₃₁ |
| 1728 | Br | n-C₁₆H₃₃ |
| 1729 | Br | n-C₁₇H₃₅ |
| 1730 | Br | n-C₁₈H₃₇ |
| 1731 | Br | n-C₁₉H₃₉ |
| 1732 | Br | n-C₂₀H₄₁ |
| 1733 | Br | n-C₂₁H₄₃ |
| 1734 | Br | n-C₂₂H₄₅ |
| 1735 | Br | n-C₂₃H₄₇ |
| 1736 | Br | n-C₂₄H₄₉ |
| 1737 | Br | n-C₂₅H₅₁ |
| 1738 | Br | n-C₂₆H₅₃ |
| 1739 | Br | n-C₂₇H₅₅ |
| 1740 | Br | n-C₂₈H₅₇ |
| 1741 | Br | n-C₂₉H₅₉ |
| 1742 | Br | n-C₃₀H₆₁ |
| 1743 | Br | n-C₆F₁₃ |
| 1744 | Br | n-C₈F₁₇ |
| 1745 | Br | n-C₁₂F₂₅ |
| 1746 | Br | H |
| 1747 | I | n-C₆H₁₃ |
| 1748 | I | n-C₈H₁₇ |
| 1749 | C1 | n-C₈H₁₇ |
| 1750 | Br | O(n-C₄H₉) |
| 1751 | Br | O(n-C₅H₁₁) |
| 1753 | Br | O(n-C₆H₁₃) |
| 1756 | Br | O(n-C₇H₁₅) |
| 1757 | Br | O(n-C₈H₁₇) |
| 1758 | Br | O(n-C₉H₁₉) |
| 1759 | Br | O(n-C₁₀H₂₁) |

| | | |
|---|---|---|
| Dashed line indicates a chemical bond. | | |

| Table 114 | | |
|---|---|---|
| 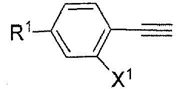 | | |

| Compound No. | X¹ | R¹ |
|---|---|---|
| 1761 | Br | O(n-C₁₁H₂₃) |
| 1762 | Br | O(n-C₁₂H₂₅) |
| 1763 | Br | O(n-C₁₃H₂₇) |
| 1764 | Br | O(n-C₁₄H₂₉) |
| 1765 | Br | O(n-C₁₅H₃₁) |
| 1766 | Br | O(n-C₁₆H₃₃) |
| 1767 | Br | O(n-C₁₇H₃₅) |
| 1768 | Br | O(n-C₁₈H₃₇) |
| 1769 | Br | O(n-C₁₉H₃₉) |
| 1770 | Br | O(n-C₂₀H₄₁) |
| 1771 | Br | O(n-C₂₁H₄₃) |
| 1772 | Br | O(n-C₂₂H₄₅) |
| 1773 | Br | O(n-C₂₃H₄₇) |
| 1774 | Br | O(n-C₂₄H₄₉) |
| 1775 | Br | O(n-C₂₅H₅₁) |
| 1776 | Br | O(n-C₂₆H₅₃) |
| 1777 | Br | O(n-C₂₇H₅₅) |
| 1778 | Br | O(n-C₂₈H₅₇) |
| 1779 | Br | O(n-C₂₉H₅₉) |
| 1780 | Br | O(n-C₃₀H₆₁) |
| 1781 | Br | O(n-C₆F₁₃) |
| 1782 | Br | O(n-C₈F₁₇) |
| 1783 | Br | O(n-C₁₂F₂₅) |
| 1784 | Br | O(n-C₁₆F₃₃) |
| 1785 | I | O(n-C₆H₁₃) |
| 1786 | I | O(n-C₈H₁₇) |
| 1787 | C1 | O(n-C₈H₁₇) |
| 1788 | Br | 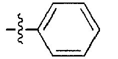 |
| 1789 | Br | 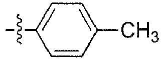 |

| | | |
|---|---|---|
| Dashed line indicates a chemical bond. | | |

| Table 115 | | |
|---|---|---|
| 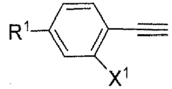 | | |

| Compound No. | X¹ | R¹ |
|---|---|---|
| 1790 | Br | 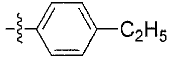 |
| 1791 | Br | 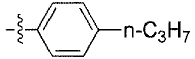 |
| 1792 | Br | 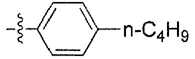 |
| 1794 | Br | 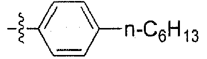 |
| 1796 | Br | 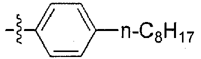 |
| 1797 | Br | 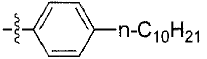 |
| 1799 | Br | 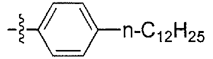 |
| 1800 | Br | 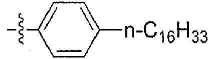 |
| 1801 | Br | 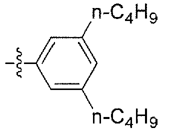 |
| 1803 | Br | 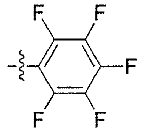 |
| 1804 | Br | 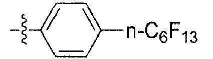 |
| 1805 | Br | 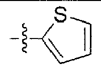 |

| | | |
|---|---|---|
| Dashed line indicates a chemical bond | | |

| Table 116 | | |
|---|---|---|
| 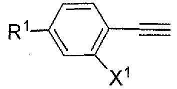 | | |

| Compound No. | X¹ | R¹ |
|---|---|---|
| 1806 | Br | 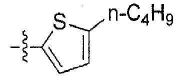 |
| 1807 | Br | 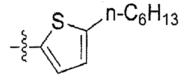 |
| 1809 | Br | 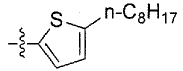 |
| 1810 | Br | 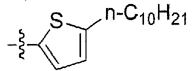 |
| 1812 | Br | 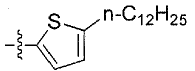 |
| 1813 | Br | 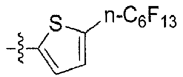 |
| 1814 | Br | 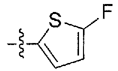 |
| 1815 | Br | 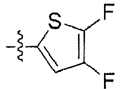 |
| 1816 | Br | 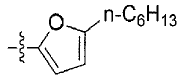 |
| 1817 | Br | 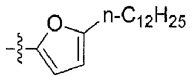 |
| 1818 | Br | 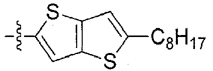 |

| | | |
|---|---|---|
| Dashed line indicates a chemical bond. | | |

| Table 117 | | |
|---|---|---|
| 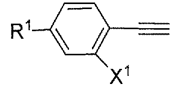 | | |

| Compound No. | X¹ | R¹ |
|---|---|---|
| 1824 | Br | 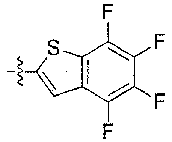 |
| 1827 | Br | 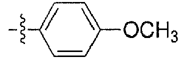 |
| 1828 | Br | 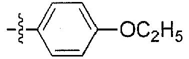 |
| 1829 | Br | 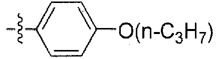 |
| 1830 | Br | 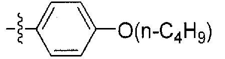 |
| 1832 | Br | 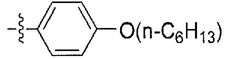 |
| 1834 | Br | 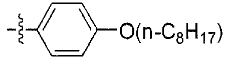 |

| | | |
|---|---|---|
| Dashed line indicates a chemical bond. | | |

| Table 118 | | |
|---|---|---|
| 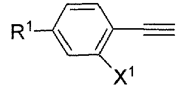 | | |

| Compound No. | X¹ | R¹ |
|---|---|---|
| 1835 | Br | 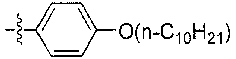 |
| 1837 | Br | 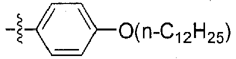 |
| 1838 | Br | 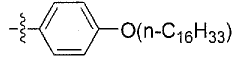 |
| 1839 | Br | 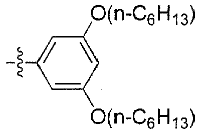 |
| 1841 | Br | 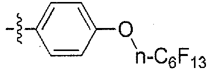 |
| 1842 | Br | 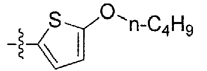 |
| 1843 | Br | 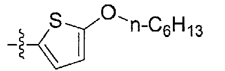 |
| 1845 | Br | 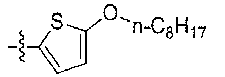 |
| 1846 | Br | 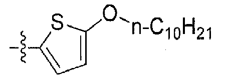 |
| 1848 | Br | 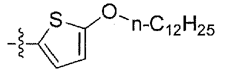 |
| 1849 | Br | 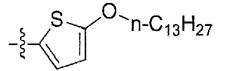 |

| Table 119 | | |
|---|---|---|
| 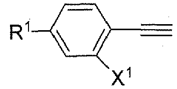 | | |

| Compound No. | X¹ | R¹ |
|---|---|---|
| 1850 | Br | 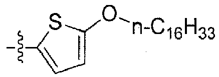 |
| 1851 | Br | 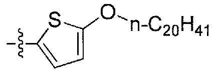 |
| 1852 | Br | 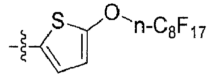 |
| 1853 | Br | 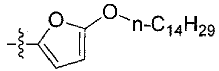 |
| 1854 | Br | 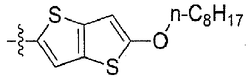 |

| | | |
|---|---|---|
| Dashed line indicates a chemical bond. | | |

| Table 120 | | |
|---|---|---|
| 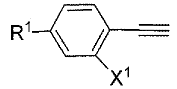 | | |

| Compound No. | X¹ | R¹ |
|---|---|---|
| 1862 | Br | 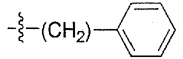 |
| 1863 | Br | 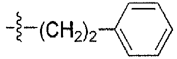 |
| 1864 | Br | 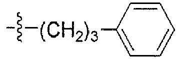 |
| 1865 | Br | 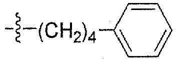 |
| 1866 | Br | 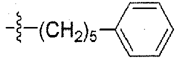 |
| 1867 | Br | 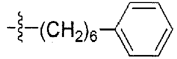 |
| 1868 | Br | 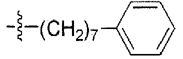 |
| 1869 | Br | 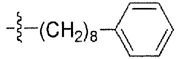 |
| 1870 | Br | 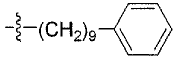 |
| 1871 | Br | 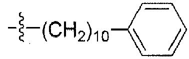 |
| 1872 | Br | 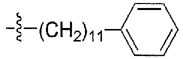 |
| 1873 | Br | 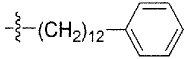 |
| 1874 | Br | 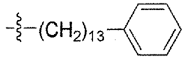 |
| 1875 | Br | 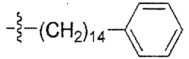 |

| | | |
|---|---|---|
| Dashed line indicates a chemical bond. | | |

| Table 121-1 | | |
|---|---|---|
| 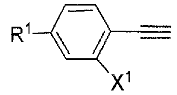 | | |

| Compound No. | X¹ | R¹ |
|---|---|---|
| 1876 | Br | 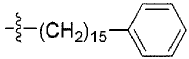 |
| 1877 | Br | 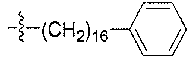 |
| 1878 | Br | 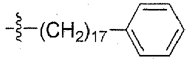 |
| 1879 | Br | 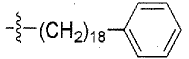 |
| 1880 | Br | 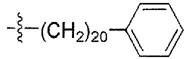 |
| 1881 | Br | 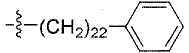 |
| 1882 | Br | 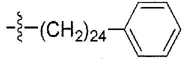 |
| 1883 | Br | 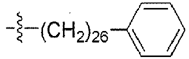 |
| 1884 | Br | 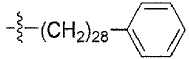 |

| | | |
|---|---|---|
| Dashed line indicates a chemical bond. | | |

| Table 121-2 | | |
|---|---|---|
| 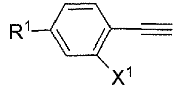 | | |

| Compound No. | X¹ | R¹ |
|---|---|---|
| 1885 | Br | 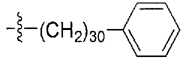 |
| 1886 | Br | 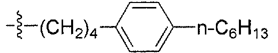 |
| 1887 | Br | 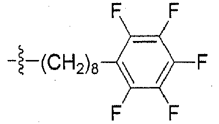 |
| 1888 | Br | 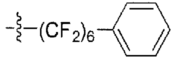 |
| 1889 | Br | 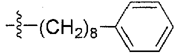 |
| 1890 | Br | 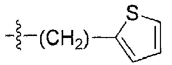 |
| 1891 | Br | 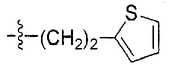 |
| 1892 | Br | 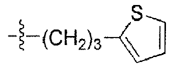 |
| 1893 | Br | 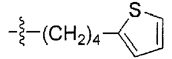 |
| 1894 | Br | 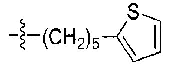 |

| | | |
|---|---|---|
| Dashed line indicates a chemical bond. | | |

| Table 122-1 | | |
|---|---|---|
| 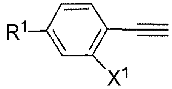 | | |

| Compound No. | X¹ | R¹ |
|---|---|---|
| 1895 | Br | 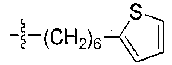 |
| 1896 | Br | 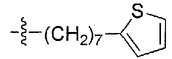 |
| 1897 | Br | 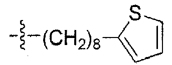 |
| 1898 | Br | 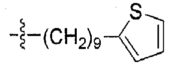 |
| 1899 | Br | 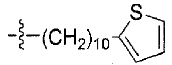 |
| 1900 | Br | 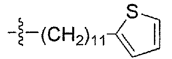 |
| 1901 | Br | 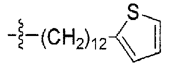 |
| 1902 | Br | 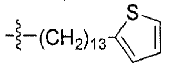 |
| 1903 | Br | 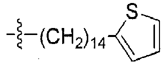 |
| 1904 | Br | 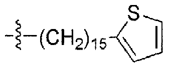 |

| | | |
|---|---|---|
| Dashed line indicates a chemical bond. | | |

| Table 122-2 | | |
|---|---|---|
| 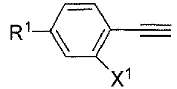 | | |

| Compound No. | X¹ | R¹ |
|---|---|---|
| 1905 | Br | 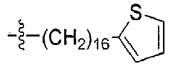 |
| 1906 | Br | 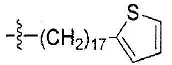 |
| 1907 | Br | 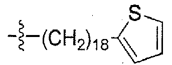 |
| 1908 | Br | 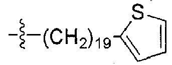 |
| 1909 | Br | 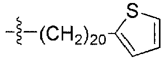 |
| 1910 | Br | 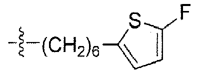 |
| 1911 | Br | 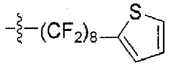 |
| 1912 | Br | 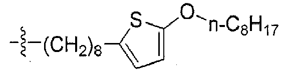 |
| 1913 | Br | 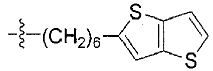 |

| | | |
|---|---|---|
| Dashed line indicates a chemical bond. | | |

| Table 123 | | |
|---|---|---|
| 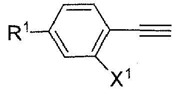 | | |

| Compound No. | X¹ | R¹ |
|---|---|---|
| 1914 | Br | 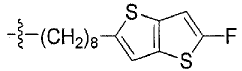 |
| 1915 | Br | 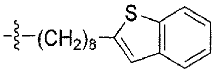 |
| 1916 | Br | 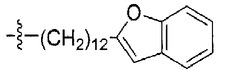 |
| 1917 | Br | 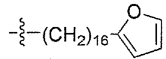 |
| 1918 | Br | -Si(CH₃)₃ |
| 1919 | Br | -Si(C₂H₅)₃ |
| 1920 | Br | -Si(i-C₃H₇)₃ |
| 1921 | Br | -Si(CH₃)₂(t-C₄H₉) |
| 1922 | Br | -Si(CH₃)₂(n-C₆H₁₃) |
| 1923 | Br | -Si(CH₃)₂(n-C₁₂H₂₅) |

| | | |
|---|---|---|
| Dashed line indicates a chemical bond. | | |

| Table 124 | | |
|---|---|---|
| 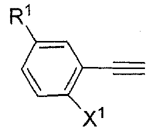 | | |

| Compound No. | X¹ | R¹ |
|---|---|---|
| 1924 | Br | n-C₄H₉ |
| 1925 | Br | s-C₄H₉ |
| 1926 | Br | n-C₅H₁₁ |
| 1927 | Br | 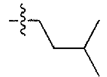 |
| 1928 | Br | n-C₆H₁₃ |
| 1929 | Br | 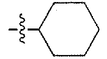 |
| 1930 | Br | 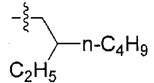 |
| 1931 | Br | n-C₇H₁₅ |
| 1932 | Br | n-C₈H₁₇ |
| 1933 | Br | n-C₉H₁₉ |
| 1934 | Br | n-C₁₀H₂₁ |
| 1924 | Br | n-C₄H₉ |
| 1926 | Br | n-C₅H₁₁ |
| 1928 | Br | n-C₆H₁₃ |
| 1931 | Br | n-C₇H₁₅ |
| 1932 | Br | n-C₈H₁₇ |
| 1933 | Br | n-C₉H₁₉ |
| 1934 | Br | n-C₁₀H₂₁ |

| | | |
|---|---|---|
| Dashed line indicates a chemical bond. | | |

| Table 125 | | |
|---|---|---|
| 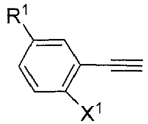 | | |

| Compound No. | X¹ | R¹ |
|---|---|---|
| 1935 | Br | 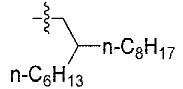 |
| 1936 | Br | n-C₁₁H₂₃ |
| 1937 | Br | n-C₁₂H₂₅ |
| 1938 | Br | n-C₁₃H₂₇ |
| 1939 | Br | n-C₁₄H₂₉ |
| 1940 | Br | n-C₁₅H₃₁ |
| 1941 | Br | n-C₁₆H₃₃ |
| 1942 | Br | n-C₁₇H₃₅ |
| 1943 | Br | n-C₁₈H₃₇ |
| 1944 | Br | n-C₁₉H₃₉ |
| 1945 | Br | n-C₂₀H₄₁ |
| 1946 | Br | n-C₂₁H₄₃ |
| 1947 | Br | n-C₂₂H₄₅ |
| 1948 | Br | n-C₂₃H₄₇ |
| 1949 | Br | n-C₂₄H₄₉ |
| 1950 | Br | n-C₂₅H₅₁ |
| 1951 | Br | n-C₂₆H₅₃ |
| 1952 | Br | n-C₂₇H₅₅ |
| 1953 | Br | n-C₂₈H₅₇ |
| 1954 | Br | n-C₂₉H₅₉ |
| 1955 | Br | n-C₃₀H₆₁ |
| 1956 | Br | n-C₆F₁₃ |
| 1957 | Br | n-C₈F₁₇ |
| 1958 | Br | n-C₁₂F₂₅ |
| 1959 | Br | n-C₁₆F₃₃ |
| 1960 | I | n-C₆H₁₃ |
| 1961 | Cl | n-C₈H₁₇ |
| 1962 | Br | n-C₆H₁₃ |
| 1963 | Br | O(n-C₄H₉) |
| 1964 | Br | O(n-C₅H₁₁) |

| | | |
|---|---|---|
| Dashed line indicates a chemical bond. | | |

| Table 126 | | |
|---|---|---|
| 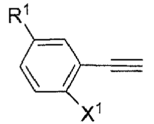 | | |

| Compound No. | X¹ | R¹ |
|---|---|---|
| 1966 | Br | O(n-C₆H₁₃) |
| 1969 | Br | O(n-C₇H₁₅) |
| 1970 | Br | O(n-C₈H₁₇) |
| 1971 | Br | O(n-C₉H₁₉) |
| 1972 | Br | O(n-C₁₀H₂₁) |
| 1974 | Br | O(n-C₁₁H₂₃) |
| 1975 | Br | O(n-C₁₂H₂₅) |
| 1976 | Br | O(n-C₁₃H₂₇) |
| 1977 | Br | O(n-C₁₄H₂₉) |
| 1978 | Br | O(n-C₁₅H₃₁) |
| 1979 | Br | O(n-C₁₆H₃₃) |
| 1980 | Br | O(n-C₁₇H₃₅) |
| 1981 | Br | O(n-C₁₈H₃₇) |
| 1982 | Br | O(n-C₁₉H₃₉) |
| 1983 | Br | O(n-C₂₀H₄₁) |
| 1984 | Br | O(n-C₂₁H₄₃) |
| 1985 | Br | O(n-C₂₂H₄₅) |
| 1986 | Br | O(n-C₂₃H₄₇) |
| 1987 | Br | O(n-C₂₄H₄₉) |
| 1988 | Br | O(n-C₂₅H₅₁) |
| 1989 | Br | O(n-C₂₆H₅₃) |
| 1990 | Br | O(n-C₂₇H₅₅) |
| 1991 | Br | O(n-C₂₈H₅₇) |
| 1992 | Br | O(n-C₂₉H₅₉) |
| 1993 | Br | O(n-C₃₀H₆₁) |
| 1994 | Br | O(n-C₆F₁₃) |
| 1995 | Br | O(n-C₈F₁₇) |
| 1996 | Br | O(n-C₁₂F₂₅) |
| 1997 | Br | O(n-C₁₆F₃₃) |
| 1998 | I | O(n-C₆H₁₃) |
| 1999 | Cl | O(n-C₈H₁₇) |

| | | |
|---|---|---|
| Dashed line indicates a chemical bond. | | |

| Table 127 | | |
|---|---|---|
| 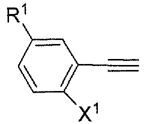 | | |

| Compound No. | X¹ | R¹ |
|---|---|---|
| 2000 | Br | O(n-C₈H₁₇) |
| 2001 | Br | 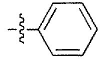 |
| 2002 | Br | 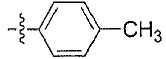 |
| 2003 | Br | 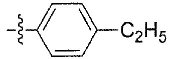 |
| 2004 | Br | 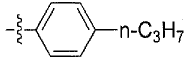 |
| 2005 | Br | 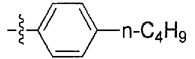 |
| 2007 | Br | 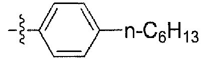 |
| 2009 | Br | 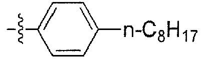 |
| 2010 | Br | 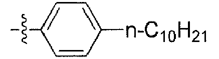 |
| 2012 | Br | 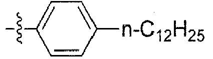 |
| 2013 | Br | 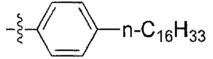 |
| 2014 | Br | 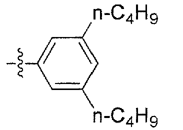 |

| | | |
|---|---|---|
| Dashed line indicates a chemical bond. | | |

| Table 128-1 | | |
|---|---|---|
| 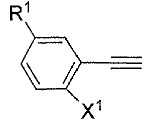 | | |

| Compound No. | X¹ | R¹ |
|---|---|---|
| 2016 | Br | 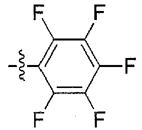 |
| 2017 | Br | 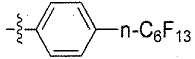 |
| 2018 | Br | 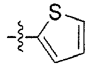 |
| 2019 | Br | 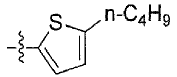 |
| 2020 | Br | 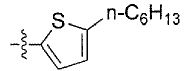 |
| 2022 | Br | 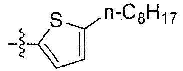 |
| 2023 | Br | 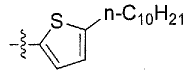 |

| | | |
|---|---|---|
| Dashed line indicates a chemical bond. | | |

| Table 128-2 | | |
|---|---|---|
| 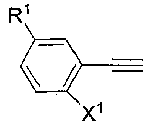 | | |

| Compound No. | X¹ | R¹ |
|---|---|---|
| 2025 | Br | 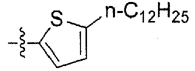 |
| 2026 | Br | 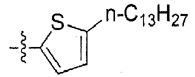 |
| 2027 | Br | 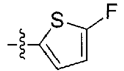 |
| 2028 | Br | 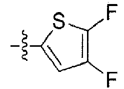 |
| 2029 | Br | 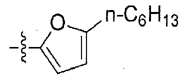 |
| 2030 | Br | 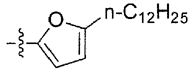 |

| | | |
|---|---|---|
| Dashed line indicates a chemical bond. | | |

| Table 129 | | |
|---|---|---|
| 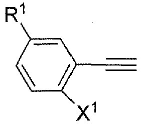 | | |

| Compound No. | X¹ | R¹ |
|---|---|---|
| 2031 | Br | 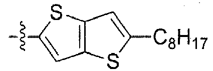 |
| 2037 | Br | 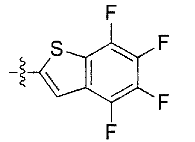 |
| 2040 | Br | 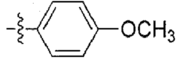 |
| 2041 | Br | 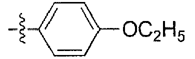 |
| 2042 | Br | 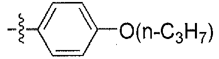 |
| 2043 | Br | 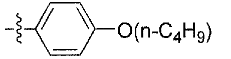 |
| 2045 | Br | 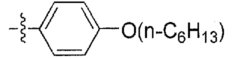 |

| | | |
|---|---|---|
| Dashed line indicates a chemical bond. | | |

| Table 130 | | |
|---|---|---|
| 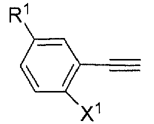 | | |

| Compound No. | X¹ | R¹ |
|---|---|---|
| 2047 | Br | 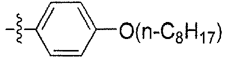 |
| 2048 | Br | 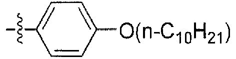 |
| 2050 | Br | 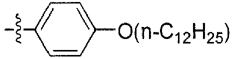 |
| 2051 | Br | 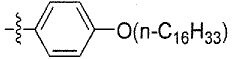 |
| 2052 | Br | 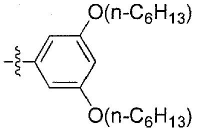 |
| 2054 | Br | 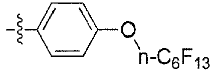 |
| 2055 | Br | 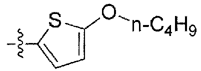 |
| 2056 | Br | 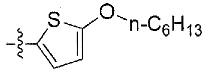 |
| 2058 | Br | 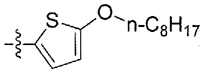 |
| 2059 | Br | 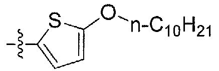 |

| | | |
|---|---|---|
| Dashed line indicates a chemical bond. | | |

| Table 131 | | |
|---|---|---|
| 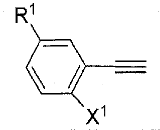 | | |

| Compound No. | X¹ | R¹ |
|---|---|---|
| 2061 | Br | 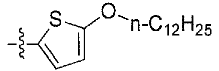 |
| 2062 | Br | 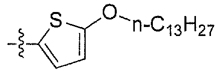 |
| 2063 | Br | 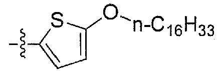 |
| 2064 | Br | 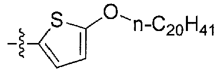 |
| 2065 | Br | 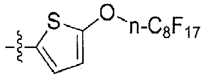 |
| 2066 | Br | 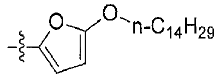 |
| 2067 | Br | 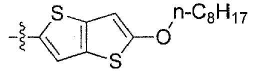 |

| | | |
|---|---|---|
| Dashed line indicates a chemical bond. | | |

| Table 132-1 | | |
|---|---|---|
| 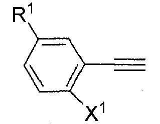 | | |

| Compound No. | X¹ | R¹ |
|---|---|---|
| 2075 | Br | 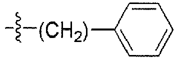 |
| 2076 | Br | 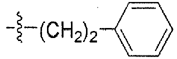 |
| 2077 | Br | 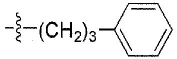 |
| 2078 | Br | 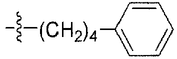 |
| 2079 | Br | 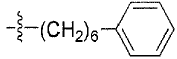 |
| 2080 | Br | 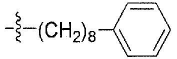 |
| 2081 | Br | 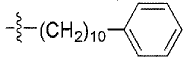 |
| 2082 | Br | 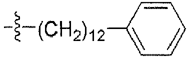 |
| 2083 | Br | 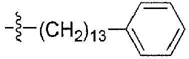 |

| | | |
|---|---|---|
| Dashed line indicates a chemical bond. | | |

| Table 132-2 | | |
|---|---|---|
| 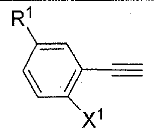 | | |

| Compound No. | X¹ | R¹ |
|---|---|---|
| 2084 | Br | 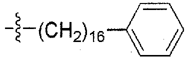 |
| 2085 | Br | 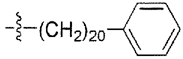 |
| 2086 | Br | 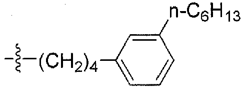 |
| 2087 | Br | 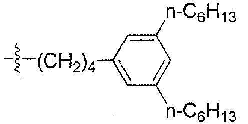 |
| 2088 | Br | 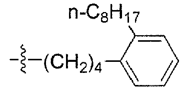 |
| 2089 | Br | 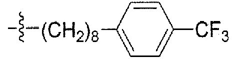 |
| 2090 | Br | 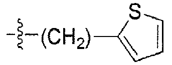 |
| 2091 | Br | 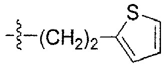 |

| | | |
|---|---|---|
| Dashed line indicates a chemical bond. | | |

| Table 133-1 | | |
|---|---|---|
| 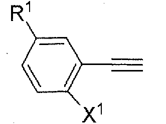 | | |

| Compound No. | X¹ | R¹ |
|---|---|---|
| 2092 | Br | 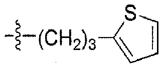 |
| 2093 | Br | 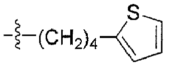 |
| 2094 | Br | 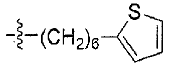 |
| 2095 | Br | 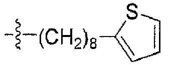 |
| 2096 | Br | 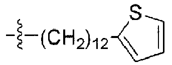 |
| 2097 | Br | 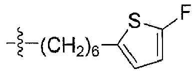 |
| 2098 | Br | 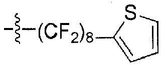 |
| 2099 | Br | 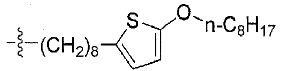 |
| 2100 | Br | 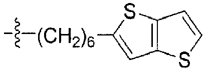 |

| | | |
|---|---|---|
| Dashed line indicates a chemical bond. | | |

| Table 133-2 | | |
|---|---|---|
| 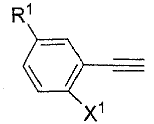 | | |

| Compound No. | X¹ | R¹ |
|---|---|---|
| 2101 | Br | 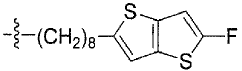 |
| 2102 | Br | 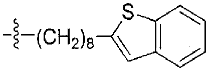 |
| 2103 | Br | 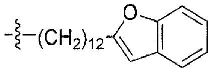 |
| 2104 | Br | -Si(CH₃)₃ |
| 2105 | Br | -Si(C₂H₅)₃ |
| 2106 | Br | -Si(i-C₃H₇)₃ |
| 2107 | Br | -Si(CH₃)₂(t-C₄H₉) |
| 2108 | Br | -Si(CH₃)₂(n-C₆H₁₃) |
| 2109 | Br | -Si(CH₃)₂(n-C₁₂H₂₅) |

| | | |
|---|---|---|
| Dashed line indicates a chemical bond. | | |

| Table 134 | | |
|---|---|---|
| 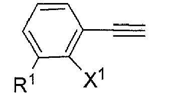 | | |

| Compound No. | X¹ | R¹ |
|---|---|---|
| 2110 | Br | n-C₄H₉ |
| 2111 | Br | n-C₅H₁₁ |
| 2112 | Br | 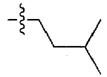 |
| 2113 | Br | n-C₆H₁₃ |
| 2114 | Br | 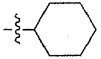 |
| 2115 | Br | 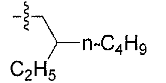 |
| 2116 | Br | n-C₈H₁₇ |
| 2117 | Br | 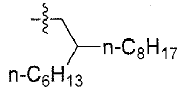 |
| 2118 | Br | n-C₁₂H₂₅ |
| 2119 | Br | n-C₁₃H₂₇ |
| 2120 | Br | n-C₁₆H₃₃ |
| 2121 | Br | n-C₁₈H₃₇ |
| 2122 | Br | n-C₂₀H₄₁ |
| 2123 | Br | n-C₂₅H₅₁ |
| 2124 | Br | n-C₃₀H₆₁ |
| 2125 | Br | n-C₆F₁₃ |
| 2126 | I | n-C₆H₁₃ |
| 2127 | C1 | n-C₈H₁₇ |
| 2128 | Br | O(n-C₄H₉) |
| 2130 | Br | O(n-C₆H₁₃) |
| 2133 | Br | O(n-C₈H₁₇) |
| 2135 | Br | O(n-C₁₂H₂₅) |

| | | |
|---|---|---|
| Dashed line indicates a chemical bond. | | |

| Table 135 | | |
|---|---|---|
| 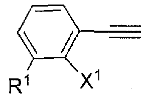 | | |

| Compound No. | X¹ | R¹ |
|---|---|---|
| 2136 | Br | O(n-C₈F₁₇) |
| 2137 | I | O(n-C₆H₁₃) |
| 2138 | Br | O(n-C₈H₁₇) |
| 2139 | Br | 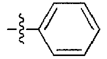 |
| 2141 | Br | 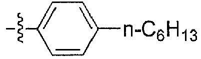 |
| 2143 | Br | 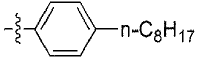 |
| 2144 | Br | 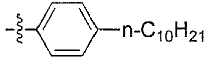 |
| 2146 | Br | 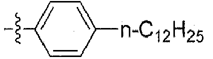 |
| 2147 | Br | 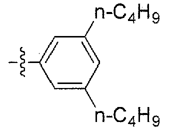 |
| 2149 | Br | 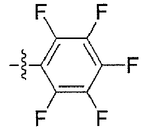 |
| 2150 | Br | 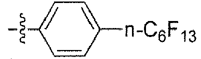 |
| 2151 | Br | 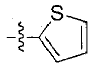 |

| | | |
|---|---|---|
| Dashed line indicates a chemical bond. | | |

| Table 136 | | |
|---|---|---|
| 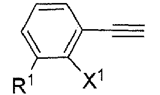 | | |

| Compound No. | X¹ | R¹ |
|---|---|---|
| 2152 | Br | 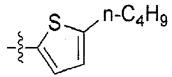 |
| 2153 | Br | 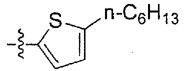 |
| 2155 | Br | 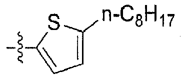 |
| 2156 | Br | 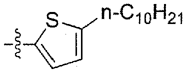 |
| 2158 | Br | 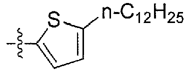 |
| 2159 | Br | 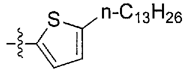 |
| 2160 | Br | 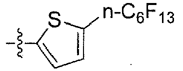 |
| 2161 | Br | 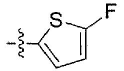 |
| 2162 | Br | 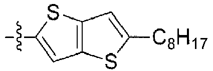 |
| 2166 | Br | 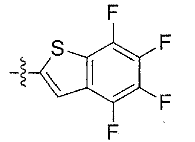 |

| | | |
|---|---|---|
| Dashed line indicates a chemical bond. | | |

| Table 137 | | |
|---|---|---|
| 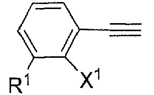 | | |

| Compound No. | X¹ | R¹ |
|---|---|---|
| 2169 | Br | 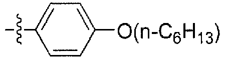 |
| 2171 | Br | 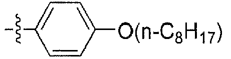 |
| 2173 | Br | 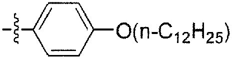 |
| 2174 | Br | 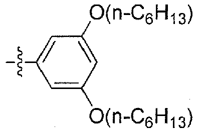 |
| 2176 | Br | 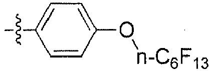 |
| 2177 | Br | 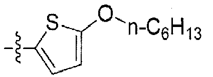 |
| 2179 | Br | 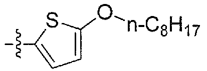 |
| 2180 | Br | 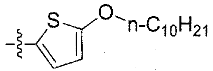 |

| | | |
|---|---|---|
| Dashed line indicates a chemical bond. | | |

| Table 138 | | |
|---|---|---|
| 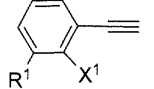 | | |

| Compound No. | X¹ | R¹ |
|---|---|---|
| 2182 | Br | 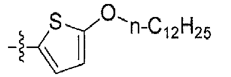 |
| 2183 | Br | 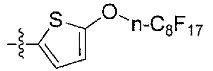 |
| 2184 | Br | 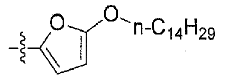 |
| 2185 | Br | 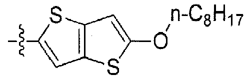 |
| 2189 | Br | 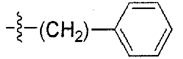 |
| 2190 | Br | 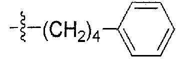 |
| 2191 | Br | 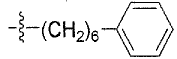 |
| 2192 | Br | 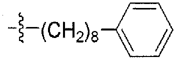 |
| 2193 | Br | 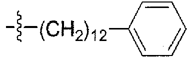 |
| 2194 | Br | 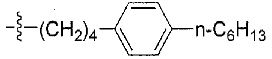 |
| 2195 | Br | 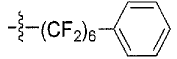 |

| | | |
|---|---|---|
| Dashed line indicates a chemical bond. | | |

| Table 139 | | |
|---|---|---|
| 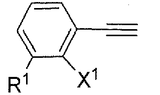 | | |

| Compound No. | X¹ | R¹ |
|---|---|---|
| 2196 | Br | 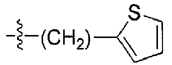 |
| 2197 | Br | 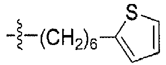 |
| 2198 | Br | 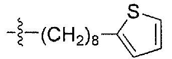 |
| 2199 | Br | 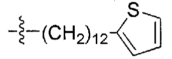 |
| 2200 | Br | 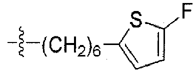 |
| 2201 | Br | 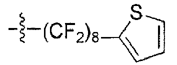 |
| 2202 | Br | 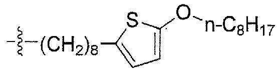 |
| 2203 | Br | 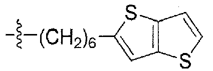 |
| 2204 | Br | 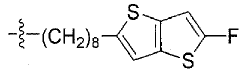 |
| 2205 | Br | 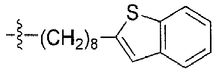 |
| 2206 | Br | -Si(CH₃)₃ |

| | | |
|---|---|---|
| Dashed line indicates a chemical bond. | | |

| Table 140 | | |
|---|---|---|
| 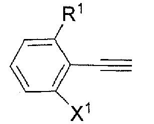 | | |

| Compound No. | X¹ | R¹ |
|---|---|---|
| 2207 | Br | n-C₆H₁₃ |
| 2208 | Br | n-C₁₂H₂₅ |
| 2209 | Br | n-C₁₃H₂₇ |
| 2210 | Br | n-C₆F₁₃ |
| 2211 | Br | O(n-C₆H₁₃) |
| 2212 | Br | 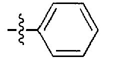 |
| 2213 | Br | 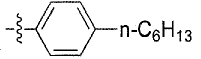 |
| 2214 | Br | 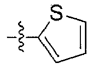 |
| 2215 | Br | 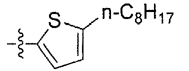 |
| 2219 | Br | 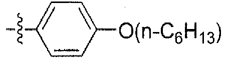 |
| 2223 | Br | 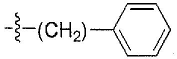 |
| 2224 | Br | 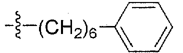 |

| | | |
|---|---|---|
| Dashed line indicates a chemical bond. | | |

| Table 141 | | |
|---|---|---|
| 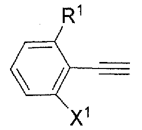 | | |

| Compound No. | X¹ | R¹ |
|---|---|---|
| 2225 | Br | 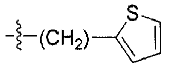 |
| 2226 | Br | 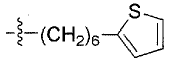 |
| 2227 | Br | 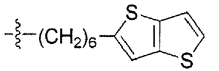 |
| 2228 | Br | 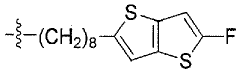 |
| 2229 | Br | 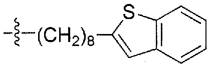 |
| 2230 | Br | -Si(CH₃)₃ |

| | | |
|---|---|---|
| Dashed line indicates a chemical bond. | | |

A process for producing the substituted benzochalcogenoacene compound (1) in the case that R¹ and R² are the same or different kinds, is exemplified by a process according to the description of the non-patent literature 1 (Advanced Materials, 19, 3008-3011 (2007)). That is, the process comprises the sequential steps of:
brominating the benzo[b]thiophene having R¹ or R² with bromine,
working lithiumisopropylamide (LDA) on the brominated product thus obtained, then
carrying out a coupling reaction using copper chloride, and
working butyl lithium (BuLi) and bis(phenylsulfonyl)sulfide ((C₆H₅SO₂)₂S) on the coupling product thus obtained to produce the compound (1), as shown in the following reaction sequence scheme.

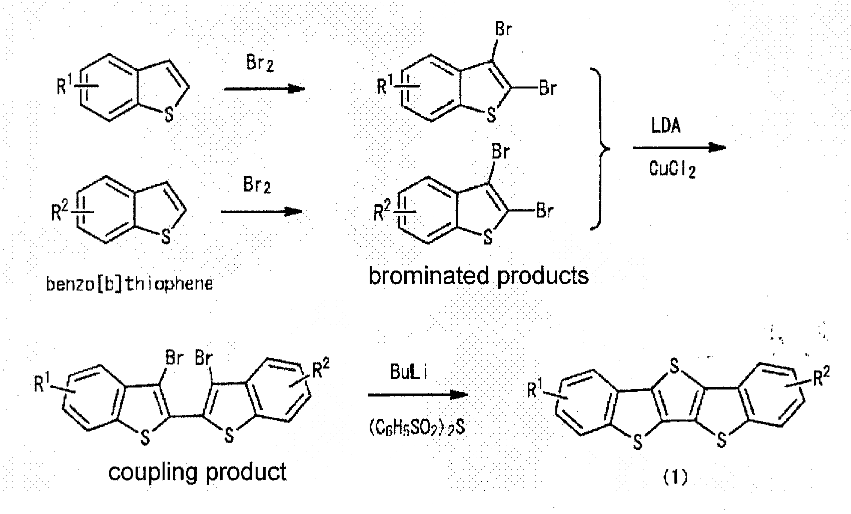

An organic semiconductor device of the present invention will be explained as follows.
A thin film of the present invention comprises the substituted benzochalcogenoacene compound (1). The thin film shows high carrier mobility. Therefore, the thin film is suitable for a material for an organic semiconductor device having the thin film as an active organic semiconductor layer.
In addition, the organic semiconductor device of the present invention comprises the thin film of the present invention. Examples of the organic semiconductor device of the present invention include an organic transistor, an electroluminescence device and a solar cell. The organic transistor of the present invention can be used, for example, in an electronic paper, a flexible display, an IC tag and a sensor.

The formation process of the thin film of the present invention is exemplified by the applying and film-forming process. The applying and film-forming process means the film-forming process which comprises the steps of dissolving the substituted benzochalcogenoacene compound (1) in a solvent and applying the obtained solution composition on a substrate or an insulating layer.
Examples of the coating process include a casting process, a dip coat process, a die coater process, a roll coater process, a bar coater process, an ink jet process, a screen printing process, an offset printing process and a microcontact printing process. These processes can be used alone or in combination of two or more of these processes.

A relevant solvent which is used for the preparation of the above solution composition can be selected properly depending on the kind of the substituted benzochalcogenoacene compound to be applied. Preferable examples of the solvent include an aromatic hydrocarbon solvent such as benzene, toluene, xylene, chlorobenzene and o-dichlorobenzene, a halogenated hydrocarbon solvent such as dichloromethane, chloroform, 1,2-dichloroethane, 1,1',2,2'-tetrachloroethane, tetrachlorocarbon, an ether solvent such as tetrahydrofuran and dioxane, and an aliphatic hydrocarbon solvent such as pentane, hexane, heptane, octane and cyclohexane. Among them, toluene, xylene, o-dichlorobenzene, dichloromethane, chloroform, tetrahydrofuran and hexane are preferable. These solvents can be used also by mixing two or more of them. The concentration of the substituted benzochalcogenoacene compound (1) in the solution composition is 0.01-50 wt%, preferably, 0.01-10 wt%, more preferably, 0.1-5 wt%. Additionally, within the range where carrier mobility is not damaged remarkably, additives such as an antioxidant or a stabilizer can be contained in the solution composition. The solution composition can be obtained by dissolving the substituted benzochalcogenoacene compound (1) in the solvent at temperatures of, for example, 10-200°C, preferably, 20-150°C.

After the solution composition thus obtained is applied to a substrate or an insulating layer to result in the formation of a coated film, a thin film can be formed on the substrate or the insulating layer by eliminating the solvent contained in the coated film. In order to eliminate the solvent, a naturally drying treatment, a heating treatment, a reduced pressure treatment, a draught drying treatment and a combination thereof can be adopted. Among them, the naturally drying treatment or the heating treatment are preferable from the point of easy operation. The operation condition for the treatment is described as still-standing under the atmosphere or heating of the substrate on a hot plate (for example, at 40-250°C, preferably, 50-250°C).

The thin film of the present invention can be formed by the applying and film-forming process by using also a dispersion of the substituted benzochalcogenoacene compound (1) in the solvent, and in this case, the process can be easily carried out by reading the solution composition as the dispersion composition.
Thus, the thin film of the present invention can be formed by a simple method such as the applying and film-forming process as described above.

Another different example of the thin film forming process of the present invention is a thin film forming process under vacuum such as a vacuum deposition process, a sputtering process, a CVD process and a molecular beam epitaxial process.

In the vacuum deposition process, the substituted benzochalcogenoacene compound is heated in a crucible or a metal boat under vacuum, and the evaporated organic semiconductor material is deposited on the substrate or the insulating material. A degree of vacuum when deposition occurs is, generally, 1x10⁻¹ Pa or lower, preferably, 1x10⁻³ Pa or lower. A substrate temperature when deposition occurs is, generally, 0°C -300°C, preferably, 20°C -200°C. A deposition speed is, for example, 0.001nm/sec - 10nm/sec, preferably, 0.01nm/sec - 1nm/sec.

A thickness of the thin film comprising the substituted benzochalcogenoacene compound (1) obtained by the above applying and film-forming process or the above vacuum process is controllable, for example, depending on a device structure of the organic transistor, and the film thickness is preferably 1nm-10µm, more preferably, 5nm-1µm.

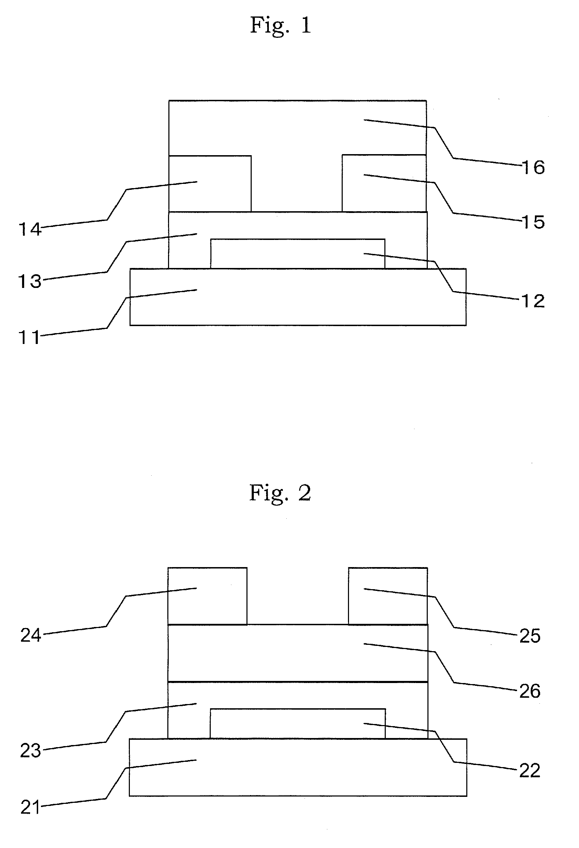

An example of the organic transistor of the present invention is the organic field effect transistor (OFET).
The structure of the organic field effect transistor is, for example, generally provided with a source electrode and a drain electrode close to the organic semiconductor active layer consisting of the thin film of the present invention, and further provided with a gate electrode across an insulator layer (a dielectric layer) close to the organic semiconductor active layer.
Examples of the device structure include the followings:
(1) a structure of a substrate/a gate electrode/an insulating layer/a source electrode-a drain electrode/an organic semiconductor active layer (refer to Fig. 1),
(2) a structure of a substrate/a gate electrode/an insulating layer/an organic semiconductor active layer/a source electrode-a drain electrode (refer to Fig. 2),
(3) a structure of a substrate/an organic semiconductor active layer/a source electrode-a drain electrode/an insulating layer/a gate electrode,
(4) a structure of a substrate/a source electrode (or a drain electrode) /an organic semiconductor active layer + an insulating layer + a gate electrode/a drain electrode (or a source electrode).
In these cases, the source electrode, the drain electrode and the gate electrode may be provided respectively in plural, and the plural of the organic semiconductor active layers may be provided within a same plane or as laminated layers.

The other components of the organic transistor will be explained by illustrating specific examples.
In manufacturing the organic transistor in the present invention, materials constituting the source electrode, the drain electrode and the gate electrode are not limited specifically as far as the materials are electrically conducting materials such as platinum, gold, silver, nickel, chromium, copper, iron, tin, lead antimony, tantalum, indium, palladium, tellurium, rhenium, iridium, aluminum, ruthenium, germanium, molybdenum, molybdenum oxide, tungsten, antimony tin oxide, indium tin oxide (ITO), zinc doped with fluorine, zinc, carbon, graphite, a glassy carbon, a silver paste and carbon paste, lithium, beryllium, sodium, magnesium, potassium, calcium, scandium, titanium, manganese, zirconium, gallium, niobium, sodium, a sodium-potassium alloy, magnesium, lithium, aluminum, a magnesium/ copper mixture, a magnesium/silver mixture, a magnesium/aluminum mixture, a magnesium/indium mixture, an aluminum/aluminum oxide mixture and a lithium/aluminum mixture. Especially, platinum, gold, silver, molybdenum oxide, indium, ITO and carbon are preferable. In addition, known conductive polymers whose conductivity is improved by doping, etc. are also suitably used. Examples of such conductive polymers include a conductive polyaniline, a conductive polypyrrole, a conductive polythiophene and a complex between polyethylenedioxythiophene and polystyrene sulfonic acid. Above all, the conductive materials which have a low electric resistance at the contact face with the semiconductor layer are preferable. These conductive materials may be used alone or in a mixture of two or more kinds. A film thickness of the electrode varies depending on the material, and the thickness is, preferably, 0.1nm-10µm, further preferably, 0.5nm-5µm, and more preferably, 1nm-3µm. In addition, when the gate electrode doubles with the substrate, the film thickness may be larger than the above values.

The source electrode and the drain electrode used in the organic transistor of the present invention may undergo a surface treatment. The surface treatment of the electrode surface contacting with the thin film (the organic semiconductor active layer) of the present invention is preferable, since the surface treatment tends to improve the transistor performances of the organic transistor comprising the thin film. An example of the surface treatment is a modification process of the electrode surfaces mentioned above by dipping the electrodes in an alcohol solution of, for example, a saturated hydrocarbon compound having a thiol group such as 1-octylthiol, 1-perfluorooctylthiol, 1-octadecylthiol and 1-perfluorooctadecylthiol, an aromatic compound having a thiol group such as benzenethiol and perfluorobenzenethiol, and a heteroaromatic compound having a thiol group such as thienylthiol and perfluoro-thienylthiol.

The electrode can be manufactured by various methods using above raw materials. Specifically, a vacuum deposition method, a sputtering method, a coating method, a thermal transfer method, a printing method and a sol-gel method are exemplified. At or after the film-forming, it is preferable to carry out patterning, optionally. The patterning can be carried out by using various methods. Specifically, a photolithography method which combines a patterning and an etching of the photoresist is exemplified. In addition, soft-lithography methods such as an inkjet printing, a screen printing, an offset printing and an anastatic printing are exemplified. These methods can be used for the patterning, alone or in combination of two or more of them.

Various insulating films can be used as the insulating layer. Inorganic oxides, inorganic nitrides and organic compounds can be exemplified as materials for the insulating films.
Examples of inorganic oxides include silicon oxide, aluminum oxide, tantalum oxide, titanium oxide, tin oxide, vanadium oxide, strontium barium titanate, barium titanate zirconate, lead titanate zirconate, lanthanum lead titanate, strontium titanate, barium titanate, magnesium barium fluoride, bismuth titanate, bismuth strontium titanate, bismuth strontium tantalite, bismuth niobate tantalite and yttrium trioxide. Silicon oxide, aluminum oxide, tantalum oxide and titanium oxide are preferable. Examples of the organic compounds include polyimide, polyamide, polyester, polyacrylate, a photo-curable resin obtained by photo-radical polymerization or photo-cationic polymerization, a copolymer comprising an acrylonitrile component, polyvinylphenol, polyvinylalcohol, a novolak resin and cyanoethylpullulan. Polyimide, polyvinylphenol and polyvinylalcohol are preferable. These materials for the insulating layer can be used alone or in combination of two or more of them. A thickness of the insulating layer varies depending on the material, and the thickness is, preferably, 0.1nm-100µm, further preferably, 0.5nm-50µm, and more preferably, 5nm-10µm.

The insulating layer can be formed by various methods. Specifically, a spin coating, a spray coating, a dip coating, a cast, a bar coating, a blade coating, a screen printing, an offset printing, an inkjet and dry process methods such as a vacuum deposition, a molecular beam epitaxial growth method, an ion cluster beam method, an ion plating method, a sputtering method, an atmospheric plasma method and a CVD method are exemplified. In addition, a sol-gel method and a method in which an oxide film is formed on a metal substrate such as an alumite on aluminum or a thermal oxide film of silicon are exemplified.

Examples of the materials of the substrate include glass, paper, quartz, ceramic and a resin sheet. Specified examples of materials for the resin sheet include polyethylene terephthalate (PET), polyethylene naphthalate (PEN), polyether sulfone (PES), polyetherimide, polyether ether ketone, polyphenylene sulfide, polyarylate, polyimide, polycarbonate (PC), cellulose triacetate (TAC) and cellulose acetate propionate (CAP). The thickness of the substrate is, preferably, 1µm-10mm, more preferably, 5µm-5mm.

In the contact parts of the insulating layer and the substrate with the thin film of the present invention (hereinafter, optionally described as the organic semiconductor active layer), a surface treatment may be carried out on the insulating layer and the substrate. By the surface treatment of the insulating layer on which the organic semiconductor active layer is laminated, the transistor performance of the organic transistor can be improved. The surface treatment is exemplified specifically by a hydrophorbic treatment by hexamethyldisilazane, octadecyltrichlorosilane, octyltrichlorosilane and phenetyltrichlorosilane, an acid treatment by hydrochloric acid, sulfuric acid and an aqueous hydrogen peroxide solution, an alkaline treatment by sodium hydroxide, potassium hydroxide, calcium hydroxide and an aqueous ammonia, an ozone treatment, a hydrogen fluoride treatment, a plasma treatment such as oxygen and argon, a film-forming treatment of Langmuir-Brodgett film, a thin-film forming treatment of other insulator and semiconductor films, a mechanical treatment, an electric treatment such as corona discharge and a rubbing treatment using fibers.

Processes for the surface treatment are exemplified by a vacuum deposition process, a sputtering process, a coating process, a printing process and a sol-gel process.

A protective film consisting of resins or inorganic compounds may be laminated on the organic semiconductor active layer. The formation of the protective film inhibits influences from the outer circumstances to result in stabilization of the transistor drive.

The thin film of the present application exhibits a high carrier-mobility, since it comprises the substituted benzochalcogenoacene compound (1). Therefore, the thin film of the present application is useful as the organic semiconductor active layer in the organic transistor, and the organic transistor having the organic semiconductor active layer comprising the thin film of the present invention exhibits excellent transistor performances and is useful for the organic semiconductor device.

### [Examples]

The present invention is further explained in detail by the following examples.

### <Preparation example 1: Synthesis of 2-bromo-4-hexylaniline>

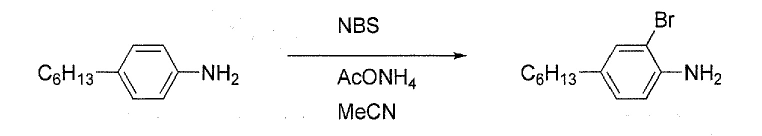

To a mixture solution of 4-hexylaniline (manufactured by Wako Pure Chemical Industries, Ltd.) (50.53g, 285mmol), ammonium acetate (AcONH₄: 2.20g, 28.5mmol) and acetonitrile (MeCN: 855mmol) in a flask dipped in a water bath, N-bromosuccinimide (NBS: 53.26g, 299.3mmol) was added, and after the water bath was taken off, the reaction mixture was stirred for 3 hours. Then, the reaction solution was condensed by an evaporator, followed by addition of ethyl acetate and washing with water and brine. The organic phase was extracted and dried with sodium sulfate, followed by condensation by the evaporator to give an oil product. The oil product was purified by a silica gel column to give 2-bromo-4-hexylaniline (35.63g, 139.1mmol, yield 48.8%).
¹H-NMR(CDCl₃, δ ppm): 7.22 (d, J=1.9Hz, 1H), 6.91 (dd, J=8.1, 1.9Hz, 1H), 6.68 (d, J=8.1Hz, 1H), 3.93(s, 2H), 2.46(t, J=7.7Hz, 2H), 1.62-1.47 (m, 2H), 1.36-1.24 (m,6H), 0.88(t, J=6.8Hz, 3H)

### <Preparation example 2: Synthesis of 2-bromo-4-hexyl-1-iodobenzene>

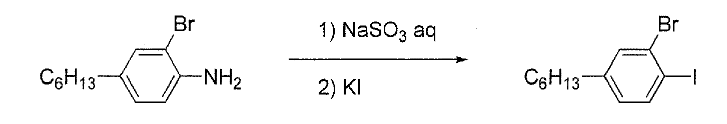

To a mixture solution of 2-bromo-4-hexylaniline (25.62g, 100.0mmol) obtained in the Preparation example 1 and water 450mL, a concentrated sulfuric acid 50g was added in drops, and the mixture was cooled to 5°C. An aqueous solution (water 20mL) of sodium sulfite (NaSO₃: 8.97g, 130.0mmol) was added in drops into the mixture and stirring was continued at 10°C for 2 hours, and subsequently, the reaction mixture was added to an aqueous solution (water 300ml) of potassium iodide (KI:132.8g, 0.80mol) at 5°C. Then, after stirring at room temperature (about 24°C) for 6 hours, the reaction mixture was refluxed for 20 minutes by heating, and then cooled to the room temperature. Subsequently, the reaction mixture was poured into an aqueous solution (water 450mL) of sodium sulfite (22.5g, 216.2mmol). Ethyl acetate was added to the reaction mixture, then, the organic phase was extracted, dried with magnesium sulfate and condensed by the evaporator to result in the formation of a brown oil (28.15 g) which contains 2-bromo-4-hexyl-1-iodobenzene as a main component (76.7mmol, yield 76.7%). ¹H-NMR (CDCl₃, δppm): 7.72 (d, J=8.1Hz, 1H), 7.45 (d, J=2.0Hz, 1H), 6.81 (dd, J=8.1, 2.0Hz, 1H), 2.52 (t, J=7.5Hz, 2H), 1.59-1.54 (m, 2H), 1.36-1.29 (m, 6H), 0.88(t, J=6.7Hz, 3H)
MS-EI 366, 368 (M+), 299, 297 (M-C₅H₁₁), 217(M-C₅H₁₁Br)

### <Preparation example 3: Synthesis of a compound represented by the formula [1715]>

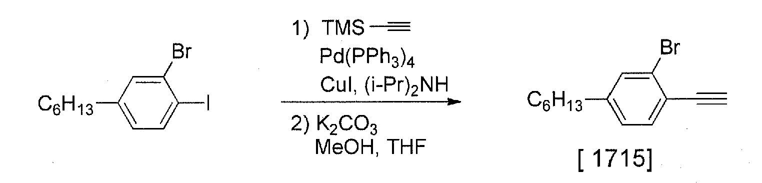

To a mixture of the oil 14.68g (40.0mmol) obtained in the Preparation example 2, tetrakis(triphenylphosphine)palladium (Pd(PPh₃)₄: 0.28g, 0.40mmol), copper iodide (CuI: 0.15g, 0.80mmol) and diisopropylamine ((i-Pr)₂NH:53.6mL), a diisopropylamine (26.4mL) solution of trimethylsilylacetylene (Me₃Si-C≡CH (TMS-C≡CH): 4.71g, 48.0mmol) was added in drops at room temperature, and stirring was continued for 2 hours. After cutting off a precipitated salt by a silica gel short column and condensing a filtrate, an oil was obtained in which 2-bromo-4-hexyl-1-(trimethylsilyl)ethynylbenzene is a main component. The obtained oil was diluted with tetrahydrofuran (THF: 80mL) and methanol (MeOH: 80mL), and subsequently, at room temperature, potassium carbonate (K₂CO₃:0.55g, 4.0mmol) was added thereto, followed by stirring for 3 hours. After the solvent was distilled off, a 1% aqueous solution of sodium ammonium and ether were added, and the organic phase was dried with magnesium sulfate, followed by condensation to result in a formation of light brown oil. Purification by a silica gel column using hexane as a developing solvent gave 2-bromo-1-ethynyl-4-hexylbenzene (hereinafter, optionally described as a compound [1715]) represented by the formula [1715] (7.56g, 28.5mmol, yield 71.2%).
¹H-NMR (CDCl₃, δppm): 7.42 (d, J=7.8Hz, 1H), 7.41 (d, J=1.8Hz, 1H), 7.07 (dd, J=7.8, 1.8Hz, 1H), 3.32 (s, 1H), 2.57 (t, J=7.7Hz, 2H), 1.67-1.51 (m, 2H), 1.37-1.22 (m, 6H), 0.88 (t, J=6.7Hz, 3H)
MS-EI 266, 264(M+), 195, 193(M-C₅H₁₁), 115(M-C₅H₁₁Br)

### <Preparation example 4: Synthesis of a compound represented by the formula [1145]>

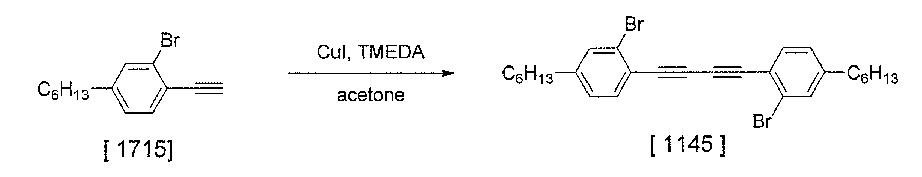

To an acetone solution (100mL) of copper iodide (CuI: 0.95g, 5.0mmol) and N,N,N',N'-tetramethylethylenediamine (TMEDA: 1.5mL, 10.0mmol), was added the compound [1715] (26.5g, 100.0mmol) obtained in the Preparation example 3, followed by stirring for 5 hours under air bubbling. Acetone was distilled off under vacuum, a 1N-hydrochloric acid was added to the residue, and then, the residue was extracted with chloroform and dried with magnesium sulfate, followed by condensation. Recrtstallization from toluene gave an orange powder of the compound represented by the formula [1145] 1,4-bis(2-bromo-4-hexylphenyl)-diacetylene (hereinafter, optionally described as the compound [1145]) (11.8g, 22.0mmol, yield 45.0%).
¹H-NMR (CDCl₃, δppm): 7.46 (d, J=7.8Hz, 2H), 7.42 (d, J=1.6Hz, 2H), 7.08 (dd, J=7.8, 1.6Hz, 2H), 2.58 (t, J=7.6Hz, 4H), 1.66-1.50 (m, 4H), 1.38-1.23 (m, 12H), 0.88 (t, J=6.7Hz, 6H)
MS-EI 528(M+), 457(M-C₅H₁₁), 386(M-C₁₀H₂₂)

### <Preparation example 5: Synthesis of a compound represented by the formula [575] >

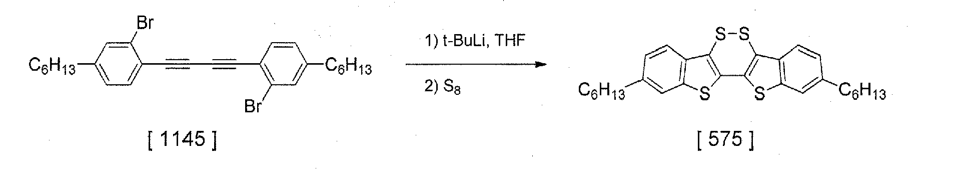

The compound [1145] (10.6g, 20mmol) obtained in the Preparation example 4 was dissolved in THF 200mL, and to this solution, under nitrogen atmosphere at -78°C, a 1.59M pentane solution (62.9mL, 100.0mmol) of t-BuLi was added in drops. After stirring at -78°C for 1 hour, a sulfur powder (3.2g, 100.0mmol) was added by small pieces, and subsequently, the temperature was slowly raised to room temperature and stirring was continued for 2 hours. A 1M sodium hydroxide solution (300mL) and K₃Fe (CN)₆ (32.9g, 100.0mmol) were added, and after stirring for 1 hour at room temperature, chloroform was added to extract the organic phase. The organic phase was washed with saturated brine, dried with magnesium sulfate and condensed by an evaporator. By recrystallization from hexane, the compound represented by the formula [575] was obtained as a deep red solid (hereinafter, optionally described as the compound [575]) (3.07g, 6.22mmol, yield 30.9%).
¹H-NMR(CDCl₃, δppm):7.69 (d, J=8.1Hz, 2H), 7.63 (d, J=1.6Hz, 2H), 7.28 (dd, J=8.1, 1.6H, 2H), 2.74 (t, J=7.6Hz, 4H), 1.72-1.62 (m, 4H), 1.40-1.24 (m, 12H), 0.89 (t, J=6.8Hz, 6H)

### <Example 1: Synthesis of a compound represented by the formula [5]>

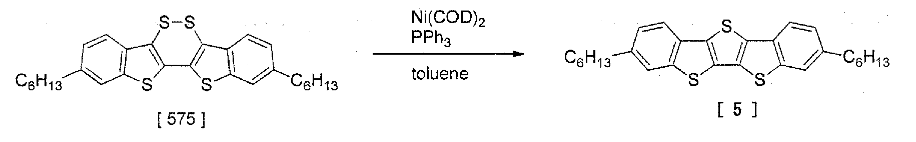

Under nitrogen atmosphere, toluene was added to the compound [575] (4.79g, 9.65mmol) obtained in the Preparation example 5, bis(1,5-cyclooctadiene)nickel(0) (Ni(COD)₂: 2.92g, 10.6mmol) and triphenylphosphine (PPhs:5.5'7g, 21.2mmol), followed by stirring at room temperature for 1 hour. Then, after stirring was continued at 110°C for 10 hours, the temperature was lowered to room temperature, and the reaction mixture was filtered with Celite®. The residue on the Celite® was extracted with a hot ortho-dichlorobenzene, and by half-condensation and cooling, the compound represented by the formula [5] (hereinafter, optionally described as the compound [5]) was obtained as a colorless plate crystal (1.82g, 3.92mmol, yield 40.6%). ¹H-NMR (CDCl₃, δppm): 7.65 (d, J=7.3Hz, 2H), 7.57 (d, J=1.0Hz, 2H), 7.19 (dd, J=7.3, 1.0Hz, 2H), 2.73 (t, J=7.0Hz, 4H), 1.73-1.63 (m, 4H), 1.40-1.29 (m, 12H), 0.90 (t, J=6.8Hz, 6H)
Elemental analysis: calculated value for C₂₈H₃₂S₃: C72.36, H6.94; observed value: C72.34, H6.85
Melting point: 236°C

### <Preparation example 7: Synthesis of a compound represented by the formula [1154] >

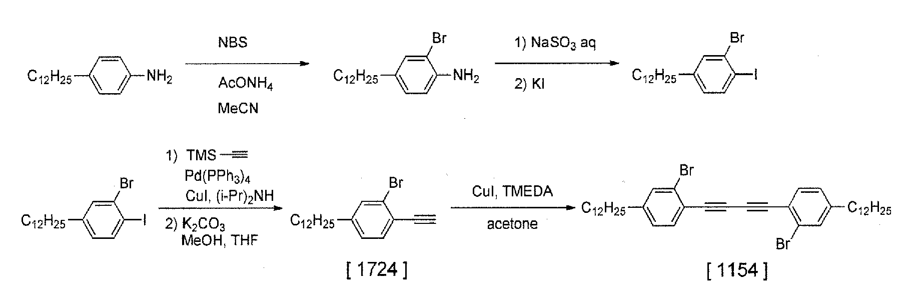

To a liquid mixture of 4-dodecylaniline (manufactured by Wako Pure Chemical Industries, Ltd.) (74.51g), ammonium acetate (AcONH₄: 2.20g) and acetonitrile (MeCN: 855mL), was added N-bromosuccinimide (NBS: 53.26g) at room temperature, and stirring was continued for 3 hours. Then, the reaction solution was condensed by the evaporator and the residue was washed with water and brine. Sequentially, ethylacetate was added, the organic phase was extracted and the extract was dried with sodium sulfate. After condensation by the evaporator, a black oil was obtained. Through purification by the silica gel chromatography using a mixed solvent of hexane: ethylacetate=1:1 as a developing solvent, a brown oil (98.13g) which contains 2-bromo-4-dodecylaniline as a main component was obtained.
By using 96.97g of the above-obtained oil as a raw material and by using water (2.14L), concentrated sulfuric acid (142.5L), sodium sulfite (NaSO₃: 25.57g) and potassium iodide (KI: 378.5g), a brown oil 104.72 g containing 2-bromo-4-dodecyl-1-iodobenzene as a main component was obtained by carrying out a similar procedure to the Preparation example 2 of 2-bromo-4-hexyl-1-iodobenzene.
By using 104.0g of the above-obtained oil as a raw material, and by using tetrakis(triphenylphosphine)palladium (Pd(PPh₃)₄ 1.01g), copper iodide (CuI: 0.548g), diisopropylamine ((i-Pr)₂NH: 88mL) and trimethylsilylacetylene (Me₃Si-C≡CH (TMS-C≡CH): 15.5g), a light brown oil 76.34g containing 2-bromo-4-dodecyl-1-(trimethylsilyl)ethynylbenzene as a main component was obtained by carrying out a similar procedure to the Preparation example 3 of 2-bromo-4-hexyl-1-(trimethylsilyl)-ethynylbenzene.
By using 73.88g of the oil obtained above as a raw material, and by using tetrahydrofuran (220mL), methanol (220mL) and potassium carbonate (1.52g), a light brown oil 65.01g containing the compound represented by the formula [1724] as a main component was obtained by carrying out a similar procedure to the Preparation example 3 of 2-bromo-1-ethynyl-4-dodecylbenzene.
Sixty four (64.0) grams of the oil obtained above were added to an acetone solution (120mL) of copper iodide (CuI: 1.10g) and N,N,N',N'-tetramethylethylenediamine (TMEDA: 1.72mL), and the reaction mixture was stirred at room temperature for 11 hours. Sequentially, acetone was distilled off under vacuum, 1N-hydrochloric acid was added to the residue and the residue was extracted with chloroform. The chloroform extract was washed with water and brine, and then, dried with magnesium sulfate and condensed. The tar-like residue obtained was dissolved in hexane, and by condensing the solution, the compound represented by the formula [1154] (hereinafter, optionally described as the compound [1154]) was obtained as a yellow powder (22.27g, 32.97mmol, yield 12%).
¹H-NMR (CDCl₃, δppm): 7.46 (d, J=7.8Hz, 2H), 7.42 (d, J=1.6Hz,2 H), 7.09 (dd, J=7.8, 1.4Hz, 2H), 2.58 (t, J=8.1Hz, 4H), 1.64-1.52 (m, 4H), 1.35-1.21 (m,36H), 0.88 (t, J=7.0Hz, 6H)

### <Preparation example 8: Synthesis of a compound represented by the formula [584]>

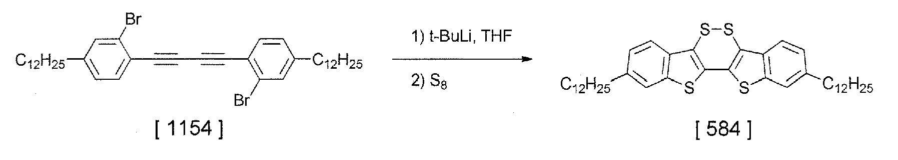

By using the compound [1154] (17.0g, 24.4mmol) obtained in the Preparation example 7, tetrahydrofuran (340mL), a 1.59M pentane solution (67.6mL, 107.36mmol) of t-BuLi, a sulfur powder (3.45g, 107.36mmol), a 1M aqueous solution of sodium hydroxide (300mL) and K₃Fe (CN)₆ (35.4g, 107.36mmol), a similar procedure to the Preparation example 5 was carried out, and a compound represented by the formula [584] (hereinafter, optionally described as the compound [584]) was obtained (7.91g, 11.89mmol, yield 49%).
¹H-NMR (CDCl₃, δppm): 7.69 (d, J=8.4Hz, 2H), 7.62 (d, J=0.8Hz, 2H), 7.28 (dd, J=8.1, 1.4Hz, 2H), 2.74 (t, J=7.8Hz, 4H), 1.73-1.61 (m, 4H), 1.38-1.22 (m, 36H), 0.88(t, J=7.0Hz, 6H)

### <Example 2: Synthesis of a compound represented by the formula [14]>

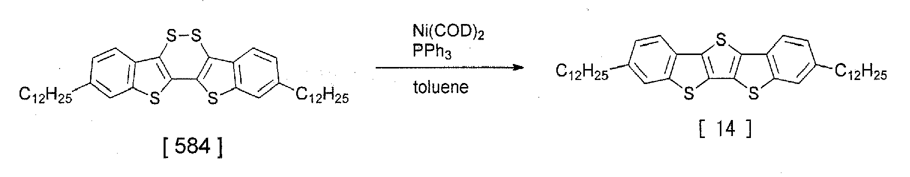

By using the compound [584] obtained in the Preparation example 8 (1.0g, 1.50mmol), bis(1,5-cyclooctadiene)nickel(0) (Ni(COD)₂: 454mg, 1.65mmol), triphenylphosphine (PPh₃: 866mg, 3.3mmol) and toluene (15mL), a similar procedure to Example 1 was carried out, and a compound represented by the formula [14] was obtained (the compound [14], 498mg, 0.787mmol, yield 52%).
¹H-NMR (CDCl₃, CS2 δppm): 7.75 (d, J=8.1Hz, 2H), 7.67 (d, J=0.8Hz, 2H), 7.26 (dd, J=8.1, 1.6Hz, 2H), 2.75 (t, J=7.6Hz, 4H), 1.73-1.65 (m, 4H), 1.40-1.22 (m, 36H), 0.88 (t, J=7.3Hz, 6H).
HRMS (EI): calculated value for C₄₀H₅₆S₃: 632.3531(M+); observed value: 632.3544
Elemental analysis: calculated value for C₄₀H₅₆S₃: C75.89, H8.92; observed value: C75.92, H8.94
Melting point: 194°C

### <Example 3: Formation of a thin film consisting of the compound [5] by vacuum deposition method and manufacture of an organic transistor having the thin film>

Electrodes of chromium in 3nm and gold in 50nm deposited in this order were formed by the vapor deposition method using a metal mask on the substrate laminated with hexamethyldisilazane by spin coating over n-doped silicon wafer having a thermally oxidized SiO₂ film.
Each of a channel width and a channel length of the electrode formed was 2000µm and 20µm, respectively. Then, the compound [5] synthesized in Example 1 and purified by sublimation was put into a quartz crucible, and the crucible was heated to form a thin film consisting of the compound [5] by the vacuum deposition method.
The degree of vacuum in the apparatus chamber used for the vacuum deposition method was 1 × 10⁻⁴ pascal or lower, and the temperature of the substrate was in a range from room temperature (24°) to 80°C or lower. The thickness of the thin film was about 200nm.
Thus, an organic transistor (refer to Fig. 1) having the thin film consisting of the compound [5] purified by sublimation was manufactured.

### <Example 4: Measurements relating to the organic transistor having the thin film consisting of the compound [5]>

Electric performances of the organic transistor having the thin film manufactured in Example 3 and consisting of the compound [5] were measured in vacuum using a parameter analyzer. The observed results showed that a minus drain current (Id) increased by increasing an applied minus gate voltage (Vg) on a gate electrode. Therefore, the organic transistor manufactured consisting of the thin film of the compound [5] was confirmed to be a p-type organic transistor. In a minus gate voltage (Vg), a change curve of the drain current (Id) vs. the drain voltage (Vd) was good and had a saturation area at a high drain voltage. In addition, a saturated field-effect mobility µ of the carrier in the organic transistor can be calculated by using the formula: $Id = \left(W/2⁢L\right) ⁢ μCi ⁢ {\left(Vg-Vt\right)}^{2}$
which represents a drain current Id in the saturation area of the electric performance of the organic transistor. In the equation (a), each of L and W represents a channel length and a channel width of the organic transistor, respectively, Ci represents an electrostatic capacitance per unit area of an insulating layer for the gate electrode (hereinafter, optionally described as a gate insulating film), Vg represents a gate voltage, Vt represents a threshold value voltage of the gate voltage. The saturated field-effect mobility µ of the carrier in the organic transistor having the thin film consisting of the compound [5] and manufactured was calculated by using the formula (a), and the following results were obtained. That is, the saturated field-effect mobility of the carrier (carrier mobility) in the organic transistor having the thin film consisting of the compound [5] and manufactured at a substrate temperature of 60°C was 1.6cm²/Vs. In addition, the ratio of the drain currents Ids at the gate voltages of 0 V and - 50 V (hereinafter, optionally described as on/of ratio) at the drain voltage Vd of -50 V was 10⁷.

### <Example 5: Solubility of the compound [5] in a solvent and formation of a thin film consisting of the compound [5] by the applying and film-forming process>

The solution composition containing the compound [5] in 0.5 wt% concentration was prepared by dissolving the compound [5] manufactured in Example 1 in tetrahydrofuran.
This solution composition was applied on the n-doped silicon wafer having a thermally oxidized SiO₂ film treated with hexamethyldisilazane using a spin coat method, and thus, the thin film consisting of the compound [5] was formed. In addition, the formed thin film was kept at 80°C for 30 minutes. The thickness of the thin film was about 30nm.

### <Example 6: Manufacture of the organic transistor having the thin film consisting of the compound [5]>

On the thin film obtained in Example 5, a molybdenum oxide layer and successively a gold layer were formed using a metal mask by the vacuum deposition method, and thus, a source electrode and a drain electrode were formed. Here, each of a channel width and a channel length of the organic TFT obtained by forming the source electrode and the drain electrode was 2000µm and 20µm, respectively. Thus, the organic transistor having the thin film comprising the compound [5] as shown in Fig. 2 was manufactured.

### <Example 7: Measurements relating to the organic transistor having the thin film consisting of the compound [5]>

The electric performances of the organic transistor manufactured in Example 6 were also measured similarly to Example 4. The results showed that each of the field-effect mobility of the carrier (carrier mobility) and the on/off ratio was 0.3cm²/Vs and 10⁷, respectively.

### <Example 8: Manufacture of the organic transistor having a thin film consisting of the compound [14] >

On the n-doped silicon wafer having a thermally oxidized SiO₂ film, a source electrode and a drain electrode (in the sequence of chromium and gold starting from the thermally oxidized SiO₂ film) having a channel width of 2000µm and a channel length of 20µm were formed. The substrate was washed with acetone in ultrasonic bath for 10 minutes and irradiated by an ozone UV for 20 minutes. Then, the substrate surface was silanized by dipping the substrate in the toluene diluent solution of phenylethyltrichlorosilane for 2 minutes. In addition, the surface of the Au electrode formed on the substrate was modified by dipping the substrate in the isopropyl alcohol diluent solution of perfluorobenzene thiol for 2 minutes, and thus, the transistor substrate was manufactured. Then, the compound [14] synthesized in Example 2 was put into the crucible, the crucible was heated, and thus, by the vacuum deposition method, a thin film consisting of the compound [14] was formed on the transistor substrate. A vacuum degree in the apparatus chamber used for the vacuum deposition was 1×10⁻⁴ pascal or less and the substrate temperature was 80°C. A thickness of the thin film was about 100nm.

### <Example 9: Measurements relating to the organic transistor having the thin film consisting of the compound [14]>

On the proviso that a drain voltage (Vd) of the organic thin film transistor device obtained in Example 8 was fixed at -40V and a gate voltage (Vg) of the transistor was varied from 20 to -40V, the transistor performances were measured. The field-effect mobility (carrier mobility) was 0.4cm²/Vs and the on/off ratio was 10⁷, both were calculated from the transmission performances obtained by the above measurements.

### <Example 10: Solubility of the compound [14] in a solvent and formation of a thin film consisting of the compound [14] by the applying and film-forming process>

On the substrate obtained by a similar procedure to Example 8, a 0.5wt% dichlorobenzene solution of the compound [14] heated at 100°C was applied by the spin coating method and dried on a hot plate of 120°C for 30 minutes to result in the formation of a thin film containing the compound and having a thickness of about 30nm.

### <Example 11: Measurements relating to the organic transistor having the thin film consisting of the compound [14]>

On the proviso that a drain voltage (Vd) of the organic thin film transistor device obtained above was fixed at -40V and a gate voltage (Vg) of the transistor was varied from 20 to -40V, the transistor performances were measured. The field-effect mobility (carrier mobility) was 0.5cm²/Vs and the on/off ratio was 10⁷, both were calculated from the transmission performances obtained by the above measurements.

### <Comparative example 1: Manufacture of an organic transistor having a thin film consisting of the compound C-1 and measurements relating to the transistor>

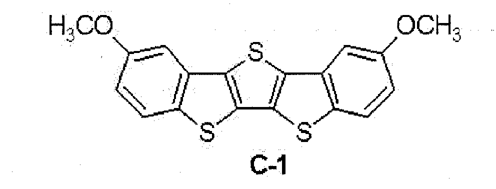

A thin film was formed by the vacuum deposition method according to the same procedure as Example 3 except using the compound C-1 represented by the above formula and disclosed in the patent document 1, then followed by manufacturing the organic transistor having the thin film.
The electric performances of the obtained organic transistor were measured according to Example 4, and the results showed that the carrier mobility and the on/off ratio of the obtained organic transistor were 10⁻⁵cm²/Vs and 10³, respectively.

### <Example 12: Preparation of the compound [14], formation of a thin film consisting of the compound [14] by the applying and film-forming process, and manufacture and measurement of a transistor having the thin film>

The compound [14] (which means the compound No. 41 in Table 2) can be obtained according to similar procedures to Preparation examples 7 and 8 and Example 2 except that 4-pentyloxyaniline is used instead of 4-dodecylaniline.
Using the compound [41], a transistor substrate is prepared according to a similar procedure to Example 8, then, an organic transistor having the thin film is produced according to a similar procedure to Example 10. By the measurement of the organic transistor obtained according to a similar procedure to Example 9, a high value of the carrier mobility can be obtained.

### <Example 13: Preparation of the compound [155]>

The compound [155] (which means the compound No. 155 in Table 9) can be obtained according to similar procedures to Preparation examples 7 and 8 and Example 2 except that 4-(4-phenylbutul)aniline is used instead of 4-dodecylaniline.
Using the compound [155], a transistor substrate is prepared according to a similar procedure to Example 8, then, an organic transistor having the thin film is produced according to a similar procedure to Example 10. By the measurement of the organic transistor obtained according to a similar procedure to Example 9, a high value of the carrier mobility can be obtained.

### <Example 14: Preparation of the compound [222]>

The compound [222] (which means the compound No. 222 in Table 13) can be obtained according to similar procedures to Preparation examples 7 and 8 and Example 2 except that 3-octylaniline is used instead of 3-octylaniline in Preparation example 1.
Using the compound [222], a transistor substrate is prepared according to a similar procedure to Example 8, then, an organic transistor having the thin film is produced according to a similar procedure to Example 10. By the measurement of the organic transistor obtained according to a similar procedure to Example 9, a high value of the carrier mobility can be obtained.

### <Example 15: Preparation of the compound [7]>

The compound [7] (which means the compound No. 7 in Table 1) can be obtained according to similar procedures to Preparation examples 7 and 8 and Example 2 except that 4-(2-ethylhexyl)aniline is used instead of 4-dodecylaniline.

### <Example 16: Preparation of the compound [12]>

The compound [12] (which means the compound No. 12 in Table 1) can be obtained according to similar procedures to Preparation examples 7 and 8 and Example 2 except that 4-(2-hexyldecyl)aniline is used instead of 4-dodecylaniline.

### <Example 17: Preparation of the compound [15]>

The compound [15] (which means the compound No. 15 in Table 1) can be obtained according to similar procedures to Preparation examples 7 and 8 and Example 2 except that 4-tridecylaniline is used instead of 4-dodecylaniline.

### <Example 18: Preparation of the compound [18]>

The compound [18] (which means the compound No. 18 in Table 2) can be obtained according to similar procedures to Preparation examples 7 and 8 and Example 2 except that 4-hexadecylaniline is used instead of 4-dodecylaniline.

### <Example 19: Preparation of the compound [42]>

The compound [42] (which means the compound No. 42 in Table 2) can be obtained according to similar procedures to Preparation examples 7 and 8 and Example 2 except that 4-(2-hexyloctyl)aniline is used instead of 4-dodecylaniline.

### <Example 20: Preparation of the compound [84]>

The compound [84] (which means the compound No. 84 in Table 4) can be obtained according to similar procedures to Preparation examples 7 and 8 and Example 2 except that 4-(4'-hexylphenyl)aniline is used instead of 4-dodecylaniline.

### <Example 21: Preparation of the compound [97]>

The compound [97] (which means the compound No. 97 in Table 5) can be obtained according to similar procedures to Preparation examples 7 and 8 and Example 2 except that 4-(2-(5-hexyl)thienyl) aniline is used instead of 4-dodecylaniline.

### <Example 22: Preparation of the compound [205]>

The compound [205] (which means the compound No. 205 in Table 12) can be obtained according to similar procedures to Preparation examples 7 and 8 and Example 2 except that 4-[(2-benzo[b]thieno)octyl]aniline is used instead of 4-dodecylaniline.

### <Example 23: Preparation of the compound [208]>

The compound [208] (which means the compound No. 208 in Table 12) can be obtained according to the following formula in the document: Advanced Materials, 19, 3008-3011 (2007):.

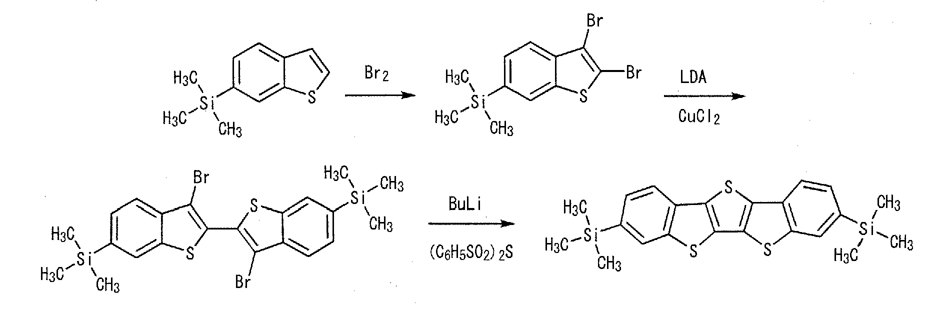

### INDUSTRIAL APPLICABILITY

The present invention can provide the new substituted benzochalcogenoacene compound, the thin film comprising the compound and the organic semiconductor device comprising the thin film.

### EXPLANATION OF LETTERS OR NUMERALS

- 11:: substrate
- 12:: gate electrode
- 13:: gate insulating film
- 14:: source electrode
- 15:: drain electrode
- 16:: organic semiconductor active layer
- 21:: substrate
- 22:: gate electrode
- 23:: gate insulating film
- 24:: source electrode
- 25:: drain electrode
- 26:: organic semiconductor active layer

## Claims

1. A substituted benzochalcogenoacene compound represented by the formula (1): 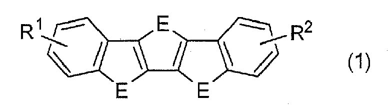 wherein each of E independently represents a sulfur or selenium atom, and each of R¹ and R² independently represents a hydrogen atom, an optionally substituted C₄₋₃₀ alkyl group, an optionally substituted C₄₋₃₀ alkoxy group, an optionally substituted C₆₋₃₀ aryl group, an optionally substituted C₇₋₃₀ aralkyl group, an optionally substituted C₄₋₃₀ heteroaryl group, an optionally substituted C₅₋₃₀ heteroaralkyl group, or an optionally fluorinated C₃₋₃₀ trialkylsilyl group, wherein R¹ and R² are not hydrogen atoms all together.

2. The compound according to claim 1 wherein all E's in the formula (1) is sulfur atom.

3. The compound according to claim 1 or 2 wherein each of R¹ and R² in the formula (1) independently represents a hydrogen atom, an optionally fluorinated C₄₋₃₀ alkyl group, an optionally fluorinated C₄₋₃₀ alkoxy group, an optionally alkylated or alkoxylated C₆₋₃₀ aryl group which is optionally fluorinated, an optionally fluorinated C₇₋₃₀ aralkyl group, an optionally alkylated or alkoxylated C₄₋₃₀ heteroaryl group which is optionally fluorinated, or an optionally fluorinated C₅₋₃₀ heteroaralkyl group.

4. The compound according to any one of claims 1 to 3 wherein the compound represented by the formula (1) is a compound represented by the formula (2): 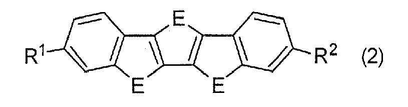 wherein E, R¹ and R² represent the same meanings as described above.

5. The compound according to claim 4 wherein, in the formula (2), each E independently represents a sulfur or selenium atom, and each of R¹ and R² independently represents a hydrogen atom, an optionally fluorinated C₄₋₃₀ alkyl group, or an optionally alkylated or fluorinated C₃₋₃₀ trialkylsilyl group.

6. The compound according to claim 5 wherein each of R¹ and R² in the formula (2) independently represents a C₄₋₃₀ alkyl group or a C₃₋₃₀ trialkylsilyl group.

7. The compound according to claim 5 wherein R¹ and R² in the formula (2) are C₄₋₃₀ alkyl groups.

8. The compound according to claim 5 wherein R¹ and R² in the formula (2) are the same and represent C₄₋₂₀ alkyl groups.

9. The compound according to claim 5 wherein R¹ and R² in the formula (2) are C₆₋₁₂ alkyl groups.

10. The compound according to claim 4 wherein each of R¹ and R² in the formula (2) independently represents a hydrogen atom, an optionally fluorinated C₄₋₃₀ alkyl group, an optionally fluorinated C₄₋₃₀ alkoxy group, an optionally alkylated C₆₋₃₀ aryl group which is optionally fluorinated, or an optionally fluorinated C₇₋₃₀ aralkyl group.

11. The compound according to claim 4 wherein R¹ and R² in the formula (2) are the same and represent C₄₋₂₀ alkoxy groups.

12. The compound according to claim 4 wherein R¹ and R² in the formula (2) are the same and represent C₆₋₁₀ aryl groups having C₁₋₂₀ alkyl groups.

13. The compound according to claim 4 wherein R¹ and R² in the formula (2) are the same and represent C₇₋₂₀ aralkyl groups.

14. The compound according to claim 5 wherein each of R¹ and R² in the formula (2) independently represents a C₃₋₃₀ trialkylsilyl group.

15. The compound according to claim 5 wherein each R¹ and R² in the formula (2) independently represents a C₃₋₁₄ trialkylsilyl group.

16. The compound according to claim 4 or 5 wherein R¹ and R² in the formula (2) are the same and represent hexyl or dodecyl.

17. The compound according to any one of claims 4 to 16 wherein all E's in the formula (2) are sulfur atoms.

18. The compound according to claim 4 wherein all E's in the formula (2) represent sulfur atoms, and R¹ and R² in the formula (2) represent hexyl.

19. The compound according to claim 4 wherein all E's in the formula (2) represent sulfur atoms, and R¹ and R² in the formula (2) are the same and represent dodecyl.

20. The compound according to claim 4 wherein all E's in the formula (2) represent sulfur atoms, and each of R¹ and R² in the formula (2) independently represents a C₆₋₁₂ alkyl group.

21. A compound represented by the formula [5], [7], [12], [15], [18] or [42] below:

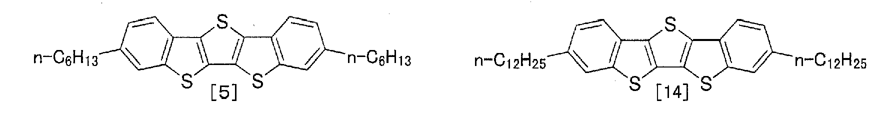

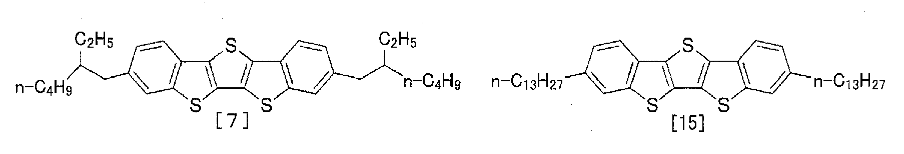

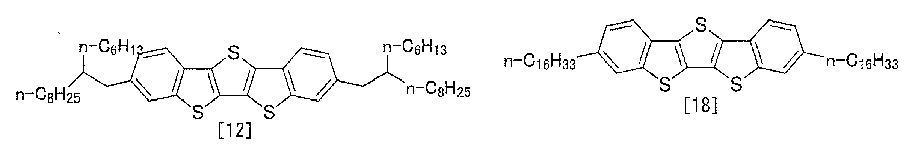

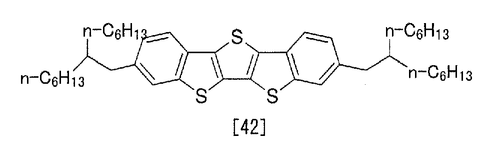

22. The compound according to any one of claims 1 to 3 wherein the compound represented by the formula (1) is a compound represented by the formula (3): 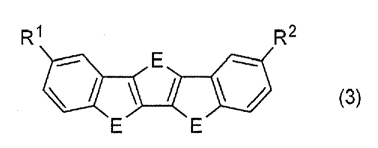 wherein E, R¹ and R² represent the same meanings as described above.

23. The compound according to claim 22 wherein R¹ and R² in the formula (3) are the same and represent C₄₋₂₀ alkyl groups.

24. A thin film comprising the compound according to any one of claims 1 to 23.

25. A thin film consisting of the compound according to any one of claims 1 to 23.

26. An organic semiconductor device comprising the thin film according to claim 24 or 25.

27. An organic transistor comprising the thin film according to claim 24 or 25.
